Europäisches Patentamt
European Patent Office
Office européen des brevets

(19)

(11) Veröffentlichungsnummer: **0 214 479**
**A2**

(12)

# EUROPÄISCHE PATENTANMELDUNG

(21) Anmeldenummer: 86110990.8

(22) Anmeldetag: 08.08.86

(51) Int.Cl.[4]: C 07 D 401/04
C 07 D 401/12, C 07 D 417/12
C 07 D 405/12, C 07 D 473/38
C 07 D 491/04, A 61 K 31/415
A 61 K 31/44, A 61 K 31/505
A 61 K 31/41

(30) Priorität: 12.08.85 CH 3455/85
10.06.86 CH 2350/86

(43) Veröffentlichungstag der Anmeldung:
18.03.87 Patentblatt 87/12

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: Fischli, Albert, Prof. Dr.
Im Wenkenberg 20
CH-4125 Riehen(CH)

(72) Erfinder: Krasso, Anna, Dr.
Rütimeyerstrasse 35
CH-4054 Basel(CH)

(72) Erfinder: Szente, André, Dr.
Baselstrasse 70
CH-4125 Riehen(CH)

(74) Vertreter: Lederer, Franz, Dr. et al,
Vanderwerth, Lederer & Riederer Patentanwälte
Lucile-Grahn-Strasse 22
D-8000 München 80(DE)

(54) **Benzimidazol-2-yl-pyridiniumverbindungen.**

(57) Benzimidazol-2-yl-pyridiniumverbindungen der allgemeinen Formel

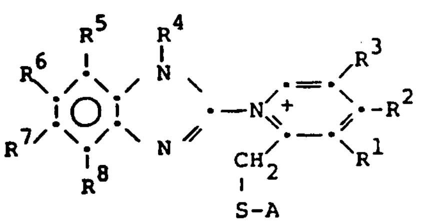

I

worin
A einen Rest der Formel

$-SR^9$, $-SO_3^-$ oder $-S-SO_3^-$ darstellt;

und $R^1$ bis $R^9$ die aus der Beschreibung ersichtlichen Bedeutungen haben können,

wobei das Molekül gesamthaft ungeladen oder einfach positiv geladen ist und wobei im letzteren Fall ein externes Anion vorliegt;

sowie pharmazeutisch annehmbare Säureadditionssalze von basisch substituierten Verbindungen der Formel I; eignen sich aufgrund ihrer magensäuresekretionshemmenden und/oder mukosaprotektiven Eigenschaften zur Bekämpfung oder Verhütung von Krankheiten des Magen-Darm-Trakts, insbesondere von Ulcus ventriculi und duodeni. Diese Verbindungen sind mit wenigen Ausnahmen neu, und sie können mit pharmazeutisch inerten Excipientien zu Arzneimitteln verarbeitet werden. Die Ausgangsprodukte für ihre Herstellung sind bekannt oder nach an sich bekannten Methoden leicht herstellbar.



Croydon Printing Company Ltd.

0214479

F. HOFFMANN-LA ROCHE & CO. Aktiengesellschaft, Basel/Schweiz

8. Aug. 1986

RAN 4038/6

# Benzimidazol-2-yl-pyridiniumverbindungen

Die vorliegende Erfindung betrifft Benzimidazol--Derivate. Im speziellen betrifft sie Benzimidazol-2-yl--pyridiniumverbindungen der allgemeinen Formel

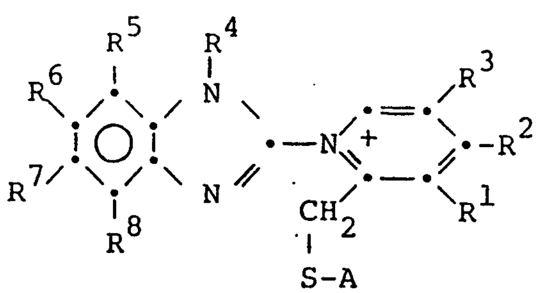

I

worin

A einen Rest der Formel

$-SR^9$, $-SO_3^-$ oder $-S-SO_3^-$ ;

$R^1$ und $R^3$ je Wasserstoff oder $(C_1-C_7)$-Alkyl;

$R^2$ Wasserstoff, $(C_1-C_7)$-Alkyl, $(C_1-C_7)$-Alkoxy oder ein negativ geladenes Sauerstoffatom;

$R^4$ Wasserstoff oder eine negative Ladung;

$R^5$, $R^6$, $R^7$ und $R^8$ je Wasserstoff, $(C_1-C_7)$-Alkyl, Aryl, Halogen, Cyan, Nitro, Formyl, $(C_2-C_7)$-Alkanoyl, Arylcarbonyl, Carboxy, Carboxy-$(C_1-C_7)$-alkyl,

Bt/ 10.06.86

($C_1$-$C_7$)-Alkoxycarbonyl, Aryloxycarbonyl, Aryl-($C_1$-$C_7$)-alkoxycarbonyl, ($C_1$-$C_7$)-Alkoxycarbonyl-($C_1$-$C_7$)-alkyl, Carbamoyl, Mono- oder Di-($C_1$-$C_7$)-alkylcarbamoyl, Pyrrolidinocarbonyl, Piperidinocarbonyl, Carbamoyl-($C_1$-$C_7$)-alkyl, Mono- oder Di-($C_1$-$C_7$)-alkylcarbamoyl-($C_1$-$C_7$)-alkyl, Pyrrolidinocarbonyl-($C_1$-$C_7$)-alkyl, Piperidinocarbonyl-($C_1$-$C_7$)-alkyl, Hydroxy, ($C_1$-$C_7$)-Alkoxy, ($C_2$-$C_7$)-Alkanoyloxy, Aryloxy, Arylcarbonyloxy, ($C_1$-$C_7$)-Alkoxycarbonyloxy, Aryl-($C_1$-$C_7$)-alkoxycarbonyloxy, Aryloxycarbonyloxy, Carbamoyloxy, Mono- oder Di-($C_1$-$C_7$)-alkylcarbamoyloxy, Pyrrolidinocarbonyloxy, Piperidinocarbonyloxy, Hydroxy-($C_1$-$C_7$)-alkyl, Trifluormethyl, Di-($C_1$-$C_7$)-alkoxymethyl oder ($C_2$-$C_3$)-Alkylendioxymethyl oder zwei benachbarte dieser Substituenten gemeinsam und zusammen mit den Kohlenstoffatomen, an welche sie gebunden sind, einen 5-, 6- oder 7-gliedrigen Ring; und

$R^9$ ($C_1$-$C_{20}$)-Alkyl, ($C_3$-$C_7$)-Cycloalkyl, ($C_3$-$C_7$)-Alkenylalkyl, ($C_3$-$C_7$)-Alkinylalkyl, substituiertes ($C_3$-$C_7$)-Alkenylalkyl, Aryl, Aryl-($C_1$-$C_7$)-alkyl, Hydroxy-($C_2$-$C_7$)-alkyl, ($C_1$-$C_7$)-Alkoxy-($C_2$-$C_7$)-alkyl, ($C_1$-$C_7$)-Alkoxycarbonyl-($C_1$-$C_7$)-alkyl, Carboxy-($C_1$-$C_7$)-alkyl, Di-($C_1$-$C_7$)-alkoxycarbonyl-($C_2$-$C_7$)-alkyl, Dicarboxy-($C_2$-$C_7$)-alkyl, Carboxy-($C_1$-$C_7$)-alkylcarbamoyl-($C_1$-$C_7$)-alkyl, gegebenenfalls N-substituiertes Amino-($C_2$-$C_7$)-alkyl, gegebenenfalls N-substituiertes Amino-carboxy-($C_2$-$C_7$)-alkyl, gegebenenfalls N-substituiertes Amino-($C_1$-$C_7$)-alkoxycarbonyl-($C_2$-$C_7$)-alkyl, Heteroaryl, Heteroaryl-($C_1$-$C_7$)-alkyl oder einen durch Elimination der SH-Gruppe von einem cysteinhaltigen Oligopeptid abgeleiteten Rest bedeuten;

wobei das Molekül gesamthaft ungeladen oder einfach positiv geladen ist und wobei im letzteren Fall ein externes Anion vorliegt;

sowie pharmazeutisch annehmbare Säureadditionssalze von basisch substituierten Verbindungen der Formel I.

Diese Verbindungen sind neu, mit Ausnahme derjenigen, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, und es hat sich gezeigt, dass sie wertvolle pharmakodynamische Eigenschaften besitzen, nämlich magensäuresekretions-hemmende und/oder Mukosa-protektive Eigenschaften (speziell gegen Indomethacin-induzierte Läsionen), sodass sie zur Bekämpfung oder Verhütung von Krankheiten des Magen-Darm-Trakts verwendet werden können, insbesondere gegen Ulcus ventriculi und duodeni.

Gegenstand der vorliegenden Erfindung sind die eingangs definierten Verbindungen und Salze als therapeutische Wirkstoffe, Arzneimittel, enthaltend eine solche Verbindung oder ein Salz davon, die Herstellung solcher Arzneimittel, die Verwendung der eingangs definierten Verbindungen und Salze bei der Bekämpfung bzw. Verhütung von Krankheiten, insbesondere bei der Bekämpfung bzw. Verhütung von Ulcus ventriculi und duodeni bzw. die Verwendung der eingangs definierten Verbindungen und Salze zur Herstellung von Arzneimitteln für die Bekämpfung bzw. Verhütung von Ulcus ventriculi und duodeni, sowie die neuen unter den eingangs defininerten Verbindungen und Salzen als solche und die Herstellung dieser neuen Verbindungen und Salze.

Wenn das Molekül gesamthaft ungeladen ist, dann liegen die Benzimidazol-2-yl-pyridiniumverbindungen der allgemeinen Formel I in Form innerer Salze vor. Dies ist dann der Fall, wenn entweder A einen Rest der Formel $-SR^9$ bedeutet und $R^9$ eine negativ geladene Gruppe trägt oder A einen Rest der Formel $-SO_3^-$ oder $-S-SO_3^-$ oder $R^4$ eine negative Ladung oder $R^2$ ein negativ geladenes Sauerstoffatom

bedeutet; in letzterem Fall können die Verbindungen auch als Pyridon-Derivate mit der Partialformel

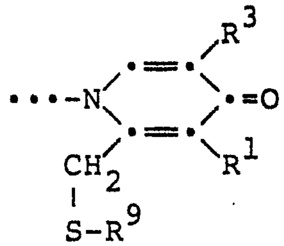

Ia

aufgefasst werden. Basisch substituierte Verbindungen der Formel I können auch Säureadditionssalze bilden.

Der Ausdruck "Alkyl" bezeichnet geradkettige oder verzweigte gesättigte Kohlenwasserstoffreste, wie Methyl, Aethyl, n-Propyl, i-Propyl, sec-Butyl, t-Butyl, n-Pentyl, n-Hexyl, n-Octyl, t-Octyl, n-Dodecyl, n-Hexadecyl und n-Octadecyl. Der Ausdruck "Alkoxy" bezeichnet über ein Sauerstoffatom gebundene Alkyl- gruppen im Sinne der vorstehenden Definition.

Der Ausdruck "Aryl" bezeichnet ein- oder mehrkernige aromatische Kohlenwasserstoffreste, welche substituiert sein können, und zwar beispielsweise durch Halogen, Trifluormethyl, Nitro, gegebenenfalls N-substituiertes Amino, ($C_1$--$C_7$)-Alkyl, ($C_1$-$C_7$)-Alkoxy, Carboxy oder ($C_1$-$C_7$)--Alkoxycarbonyl; Beispiele für derartige Arylreste sind Phenyl, o-Tolyl, p-Tolyl, o-Chlorphenyl, m-Chlorphenyl, p-Chlorphenyl, 2,3-Dichlorphenyl, 2,4-Dichlorphenyl, 2,5-Dichlorphenyl, 2,6-Dichlorphenyl, 3,4-Dichlorphenyl, m-Bromphenyl, p-Bromphenyl, m-Fluorphenyl, p-Fluorphenyl, Pentafluorphenyl, o-Carboxyphenyl, o-Methoxycarbonylphenyl, o-Methoxyphenyl, m-Methoxyphenyl, p-Methoxyphenyl, o-Nitrophenyl, m-Nitrophenyl, p-Nitrophenyl, o-Aminophenyl, m-Aminophenyl, p-Acetylaminophenyl und m-Trifluormethylphenyl. Der Ausdruck "Halogen" umfasst die vier Formen Chlor, Fluor, Brom und Jod. Der Ausdruck "Alkylen" umfasst in erster Linie Polymethylenketten. Der Ausdruck "Cycloalkyl" umfasst in erster Linie unsubstituierte cycloaliphatische Reste, wie Cyclopentyl und Cyclohexyl. Die Ausdrücke "Alkenylalkyl" und "Alkinylalkyl" bezeichnen Kohlenwasserstoffreste, welche eine Kohlenstoff-Kohlenstoff-Doppel- oder -Dreifachbindung enthalten und in welchen das Kohlenstoffatom, über welches sie gebunden sind, nicht ungesättigt ist, z.B. also Reste wie Allyl. Der Ausdruck "substituiertes Alkenylalkyl" bezeichnet einen Alkenylalkylrest im Sinne der vorstehenden Definition, welcher vinylisch substituiert ist, insbesondere durch Halogen, wie Chlor; ein Beispiel eines solchen Restes ist 2-Chlorallyl. Die Ausdrücke "Dicarboxyalkyl" und "Di-alkoxycarbonylalkyl" bezeichnen Alkylreste, welche durch zwei, jedoch nicht am gleichen Kohlenstoffatom sitzende Carboxygruppen bzw. Alkoxycarbonylgruppen substituiert sind. Der Ausdruck "N-substituiert" besagt, dass eine Aminogruppe durch ($C_1$- -$C_7$)-Alkyl oder ($C_1$-$C_7$)-Alkanoyl mono-substituiert oder durch ($C_1$-$C_7$)-Alkyl und ($C_1$-$C_7$)-Alkanoyl oder durch zwei ($C_1$-$C_7$)-Alkylreste disubstituiert ist. Der Ausdruck "Heteroaryl" bezeichnet ein- oder mehrkernige heteroaromatische Reste, insbesondere solche, welche als Heteroatom bzw. Heteroatome 1-4 Stickstoffatome und/oder ein Sauerstoff- oder Schwefelatom enthalten, und welche gegebenenfalls substituiert sein können, und zwar beispielsweise durch ($C_1$-$C_7$)-Alkyl, Aryl, Hydroxy, gegebenenfalls N-substituiertes Amino oder Nitro; Beispiele für solche Heteroarylreste sind 2-Pyridyl, 4-Pyridyl, 2-Pyrimidinyl, 1-H-s-Triazol-3-yl, Purin-6-yl, Furyl, 4,6-Dimethyl-2-pyrimidinyl, 5-Amino-1,3,4-thiadiazol-2-yl, 5-Methyl-1,3,4-thiadiazol-2-yl, 4-Methyl-4H-1,2,4-triazol--3-yl, 1-Phenyl-1H-tetrazol-5-yl, 4-Hydroxy-6-propyl-2-pyrimidinyl, 2-Benzimidazolyl, 5-Nitro-2-benzimidazolyl und dergleichen. Der Ausdruck "cysteinhaltiges Oligopeptid" bezeichnet ein 2-10 Aminosäuren enthaltendes Peptid, wobei eine dieser Aminosäuren Cystein ist; ein Beispiele eines solchen Oligopeptids ist das Glutathion (γ-Glutamylcysteinylglycin). Der Ausdruck "Alkanoyl" bezeichnet Reste, wie Acetyl, Propionyl und dergleichen. Der Ausdruck "Aminocarboxyalkyl" umfasst Reste wie 2-Amino-2-carboxyäthyl,

3-Amino-3-carboxyäthyl und 2-Amino-2-carboxy-1,1-dimethyläthyl. Der Ausdruck "Amino-alkoxycarbonyl" umfasst Reste wie 2-Amino-2-äthoxycarbonyläthyl und dergleichen.

Der 5-, 6- oder 7-gliedrige Ring, den zwei benachbarte der Substituenten $R^5$, $R^6$, $R^7$ und $R^8$ gemeinsam und zusammen mit den Kohlenstoffatomen, an welche sie gebunden sind, bilden können, kann heterocyclisch oder carbocyclisch sein, er kann gegebenenfalls eine oder mehrere zusätzliche Doppelbindungen enthalten, wobei er aromatisch oder nicht aromatisch sein kann, und er kann substituiert oder unsubstituiert sein; als Substituenten kommen Hydroxy, $(C_1$-$C_7)$-Alkyl, $(C_1$-$C_7)$-Alkoxy, $(C_2$-$C_7)$-Alkanoyloxy, Oxo, Oximino, $(C_1$-$C_7)$-Alkoxyimino oder dergleichen in Betracht. Wenn ein solcher Ring vorhanden ist, dann bedeuten die beiden übrigen der fraglichen Substituenten zweckmässigerweise je Wasserstoff. Wenn $R^5$ und $R^6$ gemeinsam und zusammen mit den Kohlenstoffatomen, an welche sie gebunden sind, einen solchen Ring bedeuten, dann handelt es sich beispielsweise um einen Thiazol-, Thiadiazol- oder 1,3-Dioxanring. Wenn $R^6$ und $R^7$ gemeinsam und zusammen mit den Kohlenstoffatomen, an welche sie gebunden sind, einen solchen Ring bilden, dann handelt es sich beispielsweise um einen Ring der Formel

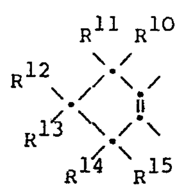
(a)
,
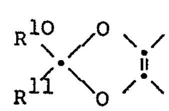
(b)
,
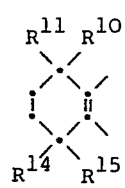
(c)
,
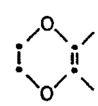
(d)
oder
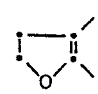
(e)

worin

$R^{10}$, $R^{11}$, $R^{14}$ und $R^{15}$ je Wasserstoff oder $(C_1$-$C_7)$-Alkyl; und

entweder $R^{12}$ Wasserstoff und $R^{13}$ Wasserstoff, Hydroxy, $(C_1-C_7)$-Alkoxy oder $(C_2-C_7)$-Alkanoyloxy oder $R^{12}$ und $R^{13}$ zusammen Oxo, Oximino oder $(C_1-C_7)$-Alkoxyimino bedeuten. Zweckmässigerweise bedeuten dabei $R^{10}$, $R^{11}$, $R^{14}$ und $R^{15}$ entweder alle Wasserstoff oder alle Methyl und $R^{12}$ und $R^{13}$ zusammen Oxo. Besonders hervorgehoben seien die Ringe der obigen Formel (a), (b) und (c).

Wenn keiner der Substituenten $R^5$, $R^6$, $R^7$ und $R^8$ Bestandteil eines Ringes ist, dann bedeuten zweckmässigerweise $R^5$ und $R^8$ je Wasserstoff. Weiterhin bedeutet in einem solchen Fall zweckmässigerweise auch $R^7$ Wasserstoff, wobei $R^6$ zweckmässigerweise Wasserstoff, Methoxy oder Trifluormethyl bedeutet.

$R^1$ kann beispielsweise Wasserstoff oder Methyl bedeuten. $R^2$ kann beispielsweise Wasserstoff, Methyl, t-Butyl, Methoxy, Aethoxy, n-Propoxy oder ein negativ geladenes Sauerstoffatom bedeuten. $R^3$ kann beispielsweise Wasserstoff oder Methyl bedeuten. Wenn A einen Rest der Formel $SR^9$ bedeutet, dann kann $R^9$ beispielsweise Methyl, Aethyl, n-Propyl, i-Propyl, s-Butyl, t-Butyl, n-Pentyl, n-Hexyl, n-Octyl, t-Octyl, n-Dodecyl, n-Hexadecyl, n-Octadecyl, Allyl, 2-Chlorallyl, Cyclopentyl, Cyclohexyl, 2-Hydroxyäthyl, Methoxycarbonylmethyl, Aethoxycarbonylmethyl, 1,2-Dicarboxyäthyl, 1,2-Bis-(äthoxycarbonyl)äthyl, Carboxymethyl, 2-Carboxyäthyl, 1-Carboxyäthyl, 2-Amino-2-carboxyäthyl, 2-Amino-2-äthoxycarbonyläthyl, 3-Amino-3-carboxypropyl, 2-Amino-2-carboxy-1,1-dimethyläthyl, Phenyl, p-Tolyl, m-Chlorphenyl, o-Carboxyphenyl, p-Fluorphenyl, p-Chlorphenyl, m-Methoxyphenyl, Pentafluorphenyl, o-Tolyl, o-Chlorphenyl, m-Fluorphenyl, p-Methoxyphenyl, o-Methoxyphenyl, 2,6-Dichlorphenyl, 2,5-Dichlorphenyl, 2,3-Dichlorphenyl, 2,4-Dichlorphenyl, 3,4-Dichlorphenyl, o-Methoxycarbonylphenyl, p-Nitrophenyl, m-Bromphenyl, p-Bromphenyl, m-Aminophenyl, o-Aminophenyl, p-Acetylaminophenyl, m-Trifluormethylphenyl, Benzyl, o-Nitrobenzyl, p-Fluorbenzyl, p-Methoxybenzyl, m-Trifluormethylbenzyl, 2-Phenyläthyl, o-Chlorbenzyl, p-Chlorbenzyl, m-Nitrobenzyl, 3,4-Dichlorbenzyl, 2,4-Dichlorbenzyl, Triphenylmethyl, 2-Pyridyl, 2-Pyrimidinyl, 5-Amino-1,3,4-thiadiazol-2-yl, 1H-s-Triazol-3-yl, Purin-6-yl, 4,6-Dimethyl-2-pyrimidinyl, Furfuryl, 5-Methyl-1,3,4-thiadiazol-2-yl, 4-Methyl-4H--1,2,4-triazol-3-yl, 1-Phenyl-1H-tetrazol-5-yl, 4-Hydroxy--6-propyl-2-pyrimidinyl, 2-Benzimidazolyl, 4-Pyridyl, 5-Nitro-2-benzimidazolyl, 2-(4-Amino-4-carboxy-butyramido)-2-(carboxymethylcarbamoyl)äthyl, 1-[(Carboxymethyl)carbamoyl]äthyl, 2-Aminoäthyl oder 2-Dimethylaminoäthyl bedeuten; A kann aber zweckmässigerweise auch einen Rest der Formel $-SO_3^-$ oder $-S-SO_3^-$ bedeuten.

Unter den Verbindungen der eingangs definierten Formel I sind diejenigen bevorzugt, worin $R^1$ $(C_1-C_7)$-Alkyl, $R^2$ $(C_1-C_7)$-Alkoxy, $R^3$ Wasserstoff oder $(C_1-C_7)$--Alkyl, A einen Rest der Formel $-SR^9$, $R^9$ n-Propyl, 2-Amino-2-carboxyäthyl, 2-(4-Amino-4-carboxybutyramido)-2-(carboxymethylcarbamoyl)äthyl, n-Hexyl, o-Chlorphenyl, Aethyl, 2-Aminoäthyl, m-Chlorphenyl, 1,2-Bis-(äthoxycarbonyl)äthyl, 2-Amino-2-äthoxycarbonyläthyl, 2-Hydroxyäthyl, 3-Amino-3-carboxypropyl, Cyclopentyl, 2-Dimethylaminoäthyl, o-Carboxyphenyl, Isopropyl, 2-Pyrimidinyl, p-Fluorphenyl, 2-Carboxyäthyl, 1-Carboxyäthyl, p-Chlorphenyl, 3-Furfuryl, n-Pentyl, 2-Chlorallyl, o-Nitrobenzyl, 3,4-Dichlorphenyl, p-Methoxyphenyl, 5-Nitro-2-benzimidazolyl, 1-[(Carboxymethyl)carbamoyl]äthyl oder o-Chlorphenyl, $R^5$ und $R^8$ je Wasserstoff und entweder $R^6$ Trifluormethyl und $R^7$ Wasserstoff oder $R^6$ und $R^7$ zusammen mit den Kohlenstoffatomen, an welche sie gebunden sind, einen Rest der oben definierten Formel (a) bedeuten; speziell bevorzugt sind dabei diejenigen dieser Verbindungen, worin $R^1$ Methyl, $R^2$ Methoxy und $R^3$ Wasserstoff oder Methyl bedeuten und worin, wenn $R^6$ und $R^7$ zusammen mit den Kohlenstoffatomen, an welche sie gebunden sind, einen Rest der oben definierten

Formel (a) bedeuten; $R^{12}$ und $R^{13}$ zusammen Oxo und $R^{10}$, $R^{11}$, $R^{14}$ und $R^{15}$ alle Methyl bedeuten.

Ganz besonders bevorzugte Verbindungen der Formel I sind:

2-[(Propyldithio)methyl]-4-methoxy-3-methyl-1-[5-(trifluormethyl)-2-benzimidazolyl]pyridiniumchlorid;

2-[[[(R)-2-Amino-2-carboxyäthyl]dithio]methyl-4-methoxy-3-methyl-1-[5-(trifluormethyl)-2-benzimidazolyl]pyridiniumchlorid; und

2-[[[(R)-2-[(S)-4-Amino-4-carboxybutyramido]-2-[(carboxymethyl)carbamoyl]äthyl]dithio]methyl]-4-methoxy-3-methyl-1-[(5-trifluormethyl)-2-benzimidazolyl]pyridiniumchlorid.

Weitere besonders bevorzugte Verbindungen der Formel I sind:

2-[(Hexyldithio)methyl]-4-methoxy-3-methyl-1-[5-(trifluormethyl)-2-benzimidazolyl]pyridiniumchlorid;

2-[[(o-Chlorphenyl)dithio]methyl]-4-methoxy-3-methyl-1-[5-(trifluormethyl)-2-benzimidazolyl]pyridiniumchlorid;

2-[(Aethyldithio)methyl]-4-methoxy-3-methyl-1-[5-(trifluormethyl)-2-benzimidazolyl]pyridiniumchlorid;

2-[[(2-Aminoäthyl)dithio]methyl]-4-methoxy-3-methyl-1-[5-(trifluormethyl)-2-benzimidazolyl]pyridiniumchlorid;

2-[[(m-Chlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[[(R)-2-Amino-2-carboxyäthyl]dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[[1,2-Bis-(äthoxycarbonyl)äthyl]dithio]methyl]-4-methoxy-3-methyl-1-[5-(trifluormethyl)-2-benzimidazolyl]-pyridiniumchlorid;

2-[(Aethyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

4-Methoxy-3-methyl-2-[(propyldithio)methyl]-1-(1,5,6,7-tetrahydro-5,5,7,7-tetramethyl-5-oxoindeno[5,6-d]imidazol-2-yl)pyridiniumchlorid;

2-[[[(R)-2-Amino-2-(äthoxycarbonyl)äthyl]dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

intramolekular deprotonisiertes 3-[[[2-(Dimethylaminoäthyl]dithio]methyl]-4-methoxy-3-methyl-1-(1,5,6,7-tetrahydro-5,5,7,7-tetramethyl-6-oxoindeno[5,6-d]-imidazol-2-yl-pyridiniumkation;

2-[[(2-Hydroxyäthyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[[(RS)-3-Amino-3-carboxypropyl]dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid; und

2-[(Cyclopentyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid.

Ebenfalls bevorzugte Verbindungen der Formel I sind beispielsweise:

2-[[(o-Carboxyphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[(Isopropyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[(2-Pyrimidinyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Fluorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(2-Carboxyäthyl)dithio]methyl]-4-methoxy-3-methyl-1-

(1,5,6,7-tetrahydro-5,5,7,7-tetramethyl-5-oxoindeno[5,6-d]imidazol-2-yl)pyridiniumchlorid;

2-[[(1-Carboxyäthyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Chlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[(Hexyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

intramolekular deprotonisiertes 2-[(3-Furfuryldithio)-methyl]-4-methoxy-3-methyl-1-(1,5,6,7-tetrahydro-5,5,7,7-tetramethyl-6-oxoindeno[5,6-d]imidazol-2-yl)pyridiniumkation;

4-Methoxy-3-methyl-2-[(pentyldithio)methyl]-1-(1,5,6,7-tetrahydro-5,5,7,7-tetramethyl-5-oxoindeno[5,6-d]imidazol-2-yl)pyridiniumchlorid;

2-[[(2-Chlorallyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

4-Methoxy-3-methyl-2-[[(o-nitrobenzyl)dithio]methyl]-1-(1,5,6,7-tetrahydro-5,5,7,7-tetramethyl-5-oxoindeno[5,6-d]imidazol-2-yl)pyridiniumchlorid;

2-[[(3,4-Dichlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Methoxyphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

4-Methoxy-3-methyl-2-[[(5-nitro-2-benzimidazolyl)dithio]-methyl]-1-(1,5,6,7-tetrahydro-5,5,7,7-tetramethyl-5-oxoin-deno[5,6-d]imidazol-2-yl)pyridiniumchlorid;

2-[[[-1-[(Carboxymethyl)carbamoyl]äthyl]dithio]methyl-4-methoxy-3-methyl-1-(1,5,6,7-tetrahydro-5,5,7,7-tetramethyl-5-oxoindeno[5,6-d]imidazol-2-yl)pyridiniumchlorid; und

2-[[(o-Chlorphenyl)dithio]methyl]-4-methoxy-3,5-dimethyl-1-(1,5,6,7-tetrahydro-5,5,7,7-tetramethyl-5-oxoindeno[5,6-d]imidazol-2-yl)pyridiniumchlorid.

Die neuen Benzimidazol-2-yl-pyridiniumverbindungen der eingangs definierten Formel I und deren Salze können erfindungsgemäss dadurch hergestellt werden, dass man

a) eine Verbindung der allgemeinen Formel

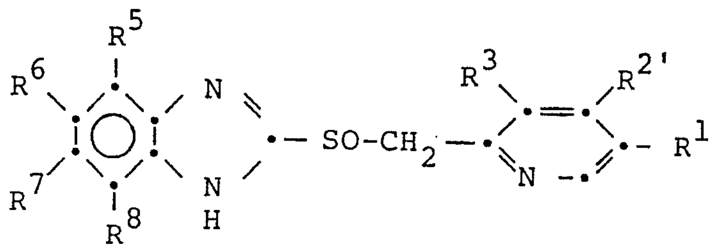

II

worin $R^1$, $R^3$, $R^5$, $R^6$, $R^7$ und $R^8$ die eingangs erwähnte Bedeutung besitzen und $R^{2'}$ Wasserstoff, $(C_1-C_7)$-Alkyl oder $(C_1-C_7)$-Alkoxy bedeutet,

in Gegenwart von Säure mit einer Verbindung der allgemeinen Formel

$$HS-R^9 \qquad III$$

worin $R^9$ die eingangs erwähnte Bedeutung besitzt,

oder mit $SO_2$ oder mit einem Alkalimetallthiosulfat umsetzt; oder

b) in einer Verbindung der eingangs definierten allgemeinen Formel I, worin $R^2$ $(C_1-C_7)$-Alkoxy bedeutet, die durch $R^2$ bezeichnete $(C_1-C_7)$-Alkoxygruppe spaltet oder durch eine andere $(C_1-C_7)$-Alkoxygruppe ersetzt; oder

c) in einer Verbindung der eingangs definierten allgemeinen Formel I, worin A einen Rest der Formel $-SR^9$ bedeutet und $R^9$ eine oder mehrere Carboxylgruppe(n) enthält, diese Carboxylgruppe(n) in eine bzw. mehreren $(C_1-C_7)$-

-Alkoxycarboxylgruppe(n) überführt; oder

d) in einer Verbindung der eingangs definierten allgemeinen Formel I, worin A einen Rest der Formel $-SR^9$ bedeutet und $R^9$ eine oder mehrere $(C_1-C_7)$-Alkoxycarbonylgruppe(n) enthält, diese $(C_1-C_7)$-Alkoxycarbonylgruppe(n) zu einer bzw. mehreren Carboxylgruppe(n) hydrolysiert;

worauf man das erhaltene Produkt als Salz oder inneres Salz isoliert und erwünschtenfalls ein basisches Produkt in ein pharmazeutisch annehmbares Säureadditionssalz überführt.

Die erfindungsgemässe Umsetzung der Verbindungen der Formeln II und III erfolgt zweckmässigerweise in verdünnnter wässriger Salzsäure, gegebenenfalls unter Zusatz eines geeigneten, mit Wasser mischbaren organischen Lösungsmittels, wie Methanol, Tetrahydrofuran, Dimethoxyäthan u.dgl., oder in einer Lösung von Chlorwasserstoff in einem geeigneten organischen Lösungsmittel, wie z.B. Methanol, Tetrahydrofuran, Dimethoxyäthan u.dgl. Anstelle von Salzsäure bzw. Chlorwasserstoff kommen auch andere Säuren in Betracht, beispielsweise verd. Schwefelsäure, Bromwasserstoffsäure, Phosphorsäure, Trifluoressigsäure, Methansulfonsäure u.dgl. Die Umsetzung erfolgt vorzugsweise bei etwa Raumtemperatur, kann aber ohne weiteres auch bei Temperaturen unterhalb oder oberhalb der Raumtemperatur durchgeführt werden, zweckmässigerweise bei Temperaturen zwischen etwa 0°C und etwa 60°C.

Bei der erwähnten Umsetzung der Verbindungen der Formeln II und III in Gegenwart von Säure erhält man entsprechende Verbindungen der Formel I, worin A einen Rest der Formel $SR^9$ bedeutet. In J. Biol. Chem. 260, 4591-4597 (1985) wird die Umsetzung von Omeprazol (Formel II: $R^1$ und $R^3$ = $CH_3$; $R^{2'}$ und $R^6$ = $OCH_3$; $R^5$, $R^7$ und $R^8$ = H) und von Timoprazol (Formel II: $R^1$, $R^{2'}$, $R^3$, $R^5$, $R^6$, $R^7$ und $R^8$ = H) mit ß-Mercaptoäthanol, Aethylmercaptan und Benzylmercaptan in Gegenwart von Salzsäure beschrieben; allerdings sind dort für die Endprodukte weder die korrekten Strukturformeln noch irgendwelche pharmakodynamischen Eigenschaften erwähnt.

Für die erfindungsgemässe Umsetzung der Verbindungen der Formel II mit $SO_2$ kann man gasförmiges $SO_2$ verwenden. Zweckmässigerweise wird jedoch das $SO_2$ in situ erzeugt, beispielsweise aus Thionylchlorid oder aus einem geeigneten Salz der schwefligen Säure, wie Natriumsulfit oder Natriumhydrogensulfit. Man kann beispielsweise so vorgehen, dass man Wasser vorlegt, dann Thionylchlorid und anschliessend eine Verbindung der Formel II zugibt, worauf man das Reaktionsgemisch während kurzer Zeit (etwa 10 Minuten) leicht erwärmt. Die Umsetzung von Verbindungen der Formel II mit $SO_2$ liefert entsprechende Verbindungen der Formel I, worin A einen Rest der Formel $-SO_3^-$ bedeutet.

Die erfindungsgemässe Umsetzung einer Verbindung der Formel II mit einem Alkalimetall-thiosulfat erfolgt in wässriger Säure, beispielsweise in verdünnter wässriger Salzsäure, wobei man als Alkalimetall-thiosulfat beispielsweise Natriumthiosulfat-pentahydrat verwenden kann. Anstelle von Salzsäure kann auch eine andere Säure verwendet werden, beispielsweise Bromwasserstoffsäure, verd. Schwefelsäure, Phosphorsäure, Trifluoressigsäure, Methansulfonsäure u.dgl. Anstelle von Natriumthiosulfat-pentahydrat können auch andere geeignete Alkalimetall-thiosulfate verwendet werden, beispielsweise Kaliumthiosulfat. Die Umsetzung erfolgt zweckmässigerweise bei Raumtemperatur, kann aber auch bei Temperaturen oberhalb und unterhalb der Raumtemperatur durchgeführt werden, beispielsweise bei 0°C bis 60°C. Die erfindungsgemässe Umsetzung von Verbindungen der Formel II mit einem Alkalimetall-thiosulfat liefert entsprechende Verbindungen der Formel I, worin A einen Rest der Formel $-S-SO_3^-$ bedeutet.

In Verbindungen der Formel I, worin $R^2$ $(C_1-C_7)$--Alkoxy bedeutet, kann die durch $R^2$ bezeichnete $(C_1-$-$C_7)$-Alkoxygruppe erfindungsgemäss gespalten oder durch eine andere $(C_1-C_7)$-Alkoxygruppe ersetzt werden. Die Spaltung der Alkoxygruppe erfolgt beispielsweise durch längeres Erhitzen mit wässriger Salzsäure, wobei die Temperatur etwa 40°C bis 90°C und die Reaktionszeit 1 h bis 2 Wochen beträgt. Anstelle von Salzsäure können aber auch andere geeignete Säuren verwendet werden, beispielsweise Methansulfonsäure u.dgl. Auf diese Weise erhält man Verbindungen der Formel I, worin $R^2$ ein negativ geladenes Sauerstoffatom bedeutet, welche auch als Pyridon-Derivate mit der weiter oben angegebenen Partialstruktur der Formel Ia aufgefasst werden können.

Der erfindungsgemässe Austausch einer durch $R^2$ bezeichneten $(C_1-C_7)$-Alkoxygruppe durch eine andere $(C_1-$-$C_7)$-Alkoxygruppe kann durch längeres Stehenlassen in dem der gewünschten $(C_1-C_7)$-Alkoxygruppe entsprechenden $(C_1-C_7)$-Alkanol erzielt werden, was bei Raumtemperatur erfolgen kann, wobei aber auch Temperaturen oberhalb und unterhalb der Raumtemeratur verwendet werden können, beispielsweise Temperaturen von etwa 0°C bis etwa 60°C. Je nach der Temperatur beträgt die Reaktionszeit 24 h bis 1 Monat; arbeitet man bei Raumtemperatur, so ist eine Reaktionszeit von etwa 1 bis etwa 4 Wochen, zweckmässigerweise etwa 2 Wochen, angezeigt.

Die erfindungsgemässe Veresterung einer Verbindung der Formel I, worin A einen Rest der Formel $-SR^9$ bedeutet und $R^9$ eine oder mehrere Carboxylgruppe(n) enthält, kann beispielsweise dadurch erfolgen, dass man zunächst ein entsprechendes reaktionsfähiges Carbonsäurederivat herstellt (z.B. ein entsprechendes Säurechlorid oder dergleichen, was mittels Thionylchlorid oder dergleichen bewerkstelligt werden kann) und dieses Derivat dann durch Behandlung mit dem entsprechenden Alkohol in den entsprechenden Ester

überführt. Die Veresterung kann aber auch anlässlich der Umsetzung einer Verbindung der Formel II mit einer Verbindung der Formel III, worin $R^9$ eine oder mehrere Carboxylgruppe(n) enthält, stattfinden, nämlich dann, wenn man in Gegenwart von im entsprechenden Alkohol gelöster Säure in Abwesenheit von Wasser arbeitet. Wird beispielsweise eine Verbindung der Formel II in Gegenwart von äthanolischer Salzsäure mit Thioäpfelsäure umgesetzt, so erhält man eine entsprechende Verbindung der Formel I, worin A einen Rest der Formel $-SR^9$ und $R^9$ 1,2-Bis-(äthoxycarbonyl)äthyl bedeuten.

Die erfindungsgemässe Hydrolyse einer Verbindung der Formel I, worin A einen Rest der Formel $-SR^9$ bedeutet und $R^9$ eine oder mehrere $(C_1-C_7)$-Alkoxycarbonylgruppe(n) enthält, kann beispielsweise unter sauren Bedingungen erfolgen, z.B. mittels wässeriger Salzsäure. Auch diese Esterhydrolyse kann anlässlich der Umsetzung der Verbindung der Formel II mit einer Verbindung der Formel III erfolgen, nämlich dann, wenn $R^9$ in Formel III eine oder mehrere $(C_1-C_7)$-Alkoxycarbonylgruppe(n) enthält und in Gegenwart von wässeriger Säure gearbeitet wird. Wird beispielsweise eine Verbindung der Formel II in Gegenwart von wässeriger Salzsäure mit Thioglykolsäureäthylester umgesetzt, so kann man eine entsprechende Verbindung der Formel I erhalten, worin A einen Rest der Formel $-SR^9$ und $R^9$ Carboxymethyl bedeuten.

Je nach der Natur der Ausgangsprodukte und den verwendeten Reaktionsbedingungen können die erhaltenen Produkte als Salze oder als innere Salze isoliert werden. Erwünschtenfalls können basisch substituierte Produkte in pharmazeutisch annehmbare Säureadditionssalze übergeführt werden, beispielsweise mit Chlorwasserstoff, Bromwasserstoff, Phosphorsäure, Schwefelsäure, Zitronensäure, p-Toluolsulfonsäure und dergleichen.

Die Ausgangsprodukte der Formel II sind bekannt oder nach an sich bekannten und jedem Fachmann geläufigen Methoden leicht herstellbar; zudem enthalten einige der nachfolgenden Beispiele detaillierte Angaben betreffend die Herstellung bestimmter Verbindungen der Formel II.

Wie eingangs erwähnt, besitzen die Benzimidazol-2-yl--pyridiniumverbindungen der allgemeinen Formel I wertvolle pharmakodynamische Eigenschaften.

Repräsentative Verbindungen der Formel I wurden auf ihre Antiulcuswirkung, auf ihre magensäuresekretionshemmende Wirkung sowie auf ihre Toxizität untersucht.

Zur Bestimmung der Antiulcuswirkung wurde die nachstehend beschriebene Versuchsanordnung verwendet:

Für jede Dosis einer Testsubstanz verwendet man Gruppen von je 8 männlichen Ratten mit einem Körpergewicht von 130-150 g. Vor Beginn des Versuchs erhalten die Tiere während 24 Stunden keine Nahrung, jedoch Wasser ad libitum. Verschiedene Dosen der zu prüfenden Substanzen (suspendiert in 0,5% Tragacanth) oder das Vehikel allein (Kontrolle) werden zweimal peroral verabreicht, und zwar 1 Stunde bevor und 2 Stunden nach peroraler Verabreichung von 20 mg/kg Indomethacin. Bei Kontrolltieren führt diese Dosis von Indomethacin innerhalb von 5 Stunden zu Läsionen des Magens. 6 Stunden nach der ersten Verabreichung der zu untersuchenden Substanz (bzw. des Vehikels allein) werden die Tiere getötet. Die Ratten, welche vor dem Auftreten makroskopisch sichtbarer Läsionen der Magenschleimhaut geschützt geblieben sind, werden gezählt. Als $ED_{50}$ bezeichnet man diejenige Dosis einer Testsubstanz, bei welcher 50% der Tiere vor dem Auftreten solcher Läsionen geschützt sind.

Zur Bestimmung der magensäuresekretionhemmenden Wirkung wurde die nachstehend beschriebene Versuchsanordnung verwendet:

Weiblichen und männlichen Beagle-Hunden wird ein Teil des Magenfundus vom restlichen Magen in Form einer Tasche vom Heidenhain-Typ abgetrennt (Modifikation der von Rudick et al. in J. Surgical Research 7, 383-398 (1967) beschriebenen Methode). In die Tasche wird eine Stahlkanüle eingenäht, welche durch die Bauchdecke nach aussen geleitet wird. Vor jedem Versuch erhalten die Tiere während 18 Stunden keine Nahrung, jedoch Wasser ad libitum. Während des Versuchs sind sie wach und stehen, und ihre Magensäuresekretion wird durch intravenöse Infusion von 4-Methylhistamin, einem selektiven Agonisten der Histamin-$H_2$-Rezeptoren, stimuliert. Die Magensäureproduktion wird in 15-Minuten-Fraktionen des Magentaschensaftes bestimmt. Sobald die Magensäureproduktion einen konstanten Wert aufweist, werden die zu prüfenden Substanzen, als trockene Pulver in Gelatinekapseln gefüllt, oral verabreicht. Als $ED_{50}$ bezeichnet man diejenige Dosis einer Testsubstanz, welche bei den behandelten Tieren im Vergleich zu Kontrolltieren eine 50%ige Hemmung der durch 4-Methylhistamin hervorgerufenen Magensäureproduktion bewirkt.

In der nachfolgenden Tabelle werden für eine Reihe repräsentativer Verbindungen der Formel I die Resultate der Prüfung auf ihre Antiulcuswirkung und auf ihre magensekretionshemmende Wirkung wiedergegeben. Ausserdem enthält diese Tabelle Angaben über die akute Toxizität ($DL_{50}$ bei einmaliger oraler Verabreichung an Mäuse).

0214479

| Verbindung | Anti-Ulcus, $ED_{50}$ mg/kg p.o. | Magensäuresekretionshemmung, $ED_{50}$ mg/kg p.o. | Toxizität, DL 50 mg/kg p.o. |
|---|---|---|---|
| A | 1,2 | 1,5 | >5000 |
| B | 2,5 | 2,8 | 1250-2500 |
| C | 3,0 | 3,0 | 1250-2500 |
| D | 1,9 | 6,8 | >5000 |
| E | 2,1 | 6,0 | >5000 |
| F | 1,7 | 1,6 | 1250-2500 |
| G | 2,3 | 1,5 | 312- 625 |
| H | 1,8 | 6,2 | 1250-2500 |
| I | 2,3 | 3,9 | 312- 625 |
| J | 2,4 | 2,2 | 1250-2500 |
| K | 2,3 | 6,5 | 312- 625 |
| L | 1,6 | 3,4 | 312- 625 |
| M | 1,6 | 4,6 | 312- 625 |
| N | 1,5 | 3,7 | 312- 625 |
| O | 2,3 | 2,9 | 312- 625 |
| P | 1,9 | 4,1 | 312- 625 |
| Q | 1,6 | 4,9 | 312- 625 |

A = 2-[(Propyldithio)methyl]-4-methoxy-3-methyl-1-[5-(trifluormethyl)-2-benzimidazolyl]pyridiniumchlorid

B = 2-[[[(R)-2-Amino-2-carboxyäthyl]dithio]methyl-4-methoxy-3-methyl-1-[5-(trifluormethyl)-2-benzimidazolyl]-pyridiniumchlorid

C = 2-[[[(R)-2-[(S)-4-Amino-4-carboxybutyramido]-2-[(carboxy-methyl)carbamoyl]äthyl]dithio]methyl]-4-methoxy-3-methyl-1-[(5-trifluormethyl)-2-benzimidazolyl]pyridiniumchlorid

D = 2-[(Hexyldithio)methyl]-4-methoxy-3-methyl-1-[5-(trifluormethyl)-2-benzimidazolyl]pyridiniumchlorid

E = 2-[[(o-Chlorphenyl)dithio]methyl]-4-methoxy-3-methyl-1-[5-(trifluormethyl)-2-benzimidazolyl]pyridiniumchlorid

F = 2-[(Aethyldithio)methyl]-4-methoxy-3-methyl-1-[5-(trifluormethyl)-2-benzimidazolyl]pyridiniumchlorid

G = 2-[[(2-Aminoäthyl)dithio]methyl]-4-methoxy-3-methyl-1-[5-(trifluormethyl)-2-benzimidazolyl]pyridiniumchlorid

H = 2-[[(m-Chlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid

I = 2-[[[(R)-2-Amino-2-carboxyäthyl]dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid

J = 2-[[[1,2-Bis-(äthoxycarbonyl)äthyl]dithio]methyl]-4-methoxy-3-methyl-1-[5-(trifluormethyl)-2-benzimidazolyl]-pyridiniumchlorid

K = 2-[(Aethyldithio)methyl]-4-methoxy-3-methyl-1-(1,5,6,7-tetrahydro-5,5,7,7-tetramethyl-5-oxoindeno[5,6-d]-imidazol-2-yl)pyridiniumchlorid

L = 4-Methoxy-3-methyl-2-[(propyldithio)methyl]-1-(1,5,6,7-tetrahydro-5,5,7,7-tetramethyl-5-oxoindeno[5,6-d]-imidazol-2-yl)pyridiniumchlorid

M = 2-[[[(R)-2-Amino-2-(äthoxycarbonyl)äthyl]dithio]-methyl]-4-methoxy-3-methyl-1-(1,5,6,7-tetrahydro-5,5,7,7-tetramethyl-5-oxoindeno[5,6-d]imidazol-2-yl)pyridiniumchlorid

N = Intramolekular deprotonisiertes 3-[[[2-(Dimethylaminoäthyl]dithio]methyl]-4-methoxy-3-methyl-1-(1,5,6,7-tetra-hydro-5,5,7,7-tetramethyl-6-oxoindeno[5,6-d]-imidazol-2-yl-pyridiniumkation

O = 2-[[(2-Hydroxyäthyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid

P = 2-[[[(RS)-3-Amino-3-carboxypropyl]dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid

Q = 2-[(Cyclopentyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid.

Die eingangs definierten Verbindungen und Salze können als Heilmittel, z.B. in Form pharmazeutischer Präparate Verwendung finden. In erster Linie kommt orale Verabreichung in Form von festen pharmazeutischen Präparaten, wie Tabletten, Lacktabletten, Dragées, Hartgelatinekapseln und Weichgelatinekapseln in Frage. Orale Verabreichung in Form flüssiger pharmazeutischer Präparate, wie Lösungen, Emulsionen und Suspensionen, rektale Verabreichung, z.B. in Form von Suppositorien, oder parenterale Verabreichung, z.B. in Form von Injektionslösungen, sind zwar weniger in Betracht zu ziehen aber auch nicht auszuschliessen.

Arzneimittel, enthaltend eine der eingangs definierten Verbindungen und Salze, sind ebenfalls Gegenstand der vorliegenden Erfindung. Die Herstellung derartiger Arzneimittel kann dadurch erfolgen, dass man eine oder mehrere der eingangs definierten Verbindungen und Salze und erwünschtenfalls einen oder mehrere andere therapeutische Wirkstoffe zusammen mit einem oder mehreren therapeutisch inerten Excipientien in eine galenische Darreichungsform bringt.

Zur Herstellung von Tabletten, Lacktabletten, Dragées und Hartgelatinekapseln können die eingangs definierten Verbindungen und Salze mit pharmazeutisch inerten, anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze etc. verwenden. Zur Herstellung von magensaftresistenten pharmazeutischen Präparaten ist noch ein magensaftresistenter Lack aufzubringen, welcher z.B. aus Hydroxypropylmethylcellulosephthalat bestehen kann.

Für Weichgelatinekapseln eignen sich als Excipientien z.B. vegetabile Oele, Wachse, Fette, halbfeste und flüssige

Polyole etc.

Zur Herstellung von Lösungen und Sirupen eignen sich als Excipientien z.B. Wasser, Polyole, Saccharose, Invertzucker, Glucose und dgl.

Für Suppositorien eignen sich als Excipientien z.B. natürliche oder gehärtete Oele, Wachse, Fette, halbflüssige oder flüssige Polyole und dgl.

Für Injektionslösungen eignen sich als Excipientien z.B. Wasser, Alkohole, Polyole, Glycerin, vegetabile Oele etc.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die eingangs definierten Verbindungen und Salze bei der Bekämpfung bzw. Verhütung von Krankheiten verwenden, beispielsweise bei der Bekämpfung bzw. Verhütung von Ulcus ventriculi und duodeni. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 30-400 mg und bei intravenöser Verabreichung eine Tagesdosis von etwa 1-50 mg angemessen sein.

Gegenstand der Erfindung ist auch die Verwendung der eingangs definierten Verbindungen und Salze zur Herstellung von Arzneimitteln für die Bekämpfung bzw. Verhütung von Ulcus ventriculi und duodeni.

In den nachfolgenden Beispielen, welche die vorliegende Erfindung illustrieren, ihren Umfang aber in keiner Weise beschränken sollen, sind sämtliche Temperaturen in Celsiusgraden angegeben.

Beispiel 1

a) Eine Lösung von 24 g (0,22 Mol) 2,3-Dimethylpyridin in 100 ml Methylenchlorid wird unter Eiskühlung mit einer Lösung von 46,6 g (0,27 Mol) m-Chlorperbenzoesäure in 100 ml Methylenchlorid versetzt. Man erhitzt das Reaktionsgemisch 2 Stunden unter Rückfluss und engt im Rotationsverdampfer ein. Der Rückstand wird an Silicagel mit Essigester/Methylenchlorid (3:1) als Elutionsmittel chromatographiert, wobei die Methode der Mitteldruck-Flashchromatographie verwendet und der Druck mit Stickstoffgas erzeugt wird. Durch Umkristallisation aus Aether erhält man 2,3-Dimethylpyridin-1-oxid vom Schmelzpunkt 56°.

b) Eine Lösung von 15 g (0,12 Mol) 2,3-Dimethylpyridin-1--oxid in 75 ml Chloroform wird am Rückfluss gekocht und so schnell wie möglich mit 37 ml Trichloressigsäurechlorid versetzt (es ist vorteilhaft, das Säurechlorid durch den Rückflusskühler zuzugeben). Man erhitzt das Reaktionsgemisch 2,5 Stunden unter Rückfluss, giesst es anschliessend auf ein Gemisch von Eis und Natriumbicarbonat und wäscht die entstandene Lösung mehrmals mit Methylenchlorid. Die organische Phase wird mit Natriumsulfat getrocknet, filtriert und eingeengt. Der Rückstand wird an Silicagel mit Methylenchlorid chromatographiert, wobei die Methode der Mitteldruck-Flashchromatographie verwendet und der Druck mit Stickstoffgas erzeugt wird. Das erhaltene 2-Chlormethyl-3-methylpyridin wird direkt weiterverarbeitet.

c) Eine Lösung von 24 g (0,17 Mol) 2-Chlormethyl-3-methylpyridin in 200 ml Methylenchlorid wird unter Eiskühlung mit einer Lösung von 44 g (0,25 Mol) m-Chlorperbenzoesäure in 200 ml Methylenchlorid versetzt. Man erhitzt das Reaktionsgemisch 2 Stunden unter Rückfluss und engt im Rotationsverdampfer ein. Der Rückstand wird an Silicagel mit Essigester/Methylenchlorid (3:1) als Elutionsmittel chromatographiert, wobei die Methode der Mitteldruck-Flashchromatographie verwendet und der Druck mit Stickstoffgas erzeugt wird. Das erhaltene 2-Chlormethyl-3-methyl-pyridin-1-oxid wird direkt weiterverarbeitet.

d) Zu 230 ml konzentrierter Salpetersäure (68%ig; d = 1,41) gibt man unter Trockeneiskühlung langsam 300 ml konzentrierte Schwefelsäure zu, wobei die Temperatur des Gemisches 5° nicht übersteigt. Man gibt eine Lösung von 38,7 g (0,25 Mol) 2-Chlormethyl-3-methylpyridin-1-oxid zu und rührt während 2 Stunden bei 80°. Das Reaktionsgemisch wird auf ein Gemisch von Eis und Methylenchlorid gegossen, die wässerige Phase wird mehrmals mit Methylenchlorid gewaschen, und die Methylenchloridlösung wird mit 10%iger Natriumbicarbonatlösung extrahiert. Die organische Phase wird mit Natriumsulfat getrocknet und eingeengt. Der Rückstand wird aus Essigester umkristallisiert, wobei man 2-Chlormethyl-3-methyl-4-nitropyridin-1-oxid erhält. Das Produkt zeigt einen Schmelzpunkt von 126-129°.

e) Eine Lösung von 4,5 g (0,024 Mol) 2-Chlormethyl-3--methyl-4-nitropyridin-1-oxid in 25 ml Methylenchlorid und 25 ml Acetonitril wird mit 5 ml Phosphortrichlorid versetzt und 20 Minuten bei Raumtemperatur gerührt. Das Reaktionsgemisch wird auf ein Gemisch von Eis und 20 g Natriumcarbonat gegossen, und die entstandene wässerige Lösung wird mehrmals mit Methylenchlorid gewaschen. Die organische Phase wird getrocknet und eingedampft. Das so erhaltene 2-Chlormethyl-3-methyl-4-nitropyridin wird direkt weiterverarbeitet.

f) Eine Lösung von 11,5 g (0,062 Mol) 2-Chlormethyl-3--methyl-4-nitropyridin und 16 g (0,06 Mol) 5,7-Dihydro-2--mercapto-5,5,7,7 -tetramethylindeno[5,6-d]imidazol-6(1H)--on in 200 ml abs. Aceton wird mit 13 g fein gemahlenem Kaliumcarbonat versetzt und bei Raumtemperatur unter Argon 18 Stunden gerührt. Im Vakuum werden 100 ml Aceton abdestilliert, worauf der Rest auf Eis gegossen wird. Das

auskristallisierte Produkt wird abfiltriert und in Methylenchlorid gelöst; die erhaltene Lösung wird mit Wasser gewaschen, getrocknet und eingeengt. Durch Umkristallisation aus Essigester/Aether erhält man 5,7-Dihydro-5,5,7,7--tetramethyl-2-[[(3-methyl-4-nitro-2-pyridyl)methyl]thio]indeno[5,6-d]imidazol-6(1H)-on vom Schmelzpunkt 181-183° (Zersetzung).

g) Eine Lösung von 4,4 g (0,011 Mol) 5,7-Dihydro-5,5,7,7--tetramethyl-2-[[(3-methyl-4-nitro -2-pyridyl)methyl]thio]-indeno[5,6-d]imidazol]-6(1H)-on in 100 ml abs. Methanol wird mit 3 g Natriummethylat versetzt, worauf während 18 Stunden unter Argon am Rückfluss gekocht wird. Nach Einengen des Reaktionsgemisches im Vakuum wird der Rückstand mit Methylenchlorid versetzt, worauf man mittels Eisessig puffert; die Methylenchloridphase wird mehrmals mit einer Natriumbicarbonatlösung extrahiert, getrocknet und eingedampft. Durch Umkristallisation aus Essigester erhält man 5,7-Dihydro-2-[[(4-methoxy-3-methyl-2-pyridyl)-methyl]thio] -5,5,7,7-tetramethylindeno[5,6-d]imidazol--6(1H)-on vom Schmelzpunkt 222-226°.

h) Eine Lösung von 6 g (0,015 Mol) 5,7-Dihydro-2-[[(4--methoxy-3-methyl-2-pyridyl)methyl]thio] -5,5,7,7-tetramethylindeno[5,6-d]imidazol-6(1H)-on in 90 ml abs. Methylenchlorid wird unter Argon bei -40 bis -50° innerhalb von 10 Minuten mit einer Lösung von 3,3 g (0,019 Mol) m-Chlorperbenzoesäure in 50 ml abs. Methylenchlorid versetzt. Anschliessend wird die Lösung noch zusätzlich 20 Minuten gerührt, mit einer 10%igen Natriumcarbonatlösung extrahiert, getrocknet und unter stetigem Ersatz von Methylenchlorid durch Essigester eingedampft. Dabei kristallisiert 5,7-Dihydro-2-[[(4-methoxy-3-methyl-2-pyridyl)methyl]sulfinyl]-5,5,7,7-tetramethylindeno[5,6-d]imidazol-6(1H)-on, welches bei 192-194° unter Zersetzung schmilzt.

i) Eine Lösung von 0,73 ml n-Propylmercaptan in 30 ml 1N wässeriger Salzsäure wird mit 3 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 4-Methoxy-3-methyl-2-[(propyldithio)-methyl -1-(1,5,6,7-tetrahydro-5,5,7,7-tetramethyl -6-oxo-indeno[5,6-d]imidazol-2-yl)pyridiniumchlorid zeigt einen Schmelzpunkt von 235° (Zersetzung).

## Beispiel 2

Eine Lösung von 1 g Methylmercaptan in 40 ml 1N wässeriger Salzsäure wird mit 6 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 4-Methoxy-3-methyl-2-[(methyldithio)-methyl]-1 -(1,5,6,7-tetrahydro-5,5,7,7-tetramethyl -6-oxo-indeno[5,6-d]imidazol-2-yl)pyridiniumchlorid zeigt einen Schmelzpunkt von 200° (Zersetzung).

## Beispiel 3

Eine Lösung von 680 mg Aethylmercaptan in 30 ml 1N wässeriger Salzsäure wird mit 4 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein

Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[(Aethyldithio)methyl]-4-methoxy-3--methyl-1 -(1,5,6,7-tetrahydro-5,5,7,7-tetramethylindeno [5,6-d]imidazol-2-yl)pyridiniumchlorid zeigt einen Schmelzpunkt von 239° (Zersetzung).

Beispiel 4

Eine Lösung von 0,1 ml Isopropylmercaptan in 20 ml 0,1N wässeriger Salzsäure wird mit 411 mg 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[(Isopropyldithio)methyl]-4-methoxy--3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 150° (Zersetzung).

Beispiel 5

Eine Lösung von 1,2 g sec.Butylmercaptan in 40 ml 1N wässeriger Salzsäure wird mit 5 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[(sec-Butyldithio)methyl]-4-methoxy-

-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 239° (Zersetzung).

## Beispiel 6

Eine Lösung von 1,2 g tert.-Butylmercaptan in 40 ml 1N wässeriger Salzsäure wird mit 5 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[(tert.-Butyldithio)methyl]-4--methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 190° (Zersetzung).

## Beispiel 7

Eine Lösung von 0,7 ml Pentanthiol in 20 ml 1N wässeriger Salzsäure und 20 ml Methanol wird mit 2 g 5,7-Dihydro--2-[[(4-methoxy-3-methyl -2-pyridyl)methyl]sulfinyl]--5,5,7,7-tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit wenig Methanol und Aether gewaschen wird. Das erhaltene 4-Methoxy-3-methyl-2-[(pentyldithio)-methyl]-1-(1,5,6,7 -tetrahydro-5,5,7,7-tetramethyl -6-oxo-indeno[5,6-d]imidazol-2-yl)pyridiniumchlorid zeigt einen Schmelzpunkt von 160-162° (Zersetzung).

0214479

Beispiel 8

Eine Lösung von 0,7 ml Hexanthiol in 20 ml 1N wässeriger Salzsäure und 80 ml Methanol wird mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht eine Lösung. Man rührt das Reaktionsgemisch bei Raumtemperatur, dampft einen Teil des Methanols ab und kühlt dann kurz auf etwa 5° ab, worauf das kristalline Produkt abfiltriert und mit kaltem Wasser und Aether gewaschen wird. Das erhaltene 2-[(Hexyldithio)methyl]-4-methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 166-167° (Zersetzung).

Beispiel 9

Eine Lösung von 1,1 g Dodecanthiol in 20 ml 1N wässeriger Salzsäure und 40 ml Methanol wird mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht eine Lösung. Man rührt das Reaktionsgemisch bei Raumtemperatur, dampft einen Teil des Methanols ab und kühlt dann kurz auf etwa 5° ab, worauf das kristalline Produkt abfiltriert und mit kaltem Wasser und Aether gewaschen wird. Das erhaltene 2-[(Dodecyldithio)methyl]-4-methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 145° (Zersetzung).

Beispiel 10

Eine Lösung von 2 ml Allylmercaptan in 25 ml 1N wässeriger Salzsäure und 80 ml Methanol wird mit 4 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht eine Lösung. Man rührt das Reaktionsgemisch bei Raumtemperatur, dampft einen Teil des Methanols ab und kühlt dann kurz auf etwa 5° ab, worauf das kristalline Produkt abfiltriert und mit kaltem Wasser und Aether gewaschen wird. Das erhaltene 2-[(Allyldithio)methyl]-4--methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 180° (Zersetzung).

## Beispiel 11

Eine Lösung von 490 mg Cyclopentanthiol in 20 ml 1N wässeriger Salzsäure und 20 ml Methanol wird mit 1,8 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht eine Lösung. Man rührt das Reaktionsgemisch bei Raumtemperatur, dampft einen Teil des Methanols ab und kühlt dann kurz auf etwa 5° ab, worauf das kristalline Produkt abfiltriert und mit kaltem Wasser und Aether gewaschen wird. Das erhaltene 2-[(Cyclopentyldithio)-methyl]-4-methoxy-3-methyl -1-(1,5,6,7-tetrahydro-5,5,7,7--tetramethyl -6-oxoindeno[5,6-d]imidazol-2-yl)pyridiniumchlorid zeigt einen Schmelzpunkt von 187° (Zersetzung).

## Beispiel 12

Eine Lösung von 0,5 ml 2-Mercaptoäthanol in 25 ml 1N wässeriger Salzsäure wird mit 2 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 1 Stunde bei Raumtemperatur und kühlt dann kurz auf etwa 5° ab, worauf das kristalline Produkt abfiltriert und mit wenig Wasser und Aether gewaschen wird. Das erhaltene 2-[[(2-Hydroxyäthyl)dithio]methyl]-4-methoxy--3-methyl -1-(1,5,6,7-tetrahydro-5,5,7,7-tetramethyl -6-oxoindeno[5,6-d]imidazol-2-yl)pyridiniumchlorid zeigt

einen Schmelzpunkt von 148-150° (Zersetzung).

## Beispiel 13

Eine Lösung von 0,73 ml Thioglykolsäuremethylester in 30 ml 1N wässeriger Salzsäure wird mit 3 g 5,7-Dihydro-2--[[(4-methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 4-Methoxy-2-[[[(methoxycarbonyl)-methyl]dithio]methyl] -3-methyl-1-(1,5,6,7-tetrahydro--5,5,7,7-tetramethyl -6-oxoindeno[5,6-d]imidazol-2-yl)pyridiniumchlorid zeigt einen Schmelzpunkt von 162-164° (Zersetzung).

## Beispiel 14

Eine Lösung von 2 g Thioäpfelsäure in 50 ml 1N wässeriger Salzsäure wird mit 5 g 5,7-Dihydro-2-[[(4-methoxy-3--methyl -2-pyridyl)methyl]sulfinyl]-5,5,7,7-tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 1 Stunde bei Raumtemperatur und kühlt dann kurz auf etwa 5° ab, worauf das kristalline Produkt abfiltriert und mit wenig Wasser und Aether gewaschen wird. Das erhaltene 2-[[(1,2-Dicarboxyäthyl)dithio]methyl]-4-methoxy-3-methyl-1-(1,5,6,7-tetrahydro-5,5,7,7-tetramethyl -6-oxoindeno-[5,6-d]imidazol-2-yl)pyridiniumchlorid zeigt einen Schmelzpunkt von 183-187° (Zersetzung).

## Beispiel 15

Eine Lösung von 107 mg Thiopropionsäure in 10 ml 1N wässeriger Salzsäure wird mit 411 mg 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch eine halbe Stunde bei Raumtemperatur und kühlt dann kurz auf etwa 5° ab, worauf das kristalline Produkt abfiltriert und mit wenig Wasser und Aether gewaschen wird. Das erhaltene 2-[[(2-Carboxyäthyl)dithio]-methyl]-4-methoxy-3-methyl -1-(1,5,6,7-tetrahydro-5,5,7,7--tetramethyl -6-oxoindeno[5,6-d]imidazol-2-yl)pyridiniumchlorid zeigt einen Schmelzpunkt von 189-190° (Zersetzung).

## Beispiel 16

Eine Lösung von 0,57 g Thiomilchsäure in 20 ml 1N wässeriger Salzsäure wird mit 2 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 6 Stunden bei Raumtemperatur und kühlt dann kurz auf etwa 5° ab, worauf das kristalline Produkt abfiltriert und mit kaltem Wasser und Aether gewaschen wird. Das erhaltene 2-[[(1-Carboxyäthyl)dithio]methyl]-4--methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 192° (Zersetzung).

## Beispiel 17

Eine Lösung von 1,81 g L-Cystein in 40 ml 1N wässeriger Salzsäure wird mit 6,1 g 5,7-Dihydro-2-[[(4-methoxy-3--methyl -2-pyridyl)methyl]sulfinyl]-5,5,7,7-tetramethyl-

indeno [5,6-d]imidazol-6(1H)-on versetzt. Es entsteht sofort eine klare Lösung. Man rührt das Reaktionsgemisch eine halbe Stunde bei 0-5° und engt dann die Lösung ein. Der Rückstand wird in Methylenchlorid und Methanol gelöst, worauf das Lösungsmittelsystem bei Normaldruck abdestilliert und dauernd durch Essigester ersetzt wird. Das kristalline Produkt wird an Sephadex LH-20 mit Methylenchlorid/Methanol (1:1) chromatographiert und wie oben beschrieben noch einmal kristallisiert. Das so erhaltene 2-[[[(R)-2-Amino-2-carboxyäthyl]dithio]methyl]-4-methoxy-3-methyl-1-(1,5,6,7-tetrahydro-5,5,7,7-tetramethyl -6-oxo-indeno[5,6-d]imidazol-2-yl)pyridiniumchlorid zeigt einen Schmelzpunkt von 170° (Zersetzung).

## Beispiel 18

Eine Lösung von 0,97 g L-Cystein-äthylester•HCl in 20 ml 1N wässeriger Salzsäure wird mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht sofort eine klare Lösung. Man rührt das Reaktionsgemisch 1 Stunde bei Raumtemperatur und engt es dann ein. Der Rückstand wird aus Methanol-Essigester kristallisiert. Das erhaltene 2-[[[(R)-2-Amino-2-(äthoxycarbonyl)äthyl]dithio]methyl] -4-methoxy-3-methyl-1-(1,5,6,7-tetrahydro-5,5,7,7 -tetramethyl-6-oxoindeno[5,6-d]imidazol -2-yl)-pyridiniumchlorid-dihydrochlorid zeigt einen Schmelzpunkt von 184° (Zersetzung).

## Beispiel 19

Eine Lösung von 0,74 g D,L-Homocystein in 20 ml 1N wässeriger Salzsäure wird mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht sofort eine klare Lösung. Man rührt das Reaktionsgemisch 1 Stunde bei Raumtemperatur und engt es dann ein.

Der Rückstand wird aus Methanol-Essigester kristallisiert. Das erhaltene 2-[[[(RS)-3-Amino-3-carboxypropyl)]dithio]-methyl] -4-methoxy-3-methyl-1-(1,5,6,7-tetrahydro-5,5,7,7 -tetramethyl-6-oxoindeno[5,6-d]imidazol -2-yl)pyridiniumchlorid-hydrochlorid zeigt einen Schmelzpunkt von 200° (Zersetzung).

Beispiel 20

610 mg Thiophenol werden in 20 ml etwa 6N methanolischer Salzsäure gelöst. Man versetzt mit 2 g 5,7-Dihydro-2--[[(4-methoxy-3-methyl -2-pyridyl)methyl]sulfinyl]-5,5,7,7--tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 4-Methoxy--3-methyl-2-[(phenyldithio)methyl]-1-(1,5,6,7 -tetrahydro--5,5,7,7-tetramethyl -6-oxoindeno[5,6-d]imidazol-2-yl)pyridiniumchlorid wird abfiltriert und mit kaltem Essigester gewaschen; es zeigt einen Schmelzpunkt von 183° (Zersetzung).

Beispiel 21

Eine Lösung von 1,3 g p-Thiokresol wird unter Erhitzen in 30 ml 1N wässeriger Salzsäure gelöst und mit 4 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 4-Methoxy-3-methyl-2-[[(4-methylphenyl)dithio]methyl] -1-(1,5,6,7-tetrahydro-5,5,7,7-tetramethyl-6-oxoindeno[5,6-d]imidazol-2-yl)pyridiniumchlorid zeigt einen Schmelzpunkt von 174° (Zersetzung).

Beispiel 22

0,723 g 3-Chlorthiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[[(m--Chlorphenyl)dithio)methyl]-4-methoxy-3-methyl-1 -(1,5,6,7--tetrahydro-5,5,7,7-tetramethyl -6-oxoindeno[5,6-d]imidazol-2-yl)-pyridiniumchlorid wird abfiltriert und mit kaltem Essigester gewaschen; es zeigt einen Schmelzpunkt von 163--165° (Zersetzung).

Beispiel 23

Eine Lösung von 0,754 g p-Thiosalicylsäure 195 ml 1N wässeriger Salzsäure wird mit 2 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[[(o-Carboxyphenyl)dithio]methyl]-4--methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 190° (Zersetzung).

## Beispiel 24

Eine Lösung von 1,3 g p-Fluorthiophenol in 30 ml 1N wässeriger Salzsäure und 100 ml Methanol wird mit 4,1 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht sofort eine klare Lösung. Die Lösung wird unter Wasserstrahlvakuum gerührt, wobei das Reaktionsprodukt kristallisiert. Man filtriert das Reaktionsprodukt ab und wäscht es mit kaltem Wasser. Das erhaltene 2-[[(p--Fluorphenyl)dithio]methyl]-4-methoxy-3-methyl -1-(1,5,6,7--tetrahydro-5,5,7,7-tetramethyl -6-oxoindeno[5,6-d]imidazol-2-yl)pyridiniumchlorid zeigt einen Schmelzpunkt von 150° (Zersetzung).

## Beispiel 25

0,8 g p-Chlorthiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl]--5,5,7,7-tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[[(p--Chlorphenyl)dithio)methyl]-4-methoxy-3-methyl -1-(1,5,6,7 -tetrahydro-5,5,7,7-tetramethyl -6-oxoindeno[5,6-d]imidazol-2-yl)-pyridiniumchlorid wird abfiltriert und mit kaltem Essigester gewaschen; es zeigt einen Schmelzpunkt von 170--180° (Zersetzung).

## Beispiel 26

0,7 g 3-Methoxythiophenol werden in 20 ml Methanol und 2 ml etwa 6N methanolischer Salzsäure gelöst. Man versetzt mit 2,05 g 5,7-Dihydro-2-[[(4-methoxy-3-methyl -2-pyridyl)-

methyl]sulfinyl]-5,5,7,7-tetramethylindeno [5,6-d]imidazol--6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 4-Methoxy-2-[[(m-methoxyphenyl)dithio)methyl]-3-methyl-1-(1,5,6,7-tetrahydro-5,5,7,7-tetramethyl -6-oxo-indeno[5,6-d]imidazol-2-yl)pyridiniumchlorid wird abfiltriert und mit kaltem Essigester gewaschen; es zeigt einen Schmelzpunkt von 158-160° (Zersetzung).

Beispiel 27

0,8 g Pentafluorthiophenol werden in 10 ml Methanol und 2 ml etwa 6N methanolischer Salzsäure gelöst. Man versetzt mit 1,6 g 5,7-Dihydro-2-[[(4-methoxy-3-methyl -2-pyridyl)-methyl]sulfinyl]-5,5,7,7-tetramethylindeno [5,6-d]imidazol--6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Aether zu und rührt das Reaktionsgemisch bis zur beendeten Kristallisation weiter. Das erhaltene 4-Methoxy-3-methyl-2-[[(pentafluorphenyl)dithio]methyl-1-(1,5,6,7 -tetrahydro-5,5,7,7-tetramethyl -6-oxoindeno-[5,6-d]imidazol-2-yl)-pyridiniumchlorid wird abfiltriert und mit kaltem Aether gewaschen; es zeigt einen Schmelzpunkt von 153-157° (Zersetzung).

Beispiel 28

0,6 ml Benzylmercaptan werden in 20 ml Methanol und 5 ml etwa 6N methanolischer Salzsäure gelöst. Man versetzt mit 2,05 g 5,7-Dihydro-2-[[(4-methoxy-3-methyl -2-pyridyl)-methyl]sulfinyl]-5,5,7,7-tetramethylindeno [5,6-d]imidazol--6(1H)-on, rührt die Lösung bei Raumtemperatur und engt dann mittels einer Wasserstrahlpumpe so lange ein, bis ein

Niederschlag auszukristallisieren beginnt. In diesem Moment gibt man etwa 20 ml Aether zu und rührt das Reaktionsgemisch bis zur beendeten Kristallisation weiter. Das erhaltene 2-[(Benzyldithio)methyl]-4-methoxy-3-methyl -1-(1,5,-6,7-tetrahydro-5,5,7,7-tetramethyl -6-oxoindeno[5,6-d]imidazol-2-yl)pyridiniumchlorid wird abfiltriert und mit kaltem Aether gewaschen; es zeigt einen Schmelzpunkt von 178-180° (Zersetzung).

Beispiel 29

1,34 g Triphenylmethylmercaptan werden in 30 ml Methanol und 20 ml etwa 6N methanolischer Salzsäure gelöst. Man versetzt mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Wasser zu und rührt das Reaktionsgemisch bis zur beendeten Kristallisation weiter. Das erhaltene 4-Methoxy-3-methyl-1-(1,5,6,7-tetrahydro--5,5,7,7 -tetramethyl-6-oxoindeno[5,6-d]imidazol -2-yl)-2--[(trityldithio)methyl]pyridiniumchlorid wird abfiltriert und mit kaltem Wasser gewaschen; es zeigt einen Schmelzpunkt von 170° (Zersetzung).

Beispiel 30

0,9 g 2-Mercaptopyridin werden in 15 ml etwa 6N methanolischer Salzsäure gelöst. Man versetzt mit 3 g 5,7-Dihydro-2-[[(4-methoxy-3-methyl -2-pyridyl)methyl]sulfinyl]--5,5,7,7-tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Aether zu und rührt das Reaktionsgemisch bis zur beendeten Kristallisation weiter. Das erhaltene 4-Methoxy-

-3-methyl-2-(2-pyridyldithiomethyl) -1-(1,5,6,7-tetrahydro--5,5,7,7-tetramethyl -6-oxoindeno[5,6-d]imidazol-2-yl)pyridiniumchlorid -hydrochlorid (1:1,5) wird abfiltriert und mit kaltem Methanol und Aether gewaschen; es zeigt einen Schmelzpunkt von 151° (Zersetzung).

Beispiel 31

Eine Lösung von 0,9 g 2-Mercaptopyrimidin in 30 ml 1N wässeriger Salzsäure wird mit 3 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[(2-Pyrimidinyldithio)methyl]-4--methoxy-3-methyl -1-(1,5,6,7-tetrahydro-5,5,7,7-tetramethyl -6-oxoindeno[5,6-d]imidazol-2-yl)pyridiniumchlorid zeigt einen Schmelzpunkt von 173° (Zersetzung).

Beispiel 32

1,1 g 2-Amino-5-mercapto-1,3,4-thiadiazol werden in 15 ml etwa 6N methanolischer Salzsäure gelöst. Man versetzt mit 3 g 5,7-Dihydro-2-[[(4-methoxy-3-methyl -2-pyridyl)-methyl]sulfinyl]-5,5,7,7-tetramethylindeno [5,6-d]imidazol--6(1H)-on, rührt die Lösung bei Raumtemperatur und engt dann mittels einer Wasserstrahlpumpe bis zur beendeten Kristallisation ein. Das erhaltene 2-[[(5-Amino-1,3,4-thiadiazol-2-yl)dithio]methyl] -1-(1,5,6,7-tetrahydro-5,5,7,7--tetramethyl -6-oxoindeno[5,6-d]imidazol-2-yl)pyridiniumchlorid -hydrochlorid wird abfiltriert und mit kaltem Methanol gewaschen; es zeigt einen Schmelzpunkt von 161° (Zersetzung).

## Beispiel 33

Eine Lösung von 0,8 g 1,2,4-Triazol-3-thiol in 30 ml 1N wässeriger Salzsäure wird mit 3 g 5,7-Dihydro-2-[[(4--methoxy-3-methyl -2-pyridyl)methyl]sulfinyl]-5,5,7,7--tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 4-Methoxy-3-methyl-1-(1,5,6,7-tetra-hydro-5,5,7,7 -tetramethyl-6-oxoindeno[5,6-d]imidazol -2-yl)-2-[(1H-S-triazol-3-yldithio)methyl]pyridiniumchlorid zeigt einen Schmelzpunkt von 177° (Zersetzung).

## Beispiel 34

0,876 g Furfurylmercaptan werden in 30 ml etwa 6N methanolischer Salzsäure gelöst. Man versetzt mit 3 g 5,7-Dihydro-2-[[(4-methoxy-3-methyl -2-pyridyl)methyl]sulfinyl]-5,5,7,7-tetramethylindeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[(3-Furfuryldithio)methyl]-4-methoxy-3-methyl -1-(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelz-punkt von 174-175° (Zersetzung).

## Beispiel 35

851 mg 6-Mercaptopurin werden in 100 ml Methanol und 50 ml etwa 6N methanolischer Salzsäure gelöst. Man versetzt

mit 2 g 5,7-Dihydro-2-[[(4-methoxy-3-methyl -2-pyridyl)-methyl]sulfinyl]-5,5,7,7-tetramethylindeno [5,6-d]imidazol--6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 50 ml Essigester zu und rührt das Reaktionsgemisch bis zur beendeten Kristallisation weiter. Das erhaltene 4-Methoxy-3-methyl-2-[(purin-6-yldithio)methyl]-1-(1,5,6,7-tetrahydro-5,5,7,7-tetramethyl -6-oxoindeno-[5,6-d]imidazol-2-yl)pyridiniumchlorid -hydrochlorid wird abfiltriert und mit kaltem Essigester gewaschen; es zeigt einen Schmelzpunkt von 172° (Zersetzung).

Beispiel 36

Eine Lösung von 800 mg 4,6-Dimethyl-2-mercaptopyrimidin in 20 ml 1N wässeriger Salzsäure wird mit 2 g 5,7-Dihydro--2-[[(4-methoxy-3-methyl -2-pyridyl)methyl]sulfinyl]--5,5,7,7-tetramethylindeno [5,6-d]imidazol-6(1H)-on versetzt. Es entsteht sofort eine klare Lösung, aus welcher dann ein Festkörper auszukristallisieren beginnt. Man rührt das Reaktionsgemisch eine halbe Stunde bei Raumtemperatur und kühlt dann kurz auf etwa 5° ab, worauf das kristalline Produkt abfiltriert und mit kaltem Wasser und Aether gewaschen wird. Das erhaltene 2-[[(4,6-Dimethyl-2-pyrimidinyl)dithio]methyl]-4-methoxy -3-methyl-1-(1,5,6,7-tetrahydro-5,5,7,7-tetramethyl -6-oxoindeno[5,6-d]imidazol-2--yl)pyridiniumchlorid zeigt einen Schmelzpunkt von 170° (Zersetzung).

Beispiel 37

120 mg Furfurylmercaptan werden in 10 ml Methanol und 2 ml etwa 6N methanolischer Salzsäure gelöst. Man versetzt mit 411 mg 5,7-Dihydro-2-[[(4-methoxy-3-methyl -2-pyridyl)-methyl]sulfinyl]-5,5,7,7-tetramethylindeno [5,6-d]imidazol--6(1H)-on, rührt die Lösung bei Raumtemperatur und engt

dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene Produkt wird abfiltriert, mit kaltem Essigester gewaschen und in Methylenchlorid gelöst, worauf man mit 10%-iger Natriumbicarbonatlösung extrahiert. Die organische Lösung wird über Natriumsulfat getrocknet und eingeengt. Durch Kristallisation des Rückstandes aus Essigester erhält man intramolekular deprotonisiertes 2-[(3-Furfuryldithio)-methyl]-4-methoxy-3-methyl-1-(1,5,6,7 -tetrahydro-5,5,7,7-tetramethyl-6-oxoindeno[5,6-d]imidazol -2-yl)pyridiniumkation, welches einen Schmelzpunkt von 155-156° (Zersetzung) zeigt.

## Beispiel 38

150 mg Dimethylaminoäthanthiol-hydrochlorid werden in 10 ml Methanol und 2 ml etwa 6N methanolischer Salzsäure gelöst. Man versetzt mit 420 mg 5,7-Dihydro-2-[[(4-methoxy-3-methyl -2-pyridyl)methyl]sulfinyl]-5,5,7,7-tetramethyl-indeno [5,6-d]imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene Produkt wird abfiltriert, mit kaltem Essigester gewaschen und in einem Gemisch von Methylenchlorid und 10%-iger Natriumbicarbonatlösung aufgenommen. Man extrahiert, trocknet die organische Phase über Natriumsulfat und engt sie ein. Durch Kristallisation des Rückstandes aus Essigester erhält man intramolekular deprotonisiertes 2-[[[2-(Dimethylamino)äthyl]dithio]methyl]-4-methoxy -3-methyl-1-(1,5,6,7-tetrahydro-5,5,7,7-tetramethyl -6-oxoindeno[5,6-d]imidazol-2-yl)pyridiniumkation, welches einen Schmelzpunkt von 146° (Zersetzung) zeigt.

## Beispiel 39

100 mg 2-[(sec-Butyldithio)methyl]-4-methoxy-3-methyl -1-(1,5,6,7-tetrahydro-5,5,7,7-tetramethyl -6-oxoindeno-[5,6-d]imidazol-2-yl)pyridiniumchlorid werden in 4 ml 1N wässeriger Salzsäure gelöst und bei 110° (Oelbadtemperatur) während 24 Stunden erhitzt. Die Reaktionsmischung wird am Rotationsverdampfer bis zur Trockene eingeengt, worauf man zweimal aus Methylenchlorid-Aether umkristallisiert. Das erhaltene 2-[(sec-Butyldithio)methyl]-4-oxy-3-methyl-1--(1,5,6,7 -tetrahydro-5,5,7,7-tetramethyl-6-oxoindeno-[5,6-d]imidazol -2-yl)pyridinium zeigt einen Schmelzpunkt von 200° (Zersetzung).

## Beispiel 40

40 ml Wasser werden unter Eiskühlung mit 5 ml Thionylchlorid und anschliessend mit 1,5 g 2-[[(3,5-Dimethyl-4--methoxy-2-pyridyl)methyl]sulfinyl] -5-methoxybenzimidazol versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Das erhaltene intramolekular deprotonisierte 4-Methoxy-1-(5-methoxy-2-benzimidazolyl)-3,5-dimethyl -2-[(sulfothio)methyl]pyridiniumkation zeigt einen Schmelzpunkt von 180-183° (Zersetzung).

## Beispiel 41

60 ml Wasser werden unter Eiskühlung mit 4 ml Thionylchlorid und anschliessend mit 2 g 2-[[(4-Methoxy-3-methyl--2-pyridyl)methyl]sulfinyl] -5-(trifluormethyl)benzimidazol versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem

Wasser gewaschen und getrocknet. Das erhaltene intramolekular deprotonisierte 4-Methoxy-3-methyl-2-[(sulfothio)-methyl]-1-[5-(trifluormethyl) -2-benzimidazolyl]pyridiniumkation zeigt einen Schmelzpunkt von 163-164° (Zersetzung).

Beispiel 42

200 ml Wasser werden unter Eiskühlung mit 25 ml Thionylchlorid und anschliessend mit 10 g 5,7-Dihydro-2-[[(4--methoxy-3-methyl-2 -pyridyl)methyl]sulfinyl]-5,5,7,7 -tetramethylindeno[5,6-d]imidazol-6(1H)-on versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Das erhaltene intramolekular deprotonisierte 4-Methoxy-3-methyl-2-[(sulfothio)methyl]-1-(1,5,6,7 -tetrahydro-5,5,7,7-tetramethyl-6-oxoindeno[5,6-d]imidazol -2-yl)pyridiniumkation zeigt einen Schmelzpunkt von 201--203° (Zersetzung). Durch Umkristallisieren aus Methylenchlorid-Methanol erhält man ein Produkt vom Schmelzpunkt 208-209° (Zersetzung).

Beispiel 43

a) Eine Suspension von 5,2 g 5,7-Dihydro-2-mercapto--5,5,7,7 -tetramethylindeno[5,6-d]imidazol-6(1H)-on und 2,6 g Kalium-t-butanolat wird unter Argon mit einer Lösung von 3 g 2-Chlormethyl-3-methylpyridin in 30 ml t-Butanol versetzt und während 18 Stunden gerührt. Nach Einengen des Gemisches wird der Rückstand mit Eisessig angesäuert und in Methylenchlorid gelöst, worauf man 10%-ige Natriumbicarbonatlösung zugibt, extrahiert und die Methylenchloridlösung trocknet und einengt. Das so erhaltene Rohprodukt wird an Kieselgel chromatographiert und mit Methylenchlorid/Essigester (1:1) gereinigt, wobei die Methode der Mitteldruck--Flashchromatographie verwendet und der Druck mit Stickstoffgas erzeugt wird. Das aus Essigester-n-Hexan erhaltene 5,7-Dihydro-5,5,7,7-tetramethyl-2-[[(3-methyl -2-pyridyl)-methyl]thio]indeno[5,6-d]imidazol-6(1H)-on schmilzt bei 161-164°.

b) 2,5 g 5,7-Dihydro-5,5,7,7-tetramethyl-2-[[(3-methyl -2-pyridyl)methyl]thio]indeno[5,6-d]imidazol-6(1H)-on werden in 100 ml Chloroform gelöst und unter Argon mit 1,5 g m-Chlorperbenzoesäure versetzt, worauf während 15 Minuten unter Eiskühlung gerührt wird. Man giesst dann die Lösung in ein Gemisch von Natriumcarbonatlösung und Eis. Nach mehrmaligem Extrahieren wird die Methylenchloridlösung getrocknet und eingeengt, und der Rückstand wird aus Aether kristallisiert. Das erhaltene 5,7-Dihydro-5,5,7,7--tetramethyl-2-[[(3-methyl -2-pyridyl)methyl]sulfinyl]-indeno[5,6-d]imidazol-6(1H)-on zeigt einen Schmelzpunkt von 176° (Zersetzung).

c) 120 ml Wasser werden unter Eiskühlung mit 6,7 ml Thionylchlorid und anschliessend mit 2 g 5,7-Dihydro-5,5,7,7--tetramethyl-2-[[(3-methyl -2-pyridyl)methyl]sulfinyl]-indeno[5,6-d]imidazol-6(1H)-on versetzt. Das Reaktionsgemisch wird während 10 Minuten leicht erwärmt und anschliessend während 2 Stunden bei Raumtemperatur gerührt, wobei ein farbloser Niederschlag auskristallisiert. Dieser Niederschlag wird abfiltriert, mit eiskaltem Wasser gewaschen und getrocknet. Das erhaltene intramolekular deprotonisierte 3-Methyl-2-[(sulfothio)methyl]-1-(1,5,6,7-tetrahydro -5,5,7,7-tetramethyl-6-oxoindeno[5,6-d]imidazol -2-yl)pyridiniumkation zeigt einen Schmelzpunkt von 194-198° (Zersetzung).

## Beispiel 44

a) Eine Lösung von 183 mg (1,49 mMol) 2,3-Dimethylpyridin--1-oxid in 0,6 ml konz. Schwefelsäure wird unter Eiskühlung mit 0,2 ml 65%iger Salpetersäure (d=1,4) versetzt. Das

Reaktionsgemisch wird 24 Stunden bei 90° gerührt und auf ein Gemisch von Eis und Natriumcarbonat gegossen, worauf man mit Methylenchlorid extrahiert und die Methylenchloridphase trocknet und eindampft. Der aus Aethanol/n-Pentan kristallisierte Rückstand ergibt 2,3-Dimethyl-4-nitropyridin-1-oxid vom Schmelzpunkt 99-102°.

b) 25 g 2,3-Dimethyl-4-nitropyridin-1-oxid werden in 1250 ml n-Propanol gelöst und während 16 Tagen bei 120° unter Argon gerührt. Die Reaktionsmischung wird eingeengt, worauf der Rückstand mit Methylenchlorid-Wasser versetzt und extrahiert wird. Man trocknet die organischen Phase und engt sie ein. Der Rückstand wird an Kieselgel chromatographiert und mit Methylenchlorid-Methanol (20:1, 10:1) gereinigt, wobei die Methode der Mitteldruck-Flashchromatographie verwendet und der Druck mit Stickstoffgas erzeugt wird. Das erhaltene 2,3-Dimethyl-4-propoxypyridin-1-oxid wird direkt weiterverarbeitet.

c) Zu einer unter Argon am Rückfluss kochenden Lösung von 13 g 2,3-Dimethyl-4-propoxypyridin-1-oxid in 250 ml Isopropylacetat wird während 1 Stunde eine Lösung von 23 ml Trichloracetylchlorid in 50 ml Isopropylacetat zugetropft. Zu Beginn zeigt die Reaktion sehr exothermen Charakter. Während der Zugabe des ersten Moläquivalentes Trichloracetylchlorid wird eine heftige Kohlendioxidentwicklung beobachtet. Anschliessend wird die Lösung noch 15 Minuten bei Rückflusstemperatur (Oelbad 130°) unter Argon gerührt, mit Eis auf 10° abgekühlt, mit 100 ml Aether versetzt und unter Eiskühlung 1 Stunde gerührt. Der kristalline, während der ganzen Reaktion ausfallende Niederschlag wird abgenutscht und mit eiskaltem Isopropylacetat und Aether gewaschen. Aus der Mutterlauge kann wiederum kristallines Reaktionsprodukt gewonnen werden. Das erhaltene 2-Chlormethyl-3-methyl-4--propoxypyridin-hydrochlorid wird direkt weiterverarbeitet.

d) Eine Lösung von 11,5 g 2-Chlormethyl-3-methyl-4-pro-

poxypyridin-hydrochlorid in 100 ml Methylenchlorid wird mit 100 ml 10%-iger Natriumcarbonatlösung behandelt. Die wässrige Lösung wird dreimal mit je 100 ml Methylenchlorid extrahiert. Die organische Phase wird über Natriumsulfat getrocknet, filtriert und eingeengt. Der Rückstand wird in 180 ml abs. Aceton gelöst, mit 10 g 5,7-Dihydro-2-mercapto-5,5,7,7 -tetramethylindeno[5,6-d]imidazol-6(1H)-on und mit 18 g gemahlenem Kaliumcarbonat versetzt. Die Reaktionsmischung wird während 18 Stunden unter Argon gerührt. Das Lösungsmittelvolumen wird bis auf etwa 20 ml eingeengt. Das verbleibende Reaktionsgemisch wird auf Eis-Wasser gegossen und das Ganze während 20 Minuten kräftig durchgerührt. Nach Absaugen und Waschen des Reaktionsproduktes wird der Filterrückstand in Methylenchlorid gelöst und mit gesättigter Natriumchloridlösung gewaschen. Die organische Phase wird über Natriumsulfat getrocknet, über Dicalite Speedex filtriert und eingeengt. Das Produkt wird an einer Silicagelkolonne mit Methylenchlorid/Essigester (1:1) chromatographiert. Das gereinigte 5,7-Dihydro-5,5,7,7-tetramethyl-2-[[(3-methyl-4-propoxy -2-pyridyl)methyl]thio]indeno-[5,6-d]imidazol-6(1H)-on kristallisiert aus Aether und schmilzt bei 162-163°.

e) 4,2 g 5,7-Dihydro-5,5,7,7-tetramethyl-2-[[(3-methyl-4-propoxy -2-pyridyl)methyl]thio]indeno[5,6-d]imidazol-6-(1H)-on werden in 50 ml Methylenchlorid gelöst und bei -40° unter Argon mit 2,2 g m-Chlorperbenzoesäure in 15 ml Methylenchlorid versetzt. Die Reaktionsmischung wird während 15 Minuten gerührt. Man giesst dann die Lösung in ein Gemisch von Natriumcarbonatlösung und Eis. Nach mehrmaligem Extrahieren wird die Methylenchloridlösung getrocknet und eingeengt, und der Rückstand wird aus Aether kristallisiert. Das erhaltene 5,7-Dihydro-5,5,7,7-tetramethyl-2-[[(3-methyl-4-propoxy -2-pyridyl)methyl]sulfinyl]-indeno[5,6-d]imidazol-6(1H)-on zeigt einen Schmelzpunkt von 171° (Zersetzung).

f) 80 ml Wasser werden unter Eiskühlung mit 4 ml Thionylchlorid und anschliessend mit 2 g 5,7-Dihydro-5,5,7,7--tetramethyl-2-[[(3-methyl-4-propoxy -2-pyridyl)methyl]sulfinyl]indeno[5,6-d]imidazol-6(1H)-on versetzt. Das Reaktionsgemisch wird während 10 Minuten leicht erwärmt und anschliessend während 30 Minuten mit Eis gekühlt, wobei ein Niederschlag auskristallisiert. Dieser Niederschlag wird abfiltriert, mit eiskaltem Wasser gewaschen und getrocknet. Das erhaltene intramolekular deprotonisierte 3-Methyl-4--propoxy-1-(1,5,6,7-tetrahydro-5,5,7,7 -tetramethyl-6-oxo-indeno[5,6-d]imidazol-2-yl) -2-[(sulfothio)methyl]pyridiniumkation wird aus n-Propanol umkristallisiert und zeigt einen Schmelzpunkt von 214° (Zersetzung).

## Beispiel 45

a) Ein Gemisch von 10,5 g Natriumäthylat, 20 g Natriumcarbonat und 400 ml Aethanol wird unter Argon bei 50° während 30 Minuten gerührt und dann mit 7 g (0,017 Mol) 5,7-Dihydro-5,5,7,7-tetramethyl-2-[[(3-methyl-4-nitro -2-pyridyl)-methyl]thio]indeno[5,6-d]imidazol-6(1H)-on versetzt. Nach 5-stündigem Rühren bei 50° unter Argon wird das Reaktionsgemisch im Vakuum eingeengt. Der Rückstand wird mit Methylenchlorid versetzt und mittels Eisessig gepuffert; die Methylenchloridlösung wird mit Natriumbicarbonatlösung extrahiert, getrocknet und eingeengt. Der Rückstand wird an Kieselgel unter Eluieren mit Methylenchlorid/Essigester (3:1) chromatograhiert, wobei die Methode der Mitteldruck--Flashchromatographie verwendet und der Druck mit Stickstoffgas erzeugt wird. Man erhält 2-[[(4-Aethoxy-3-methyl--2-pyridyl)methyl]thio]-5,7-dihydro -5,5,7,7-tetramethyl-indeno-[5,6-d]imidazol-6(1H)-on, welches nach Umkristallisieren aus Aether bei 175-176° schmilzt.

b) Eine Lösung von 2 g (0,0049 Mol) 2-[[(4-Aethoxy-3--methyl-2-pyridyl)methyl]thio]-5,7-dihydro -5,5,7,7-tetramethylindeno[5,6-d]imidazol-6(1H)-on in 35 ml abs. Methylenchlorid wird unter Argon bei -40 bis -50° innerhalb 10 Minuten mit 1,1 g (0,0064 Mol) m-Chlorperbenzoesäure in 10 ml abs. Methylenchlorid versetzt. Anschliessend wird die Lösung noch zusätzlich 20 Minuten gerührt, mit 10%iger Natriumcarbonatlösung extrahiert, getrocknet und unter stetigem Ersatz von Methylenchlorid durch Essigester eingeengt. Dabei kristallisiert 2-[[(4-Aethoxy-3-methyl-2-pyridyl)methyl]sulfinyl]-5,7 -dihydro-5,5,7,7-tetramethylindeno[5,6-d]imidazol-6(1H)-on, welches bei 180° unter Zersetzung schmilzt.

c) 80 ml Wasser werden unter Eiskühlung mit 4 ml Thionylchlorid und dann mit 2 g 2-[[(4-Aethoxy-3-methyl-2-pyridyl)methyl]sulfinyl]-5,7 -dihydro-5,5,7,7-tetramethylindeno[5,6-d]imidazol-6(1H)-on versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Man löst das Produkt in Methylenchlorid, filtriert und versetzt mit Aethanol. Das Lösungsmittelgemisch wird bis zur Kristallisation abgedampft. Das durch Filtration erhaltene intramolekular deprotonisierte 4-Aethoxy-3--methyl-2-[(sulfothio)methyl]-1-(1,5,6,7 -tetrahydro-5,5,-7,7-tetramethyl-6-oxoindeno[5,6-d]imidazol -2-yl)pyridiniumkation zeigt einen Schmelzpunkt von 199-204° (Zersetzung).

## Beispiel 46

a) Eine Lösung von 45 ml 2,3-Lutidin (2,3-Dimethylpyridin) und 4,3 g Kupfer(I)-jodid in 500 ml Tetrahydrofuran wird auf -20° gekühlt und unter Argon mit 31 ml Chlorameisensäureäthylester versetzt. Die Reaktionsmischung wird auf 30° erhitzt und innerhalb von 30 Minuten tropfenweise mit 125 ml t-Butylmagnesiumchlorid (2,6 molar, in Tetrahydrofuran) versetzt, wobei die Reaktionstemperatur 40-45°

beträgt. Das als Zwischenprodukt erhaltene Aethyl 4-tert--butyl-2,3-dimethyl-1(4H)-pyridincarboxylat kann wie folgt isoliert werden: Das Reaktionsgemisch wird auf -10° abgekühlt und mit 300 ml wässeriger (20%) Ammoniumchloridlösung versetzt, worauf 500 ml Aether zugegeben werden und die organische Lösung mehrmals mit einer 20%-igen Ammoniumchlorid/Ammoniak-(1:1)-Lösung, 2N Salzsäure und 10%-iger Natriumcarbonatlösung gewaschen wird. Die organische Phase wird getrocknet und eingeengt, und der Rückstand wird an einer Silicagelkolonne gereinigt, wobei die Methode der Mitteldruck-Flashchromatographie (Lösungsmittel: n-Hexan/-Aether [20:1]) verwendet und der Druck mit Stickstoffgas erzeugt wird. Das erhaltene Aethyl-4-tert-butyl-2,3--dimethyl-1(4H)-pyridincarboxylat (2,1 g) wird direkt weiterverarbeitet, indem es mit 420 mg subl. Schwefel während 1,5 Stunden auf 170° erhitzt wird. Die Reaktionsmischung wird in 200 ml Aether gelöst, worauf mit 2N HCl extrahiert und die wässerige Lösung mit Aether gewaschen, mit einer 10%-igen Natriumcarbonatlösung alkalisch gestellt und mit Methylenchlorid extrahiert wird. Die organische Lösung wird getrocknet und bei Normaldruck eingeengt. Das erhaltene 4-tert-Butyl-2,3-dimethylpyridin wird direkt weiterverarbeitet.

b) Eine Lösung von 10,5 g 4-tert-Butyl-2,3-dimethylpyridin in 120 ml Methylenchlorid wird bei Raumtemperatur mit 14,4 g m-Chlorperbenzoesäure versetzt. Die Reaktionsmischung wird mit einer gesättigten Natriumcarbonatlösung basisch gestellt und extrahiert. Die organische Lösung wird getrocknet und eingeengt. Der Rückstand wird an einer Silicagelkolonne gereinigt, wobei die Methode der Mitteldruck--Flashchromatographie (Lösungsmittel: Methylenchlorid/-Essigester, mit steigenden Essigesterkonzentrationen) verwendet und der Druck mit Stickstoffgas erzeugt wird. Das erhaltene 4-tert-Butyl-2,3-dimethylpyridin-N-oxid wird direkt weiterverarbeitet.

c) Eine Lösung von 9,8 g 4-tert-Butyl-2,3-dimethylpyridin--N-oxid in 120 ml abs. Chloroform wird unter Argon am Rückfluss gekocht und durch den Kühler mit 25,5 ml Trichloressigsäurechlorid versetzt. Nach 22 Stunden wird das Reaktionsgemisch auf Eis gegossen, worauf man 10%-ige Natriumbicarbonatlösung zugibt, mit Methylenchlorid extrahiert und die Methylenchloridlösung trocknet und einengt. Das so erhaltene Rohprodukt wird an Kieselgel chromatographiert (Lösungsmittel: Methylenchlorid/Aether [10:1]). Das erhaltene 2-Chlormethyl-4-tert-butyl-3-methylpyridin wird direkt weiterverarbeitet.

d) Eine Lösung von 4,4 g 2-Chlormethyl-4-tert-butyl-3--methylpyridin in 150 ml Aceton wird mit 5,7 g 5,7-Dihydro--2-mercapto-5,5,7,7 -tetramethylindeno[5,6-d]imidazol--6(1H)-on und 5 g gemahlenem Kaliumcarbonat versetzt. Die Reaktionsmischung wird während 18 Stunden unter Argon gerührt, worauf das Lösungsmittelvolumen bis auf etwa 20 ml eingeengt wird. Das Reaktionsgemisch wird mit Methylenchlorid/Wasser versetzt und extrahiert. Die organische Phase wird getrocknet und eingeengt, und der Rückstand wird aus Aether kristallisiert. Das erhaltene 2-[[(4-tert-Butyl--3-methyl-2-pyridyl)methyl]thio]-5,7 -dihydro-5,5,7,7--tetramethylindeno[5,6-d]imidazol-6(1H)-on zeigt einen Schmelzpunkt von 156-158°.

e) 1,1 g rohes 2-[[(4-tert-Butyl-3-methyl-2-pyridyl)-methyl]thio]-5,7 -dihydro-5,5,7,7-tetramethylindeno[5,6-d]-imidazol-6(1H)-on werden in 20 ml Methylenchlorid gelöst und bei -40° unter Argon langsam mit 600 mg m-Chlorperbenzoesäure in 5 ml Methylenchlorid versetzt. Die Reaktionsmischung wird während 15 Minuten gerührt. Dann giesst man die Lösung in ein Gemisch von Natriumcarbonatlösung und Eis. Nach mehrmaligem Extrahieren wird die organische Lösung getrocknet und eingeengt, und der Rückstand wird aus Essigester kristallisiert. Das erhaltene 2-[[(4-tert-Butyl--3-methyl-2-pyridyl)methyl]sulfinyl]-5,7 -dihydro-5,5,7,7-

-tetramethylindeno[5,6-d]imidazol-6(1H)-on zeigt einen Schmelzpunkt von 200-204° (Zersetzung).

f) 20 ml Wasser werden unter Eiskühlung mit 2 ml Thionylchlorid und mit 900 mg 2-[[(4-tert-Butyl-3-methyl-2-pyridyl)methyl]sulfinyl]-5,7 -dihydro-5,5,7,7-tetramethylindeno[5,6-d]imidazol-6(1H)-on versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Das erhaltene intramolekular deprotonisierte 4-tert-Butyl-3-methyl-2-[(sulfothio)methyl]-1-(1,5,6,7-tetrahydro-5,5,7,7-tetramethyl-6-oxoindeno[5,6-d]imidazol-2-yl)pyridiniumkation zeigt einen Schmelzpunkt von 209-210° (Zersetzung).

## Beispiel 47

a) Eine Lösung von 6,75 g 2,3-Lutidin und 475 mg Kupfer-(I)-jodid in 150 ml abs. Tetrahydrofuran wird auf -20° gekühlt und unter Argon mit 3,4 ml Chlorameisensäureäthylester versetzt. Das Reaktionsgemisch wird auf 30° erhitzt und innerhalb von 10 Minuten tropfenweise mit 14 ml Methylmagnesiumchlorid (3 molar, in Tetrahydrofuran) versetzt. Das als Zwischenprodukt erhaltene Aethyl-2,3,4-trimethyl-1(4H)-pyridincarboxylat kann wie folgt isoliert werden: Das Reaktionsgemisch wird auf -10° gekühlt und mit 38 ml wässeriger (20%) Ammoniumchloridlösung versetzt, worauf 100 ml Aether zugegeben werden und die organische Lösung mehrmals mit einer 20%-igen Ammoniumchlorid/Ammoniak-(1:1)-Lösung, 2N Salzsäure und 10%-iger Natriumcarbonatlösung gewaschen wird. Die organische Lösung wird getrocknet und eingeengt, und der Rückstand wird an einer Silicagelkolonne gereinigt, wobei die Methode der Mitteldruck-Flashchromatographie (Lösungsmittel: n-Hexan/Aether [20:1]) verwendet

und der Druck mit Stickstoffgas erzeugt wird.

Das erhaltene Zwischenprodukt (975 mg) wird direkt weiterverarbeitet, indem es mit 160 mg subl. Schwefel während 1 Stunde bei 140° erhitzt wird. Die Reaktionsmischung wird in 100 ml Aether gelöst, worauf mit 2N HCl extrahiert und die wässerige Lösung mit Aether gewaschen, mit einer 10%-igen Natriumcarbonatlösung alkalisch gestellt und mit Methylenchlorid extrahiert wird. Die organische Lösung wird getrocknet und bei Normaldruck eingeengt. Das erhaltene 2,3,4-Trimethylpyridin wird direkt weiterverarbeitet.

b) Eine Lösung von 14 g 2,3,4-Trimethylpyridin in 140 ml Methylenchlorid wird unter Eiskühlung vorsichtig mit 21 g m-Chlorperbenzoesäure versetzt. Die Reaktionsmischung wird 1 Stunde am Rückfluss gekocht, dann mit einer gesättigten Sodalösung basisch gestellt und anschliessend extrahiert. Die organische Phase wird getrocknet und eingeengt. Der Rückstand wird an einer Silicagelkolonne gereinigt, wobei die Methode der Mitteldruck-Flashchromatographie (Lösungsmittel: Methylenchlorid/Methanol [5:1]) verwendet und der Druck mit Stickstoffgas erzeugt wird. Das erhaltene 2,3,4--Trimethylpyridin-N-oxid wird direkt weiterverarbeitet.

c) Eine Lösung von 13,3 g 2,3,4-Trimethylpyridin-N-oxid in 130 ml abs. Chloroform wird unter Argon am Rückfluss gekocht und durch den Kühler mit 33,2 ml Trichloressigsäurechlorid versetzt. Nach 18 Stunden wird das Reaktionsgemisch auf Eis gegossen, worauf man 10%-ige Natriumbicarbonatlösung zugibt, mit Methylenchlorid extrahiert und die Methylenchloridlösung trocknet und einengt. Das so erhaltene Rohprodukt wird an Kieselgel chromatographiert (Lösungsmittel: Methylenchlorid/Aether [5:1]). Das erhaltene 2-Chlormethyl-3,4-dimethylpyridin wird direkt weiterverarbeitet.

d) Eine Lösung von 4,5 g 2-Chlormethyl-3,4-dimethylpyridin in 80 ml Aceton wird mit 7,3 g 5,7-Dihydro-2-mercapto--5,5,7,7 -tetramethylindeno[5,6-d]imidazol-6(1H)-on und 5,4 g gemahlenem Kaliumcarbonat versetzt. Die Reaktionsmischung wird während 18 Stunden unter Argon gerührt, worauf das Lösungsmittelvolumen bis auf etwa 15 ml eingeengt wird. Das Reaktionsgemisch wird auf Eis-Wasser gegossen; der Festkörper wird abfiltriert, mit Wasser gewaschen und aus Acetonitril umkristallisiert. Das erhaltene 2-[[(3,4-Dimethyl-2-pyridyl)methyl]thio]-5,7 -dihydro--5,5,7,7-tetramethylindeno[5,6-d]imidazol-6(1H)-on zeigt einen Schmelzpunkt von 163-165°.

e) 3,8 g 2-[[(3,4-Dimethyl-2-pyridyl)methyl]thio]-5,7 -dihydro-5,5,7,7-tetramethylindeno[5,6-d]imidazol-6(1H)-on werden in 50 ml abs. Methylenchlorid gelöst und bei -40° unter Argon langsam mit 2,2 g m-Chlorperbenzoesäure in 30 ml Methylenchlorid versetzt. Die Reaktionsmischung wird während 20 Minuten gerührt. Dann giesst man die Lösung in ein Gemisch von Natriumcarbonatlösung und Eis. Nach mehrmaligem Extrahieren wird die organische Lösung getrocknet und eingeengt, und der Rückstand wird aus Methylenchlorid/ Essigester kristallisiert. Das erhaltene 2-[[(3,4-Dimethyl--2-pyridyl)methyl]sulfiny]-5,7-dihydro -5,5,7,7-tetramethylindeno[5,6-d]imidazol-6(1H)-on zeigt einen Schmelzpunkt von 197-200° (Zersetzung).

f) 30 ml Wasser werden unter Eiskühlung mit 3 ml Thionylchlorid und anschliessend mit 1,5 g 2-[[(3,4-Dimethyl-2--pyridyl)methyl]sulfinyl]-5,7-dihydro -5,5,7,7-tetramethylindeno[5,6-d]imidazol-6(1H)-on versetzt. Das Reaktionsgemisch wird während 10 Minuten leicht erwärmt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Das erhaltene intramolekular deprotonisierte 3,4-Dimethyl-2-[(sulfothio)methyl]-1-(1,5,6,7-tetrahydro

-5,5,7,7-tetramethyl-6-oxoindeno[5,6-d]imidazol -2-yl)pyridiniumkation zeigt nach Umkristallisieren aus Methylenchlorid/Methanol einen Schmelzpunkt von 156° (Zersetzung).

## Beispiel 48

a) Eine Lösung von 7,3 g (0,039 Mol) 2-Chlormethyl-3--methyl-4-nitro-pyridin und 7,5 g (0,039 Mol) 5H-1,3--Dioxolo[4,5-f]benzimidazol-6-thiol in 200 ml abs. Aceton wird mit 8 g fein gemahlenem Kaliumcarbonat versetzt, worauf bei Raumtemperatur unter Argon 2 Stunden gerührt wird. Die Mischung wird auf Eis gegossen, und die entstandenen Kristalle werden abfiltriert, gründlich mit Wasser gewaschen und in Acetonitril gelöst. Die erhaltene Lösung wird heiss filtriert. Beim Abkühlen des Filtrats kristallisiert 6-[[(3-Methyl-4-nitro-2-pyridyl)methyl]thio]-5H-1,3 -dioxolo[4,5-f]benzimidazol vom Schmelzpunkt 204-205° (Zersetzung).

b) Eine Lösung von 500 mg (1,45 mMol) 6-[[(3-Methyl-4--nitro-2-pyridyl)methyl]thio]-5H-1,3 -dioxolo[4,5-f]benzimidazol in 20 ml abs. Methanol wird mit 300 mg Natriummethylat versetzt, worauf unter Argon während 18 Stunden am Rückfluss gekocht wird. Die Reaktionsmischung wird mittels Eisessig gepuffert und im Vakuum eingeengt. Der Rückstand wird mit Methylenchlorid/Natriumbicarbonatlösung versetzt, worauf man die organische Lösung trocknet und einengt. Durch Umkristallisieren aus Essigester erhält man 6-[[(4--Methoxy-3-methyl-2-pyridyl)methyl]thio]-5H-1,3 -dioxolo-[4,5-f]benzimidazol vom Schmelzpunkt 215-220°.

c) Eine Lösung von 330 mg (1 mMol) 6-[[(4-Methoxy-3--methyl-2-pyridyl)methyl]thio]-5H-1,3 -dioxolo[4,5-f]benzimidazol in 5 ml Chloroform wird unter Eiskühlung und Rühren portionenweise mit 200 mg (1,2 mMol) m-Chlorperbenzoesäure versetzt. Nach 15 Minuten wird die Reaktionsmischung mit 10%iger Natriumcarbonatlösung extrahiert,

getrocknet und eingeengt. Der Rückstand wird an Kieselgel mit Methylenchlorid/Methanol (8,5:1,5) als Elutionsmittel chromatographiert, wobei die Methode der Mitteldruck-Flashchromatographie verwendet und der Druck mit Stickstoffgas erzeugt wird. Durch Umkristallisieren aus Aether erhält man 6-[[(4-Methoxy-3-methyl-2-pyridyl)methyl]sulfinyl]-5H-1,3 -dioxolo[4,5-f]benzimidazol vom Schmelzpunkt 185-186° (Zersetzung).

d) 10 ml Wasser werden unter Eiskühlung mit 1 ml Thionylchlorid und anschliessend mit 500 mg 6-[[(4-Methoxy-3--methyl-2-pyridyl)methyl]sulfiny]-5H-1,3 -dioxolo[4,5-f]-benzimidazol versetzt. Das Reaktionsgemisch wird mit 40 ml Wasser verdünnt und anschliessend während 30 Minuten mit Eis gekühlt, wobei ein farbloser Niederschlag auskristallisiert. Dieser Niederschlag wird abfiltriert, mit eiskaltem Wasser gewaschen und getrocknet. Das erhaltene intramolekular deprotonisierte 1-(5H-[1,3]Dioxolo[4,5-f]benzimidazol--6-yl)-4-methoxy -3-methyl-2-[(sulfothio)methyl]pyridiniumkation zeigt einen Schmelzpunkt von 201° (Zersetzung).

## Beispiel 49

a) Eine Lösung von 2,5 g (0,015 Mol) 2,3-Dimethyl-4-nitropyridin-1-oxid in 50 ml abs. Methanol wird mit 0,883 g Natriummethylat versetzt, worauf unter Argon bei Raumtemperatur während 2 Tagen gerührt wird. Die Reaktionsmischung wird eingeengt, und der Rückstand wird mit Methylenchlorid und gesättigter Natriumchloridlösung extrahiert. Die Methylenchloridphase wird getrocknet und eingedampft. Der aus Methylenchlorid/Aether kristallisierte Rückstand ergibt 4-Methoxy-2,3-dimethylpyridin-1-oxid vom Schmelzpunkt 80-83°.

b) Eine Lösung von 500 mg (3,26 mMol) 4-Methoxy-2,3-dimethylpyridin-1-oxid in 20 ml 1,2-Dichloräthan wird am Rückfluss gekocht und mit 8,3 g Trichloressigsäurechlorid ver-

setzt. Nach 35 Minuten wird das Reaktionsgemisch auf Eis gegossen, worauf man 10%ige Natriumcarbonatlösung zugibt, mit Methylenchlorid extrahiert und die Methylenchloridlösung trocknet und einengt. Das so erhaltene 2-Chlormethyl-4-methoxy-3-methylpyridin wird als Rohprodukt direkt weiterverarbeitet.

c) Eine Lösung von 4,5 g 2-Chlormethyl-4-methoxy-3-methylpyridin in 80 ml Aceton wird mit 7,3 g 5,6,7,8-Tetrahydro-5,5,8,8-tetramethyl-1H -naphth[2,3-d]imidazol-2-thiol und 5,4 g gemahlenem Kaliumcarbonat versetzt. Die Reaktionsmischung wird während 18 Stunden unter Argon gerührt, worauf man das Lösungsmittelvolumen bis auf etwa 15 ml einengt und auf Eis-Wasser giesst. Der Festkörper wird abfiltriert und mit Wasser gewaschen. Aus Methylenchlorid/-n-Hexan umkristallisiert zeigt das erhaltene 5,6,7,8-Tetrahydro-5,5,8,8-tetramethyl-2-[[(4-methoxy -3-methyl-2-pyridyl)methyl]thio]-1H-naphth[2,3-d]imidazol einen Schmelzpunkt von 170°.

d) 3,1 g 5,6,7,8-Tetrahydro-5,5,8,8-tetramethyl-2-[[(4-methoxy -3-methyl-2-pyridyl)methyl]thio]-1H-naphth[2,3-d]-imidazol werden in 40 ml Chloroform gelöst und unter Eiskühlung mit 1,5 g m-Chlorperbenzoesäure versetzt, worauf 10 Minuten gerührt und dann auf ein Gemisch von Natriumcarbonatlösung und Eis gegossen wird. Nach mehrmaligem Extrahieren wird die organische Lösung getrocknet und eingeengt. Der Rückstand wird an Kieselgel chromatographiert (Lösungsmittel: Methylenchlorid/Methanol [20:1]). Das erhaltene 5,6,7,8-Tetrahydro-2-[[(4-methoxy-3-methyl -2-pyridyl)-methyl]sulfinyl]-5,5,8,8-tetramethyl-1H -naphth[2,3-d]imidazol wird aus Aether kristallisiert und zeigt dann einen Schmelzpunkt von 167° (Zersetzung).

e) 83 ml Wasser werden unter Eiskühlung mit 12,4 ml Thionylchlorid und mit 4,8 g 5,6,7,8-Tetrahydro-2-[[(4-methoxy-3-methyl -2-pyridyl)methyl]sulfinyl]-5,5,8,8-tetramethyl-

-1H-naphth[2,3-d]imidazol versetzt. Das Reaktionsgemisch wird mit wenig Methanol versetzt, bis vollständige Lösung eintritt. Hierauf wird während 10 Minuten leicht erwärmt und anschliessend während 2 Stunden bei Raumtemperatur gerührt, wobei ein farbloser Niederschlag auskristallisiert. Dieser Niederschlag wird abfiltriert, mit eiskaltem Wasser gewaschen und getrocknet. Das erhaltene intramolekular deprotonisierte 4-Methoxy-3-methyl-2-[(sulfothio)-methyl]-1-(5,6,7,8 -tetrahydro-5,5,8,8-tetramethyl-1H--naphth[2,3-d]imidazol -2-yl)pyridiniumkation zeigt einen Schmelzpunkt von 206-208° (Zersetzung).

Beispiel 50

Eine Lösung von 4,1 g 5,7-Dihydro-2-[[(4-methoxy-3--methyl-2 -pyridyl)methyl]sulfinyl]-5,5,7,7 -tetramethyl-indeno[5,6-d]imidazol-6(1H)-on in 300 ml 1N wässeriger Salzsäure wird mit einer wässerigen Lösung von 2,8 g Natriumthiosulfatpentahydrat versetzt. Es entsteht sofort eine klare Lösung, aus welcher dann ein Festkörper auszukristallisieren beginnt. Man rührt das Reaktionsgemisch bei Raumtemperatur und kühlt dann kurz auf etwa 5° ab, worauf das kristalline Produkt abfiltriert und mit kaltem Wasser und Aether gewaschen wird. Das erhaltene intramolekular deprotonisierte 4-Methoxy-3-methyl-2-[(sulfodithio)methyl]--1-(1,5,6,7 -tetrahydro-5,5,7,7-tetramethyl-6-oxoindeno-[5,6-d]imidazol -2-yl)pyridiniumkation zeigt nach Umkristallisieren aus Methanol einen Schmelzpunkt von 198-199° (Zersetzung).

Beispiel 51

Eine Lösung von 3,4 g 2-[[(4-Methoxy-3-methyl-2-pyridyl)methyl]sulfinyl]-5 -(trifluormethyl)benzimidazol in 50 ml 1N wässeriger Salzsäure wird mit einer wässerigen Lösung von 2,5 g Natriumthiosulfatpentahydrat versetzt. Es entsteht sofort eine klare Lösung, aus welcher dann ein

Festkörper auszukristallisieren beginnt. Man rührt das Reaktionsgemisch bei Raumtemperatur und kühlt dann kurz auf etwa 5° ab, worauf das kristalline Produkt abfiltriert und mit kaltem Wasser und Aether gewaschen wird. Das Reaktionsgemisch wird an einer Kieselgelkolonne (Lösungsmittel: Methylenchlorid/Methanol [10:1]) gereinigt und aus Essigester kristallisiert. Das erhaltene intramolekular deprotonisierte [4-Methoxy-3-methyl-2-[[(sulfodithio)methyl] -5-(trifluormethyl)-2-benzimidazolyl]pyridiniumkation zeigt nach Umkristallisieren aus Methanol einen Schmelzpunkt von 186° (Zersetzung).

## Beispiel 52

Eine Lösung von 1,2 g intramolekular deprotonisiertem 4-Methoxy-1-(5-methoxy-2-benzimidazolyl)-3,5-dimethyl -2-[(sulfothio)methyl]pyridiniumkation in 20 ml Methylenchlorid und 200 ml Aethanol wird während 2 Wochen in einem offenen Kolben stehen gelassen. Das erhaltene Edukt-Produktgemisch wird an einer Silicagelkolonne gereinigt (Lösungsmittel: Methylenchlorid/Methanol, 9:1). Das gereinigte intramolekular deprotonisierte 4-Aethoxy-1-(5--methoxy-2-benzimidazolyl)-3,5-dimethyl -2-[(sulfothio)-methyl]pyridiniumkation wird aus Aethanol kristallisiert und schmilzt dann bei 161-168° (Zersetzung).

## Beispiel 53

500 mg 2-[[(o-Carboxyphenyl)dithio]methyl] -4-methoxy--3-methyl-1-(1,5,6,7-tetrahydro -5,5,7,7-tetramethyl--6-oxoindeno[5,6-d]imidazol -2-yl)pyridiniumchlorid werden mit 0,5 ml Thionylchlorid bei Raumtemperatur gerührt, worauf die Reaktionsmischung zum Teil eingeengt, mit Methanol versetzt und während 30 Minuten leicht erwärmt wird. Das auskristallisierte Material wird abgesaugt, mit Methanol gewaschen und an Kieselgel mit Methylenchlorid-Methanol (10:1) gereinigt, wobei die Methode der Mitteldruck-Flash--chromatographie verwendet und der Druck mit Stickstoffgas erzeugt wird. Das erhaltene intramolekular deprotonisierte 4-Methoxy-2-[[(o-methoxycarbonylphenyl)dithio]methyl] -3-methyl-1-(1,5,6,7-tetrahydro-5,5,7,7-tetramethyl -6-oxo-indeno[5,6-d]imidazol-2-yl]pyridiniumkation zeigt nach Kristallisation aus Essigester einen Schmelzpunkt von 132° (Zersetzung).

Beispiel 54

Eine Lösung von 700 mg Aethylmercaptan wird in einer Mischung von 20 ml 1N wässeriger Salzsäure und 100 ml Wasser bei Raumtemperatur mit 3,4 g 2-[[(3,5-Dimethyl--4-methoxy-2-pyridyl)methyl]sulfinyl] -5-methoxybenzimidazol versetzt.

Die Lösung wird 30 Minuten gerührt und dreimal mit Methylenchlorid extrahiert; die organische Lösung wird getrocknet und eingeengt. Man erhitzt den Rückstand auf dem Dampfbad und rührt anschliessend bei Raumtemperatur so lange, bis ein Festkörper vollständig auskristallisiert ist. Das kristalline Produkt wird abfiltriert, mit kaltem Essigester gewaschen und getrocknet.

Das erhaltene 2-[(Aethyldithio)methyl] -1-(5-methoxy--2-benzimidazolyl)-3,5-dimethyl -4-oxy-pyridinium zeigt einen Schmelzpunkt von 190-191.

Das als Zwischenprodukt entstehende 2-[(Aethyldithio)-methyl]-1-(5-methoxy -2-benzimidazolyl)-3,5-dimethyl -4-methoxy-pyridiniumchlorid wird nicht gefasst.

Beispiel 55

850 mg p-Nitrothiophenol werden in 20 ml etwa 6N methanolischer Salzsäure auf dem Dampfbad gelöst. Man versetzt mit 2 g 5,7-Dihydro-2-[[(4-methoxy-3-methyl -2-pyridyl)-methyl]sulfinyl] -5,5,7,7-tetramethylindeno[5,6-d]imidazol-6(1H)-on und engt mittels einer Wasserstrahlpumpe so lange ein, bis ein Niederschlag auskristallisiert. Das erhaltene 4-Methoxy-3-methyl-2-[[(p-nitrophenyl)dithio]-methyl] -1-(1,5,6,7-tetrahydro-5,5,7,7-tetramethyl -6-oxo-indeno[5,6-d]imidazol-2-yl]pyridiniumchlorid wird abfiltriert und mit kaltem Methanol gewaschen; es zeigt einen Schmelzpunkt von 185° (Zersetzung).

## Beispiel 56

Eine Lösung von 820 mg D-Penicillamin in 20 ml 1N wässeriger Salzsäure wird mit 2 g 5,7-Dihydro-2-[[(4--methoxy -3-methyl-2-pyridyl)methyl]sulfinyl] -5,5,7,7--tetramethylindeno[5,6-d]imidazol-6(1H)-on versetzt. Es entsteht eine klare Lösung, welche eingeengt und mit Essigester versetzt wird. Das Reaktionsprodukt kristallisiert aus, worauf es abfiltriert und mit Essigester gewaschen wird. Das erhaltene 2-[[[(S)-2-Amino-2-carboxy-1,1-dimethyläthyl]dithio]methyl] -4-methoxy-3-methyl-1-(1,5,6,7--tetrahydro-5,5,7,7-tetramethyl-6-oxoindeno[5,6-d]imidazol -2-yl)pyridiniumchlorid-hydrochlorid zeigt einen Schmelzpunkt von 183° (Zersetzung).

## Beispiel 57

Eine Lösung von 307 mg L-Glutathion in 10 ml 1N wässeriger Salzsäure wird mit 412 mg 5,7-Dihydro-2-[[(4--methoxy-3-methyl -2-pyridyl)methyl]sulfinyl] -5,5,7,7--tetramethylindeno[5,6-d]imidazol-6(1H)-on versetzt. Es entsteht eine klare Lösung, welche eingeengt und mit Essigester versetzt wird. Das Reaktionsprodukt kristallisiert aus, worauf es abfiltriert und mit Essigester gewaschen wird. Das erhaltene 2-[[[(R)-2-[(S)-4-Amino -4-carboxy-butyramido]-2-[(carboxymethylcarbamoyl] äthyl]dithio]-methyl] -4-methoxy-3-methyl-1-(1,5,6,7-tetrahydro --5,5,7,7-tetramethyl-6-oxoindeno[5,6-d]imidazol -2-yl)pyridiniumchlorid-hydrochlorid zeigt einen Schmelzpunkt von 202° (Zersetzung); $[\alpha]_D^{20}$ = -68,3° (Methanol, 1%).

Beispiel 58

780 mg 4-Fluorbenzylmercaptan werden in 20 ml etwa 6N methanolischer Salzsäure gelöst. Man versetzt mit 2 g 5,7-Dihydro-2-[[(4-methoxy-3-methyl-2-pyridyl)methyl] sulfinyl]-5,5,7,7-tetramethylindeno[5,6-d]imidazol-6(1H) -on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(p-Fluorbenzyl)dithio]methyl] -4-methoxy-3-methyl- -1-(1,5,6,7-tetrahydro -5,5,7,7-tetramethyl-6-oxoindeno- [5,6-d]imidazol -2-yl)pyridiniumchlorid wird abfiltriert und mit kaltem Essigester gewaschen; es zeigt einen Schmelzpunkt von 160° (Zersetzung).

Beispiel 59

625 mg 3-Aminothiophenol werden in 250 ml Tetrahydrofuran gelöst; anschliessend wird so lange gasförmiger Chlorwasserstoff eingeleitet bis der auskristallisierte Festkörper wieder in Lösung geht. Man versetzt mit 2 g 5,7-Dihydro-2-[[(4-methoxy-3-methyl -2-pyridyl)methyl]sulfinyl]-5,5,7,7-tetramethylindeno [5,6-d]imidazol-6(1H)-on und rührt die Lösung bei Raumtemperatur, wobei ein Festkörper zu kristallisieren beginnt. Ein Teil des Lösungsmittels wird eingeengt, und der auskristallisierte Festkörper wird abfiltriert und mit Tetrahydrofuran und Aether gewaschen. Das erhaltene 2-[[(m-Aminophenyl)dithio]methyl] -1-(1,5,6,7-tetrahydro-5,5,7,7-tetramethyl -6-oxoindeno- [5,6-d]imidazol-2-yl) -4-methoxy-3-methylpyridinium- chlorid-hydrochlorid zeigt einen Schmelzpunkt von 180-210°

(Zersetzung).

Beispiel 60

400 mg Cysteamin werden in 50 ml einer 3,5N Lösung von gasförmigem Chlorwasserstoff in Tetrahydrofuran gelöst. Man versetzt mit 2 g 5,7-Dihydro-2[[(4-methoxy -3-methyl--2-pyridyl)methyl]sulfinyl] -5,5,7,7-tetramethylindeno-[5,6-d]imidazol-6(1H)-on, wobei ein Festkörper auskristallisiert, welcher abfiltriert und mit Tetrahydrofuran gewaschen wird. Das erhaltene 2-[[(2-Aminoäthyl)dithio]-methyl] -4-methoxy-3-methyl-1-(1,5,6,7-tetrahydro --5,5,7,7-tetramethyl-6-oxoindeno [5,6-d]imidazol-2-yl)-pyridiniumchlorid-hydrochlorid zeigt einen Schmelzpunkt von 197-200° (Zersetzung).

Beispiel 61

625 mg 2-Aminothiophenol werden in 250 ml Tetrahydrofuran gelöst. Man rührt und erwärmt und leitet gasförmigen Chlorwasserstoff so lange ein, bis der auskristallisierte Festkörper wieder gelöst ist. Man versetzt mit 2 g 5,7-Dihydro-2[[(4-methoxy-3-methyl -2-pyridyl)methyl]sulfinyl] -5,5,7,7-tetramethylindeno[5,6-d]imidazol-6(1H)-on, wobei ein Festkörper auskristallisiert. Das Lösungsmittel wird zum Teil entfernt, und der Festkörper wird abfiltriert und mit Tetrahydrofuran gewaschen. Das erhaltene 2-[[(o-Aminophenyl)dithio]methyl] -4-methoxy-3-methyl-1-(1,5,6,7-tetrahydro -5,5,7,7-tetramethyl-6-oxoindeno[5,6-d]imidazol -2-yl)pyridiniumchlorid-dihydrochlorid zeigt einen Schmelzpunkt von 159° (Zersetzung).

Beispiel 62

Eine Lösung von 820 mg D,L-Penicillamin in 20 ml 1N wässeriger Salzsäure wird mit 2 g 5,7-Dihydro-2-[[(4--methoxy -3-methyl-2-pyridyl)methyl]sulfinyl] -5,5,7,7-

-tetramethylindeno[5,6-d]imidazol-6(1H)-on versetzt. Es entsteht eine klare Lösung, welche eingeengt und mit Essigester versetzt wird. Der auskristallisierte Festkörper wird abfiltriert und mit Essigester gewaschen. Das erhaltene 2-[[[(RS)-2-Amino-2-carboxy -1,1-dimethyläthyl]dithio]-methyl] -4-methoxy-3-methyl-1-(1,5,6,7-tetrahydro -5,5,7,7--tetramethyl -6-oxoindeno[5,6-d]imidazol-2-yl)pyridiniumchlorid zeigt einen Schmelzpunkt von 176° (Zersetzung).

## Beispiel 63

680 mg o-Thiokresol werden in 30 ml etwa 6N methanolischer Salzsäure gelöst. Man versetzt mit 2 g 5,7-Dihydro--2-[[(4-methoxy-3-methyl -2-pyridyl)methyl]sulfinyl] --5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy-3-methyl-1-(1,5,6,7-tetrahydro -5,5,7,7-tetramethyl-6-oxoindeno[5,6-d]imidazol -2-yl)-2-[(o-tolyldithio)methyl]pyridiniumchlorid wird abfiltriert und mit kaltem Essigester gewaschen; es zeigt einen Schmelzpunkt von 179° (Zersetzung).

## Beispiel 64

850 mg p-Methoxybenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy-2-[[(p-methoxybenzyl)dithio]methyl] -3-methyl--1-(1,5,6,7-tetrahydro-5,5,7,7-tetramethyl -6-oxoindeno-

[5,6-d]imidazol -2-yl]pyridiniumchlorid-hydrochlorid wird abfiltriert und mit kaltem Essigester gewaschen; es zeigt einen Schmelzpunkt von 159-160° (Zersetzung).

## Beispiel 65

1 g m-Trifluormethylbenzylmercaptan wird in 10 ml Methanol und in 10 ml etwa 6N methanolischer Salzsäure gelöst. Man versetzt mit 2 g 5,7-Dihydro-2-[[(4-methoxy--3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy--3-methyl-1-(1,5,6,7-tetrahydro -5,5,7,7-tetramethyl--6-oxoindeno[5,6-d]imidazol -2-yl)-2-[[[m-(trifluormethyl)-benzyl]dithio]methyl] pyridiniumchlorid wird abfiltriert und mit kaltem Essigester gewaschen; es zeigt einen Schmelzpunkt von 162° (Zersetzung).

## Beispiel 66

0,8 ml 2-Phenyl-äthylmercaptan werden in 30 ml einer 3,5N Lösung von gasförmigem Chlorwasserstoff in Tetrahydrofuran gelöst. Man versetzt mit 2 g 5,7-Dihydro-2[[(4--methoxy -3-methyl-2-pyridyl)methyl]sulfinyl] --5,5,7,7-tetramethylindeno[5,6-d]imidazol-6(1H)-on, rührt die Lösung bei Raumtemperatur und engt sie dann mittels einer Wasserstrahlpumpe so lange ein, bis ein Niederschlag auszukristallisieren beginnt. Der Niederschlag wird abfiltriert und mit Tetrahydrofuran gewaschen. Das erhaltene 4-Methoxy-3-methyl-2-[(phenäthyldithio)methyl] -1-(1,5,6,7--tetrahydro-5,5,7,7-tetramethyl -6-oxoindeno[5,6-d]imidazol-2-yl]pyridiniumchlorid zeigt einen Schmelzpunkt von

0214479

165° (Zersetzung).

## Beispiel 67

870 mg 4-Chlorbenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(p-Chlorbenzyl)dithio]methyl] -4-methoxy-3-methyl--1-(1,5,6,7-tetrahydro -5,5,7,7-tetramethyl-6-oxoindeno-[5,6-d]imidazol -2-yl)pyridiniumchlorid wird abfiltriert und mit kaltem Essigester gewaschen; es zeigt einen Schmelzpunkt von 178° (Zersetzung).

## Beispiel 68

730 mg 2-Mercapto-5-methyl-1,3,4-thiadiazol werden in 20 ml etwa 6N methanolischer Salzsäure gelöst. Man versetzt mit 2 g 5,7-Dihydro-2-[[(4-methoxy-3-methyl -2-pyridyl)-methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy-2-[[(5-methyl-1,3,4-thiadiazol -2-yl)dithio]methyl] -3-methyl-1-(1,5,6,7-tetrahydro--5,5,7,7-tetramethyl -6-oxoindeno[5,6-d]imidazol -2-yl)-pyridiniumchlorid-dihydrochlorid wird abfiltriert und mit kaltem Essigester gewaschen, es zeigt einen Schmelzpunkt von 145° (Zersetzung).

0214479

## Beispiel 69

930 mg m-Nitrobenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristalliation weiter. Das erhaltene 4-Methoxy-3-methyl -2-[[(m-nitrobenzyl)dithio]methyl] -1-(1,5,6,7-tetrahydro-5,5,7,7-tetramethyl -6-oxoindeno-[5,6-d]imidazol -2-yl)pyridiniumchlorid wird abfiltriert und mit kaltem Essigester gewaschen; es zeigt einen Schmelzpunkt von 166° (Zersetzung).

## Beispiel 70

1,06 g 3,4-Dichlorbenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(3,4-Dichlorbenzyl)dithio]methyl] -4-methoxy--3-methyl-1-(1,5,6,7-tetrahydro -5,5,7,7-tetramethyl--6-oxoindeno[5,6-d]imidazol -2-yl)pyridiniumchlorid wird abfiltriert und mit kaltem Essigester gewaschen; es zeigt einen Schmelzpunkt von 180° (Zersetzung).

## Beispiel 71

106 mg 2,4-Dichlorbenzylmercaptan werden in 5 ml etwa 6N methanolischer Salzsäure gelöst. Man versetzt mit 200 mg 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 2 ml Essigester zu und rührt das Reaktionsgemisch bis zur beendeten Kristallisation weiter. Das erhaltene 2-[[(2,4-Dichlorbenzyl)dithio]methyl] -4-methoxy-3-methyl- -1-(1,5,6,7-tetrahydro -5,5,7,7-tetramethyl-6-oxoindeno- [5,6-d]imidazol -2-yl)pyridiniumchlorid wird abfiltriert und mit kaltem Essigester gewaschen; es zeigt einen Schmelzpunkt von 183° (Zersetzung).

Beispiel 72

600 mg 2-Chlorallylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(2-Chlorallyl)dithio]methyl] -4-methoxy-3-methyl- -1-(1,5,6,7-tetrahydro -5,5,7,7-tetramethyl-6-oxoindeno- [5,6-d]imidazol -2-yl)pyridiniumchlorid wird abfiltriert und mit kaltem Essigester gewaschen; es zeigt einen Schmelzpunkt von 176° (Zersetzung).

Beispiel 73

930 mg o-Nitrobenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch

bis zur beendeten Kristallisation weiter. Das erhaltene 4-Methoxy-3-methyl-2-[[(o-nitrobenzyl)dithio]methyl] -1-(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 175° (Zersetzung).

Beispiel 74

630 mg 3-Mercapto-4-methyl-4H-1,2,4-triazol werden in 20 ml etwa 6N methanolischer Salzsäure gelöst. Man versetzt mit 2 g 5,7-Dihydro-2-[[(4-methoxy-3-methyl -2-pyridyl)-methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy-2-[[(4-methyl-4H -1,2,4-triazol--3-yl)dithio]methyl] -3-methyl-1-(1,5,6,7-tetrahydro--5,5,7,7-tetramethyl -6-oxoindeno[5,6-d]imidazol --2-yl)pyridiniumchlorid-dihydrochlorid wird abfiltriert und mit kaltem Essigester gewaschen; es zeigt einen Schmelzpunkt von 190° (Zersetzung).

Beispiel 75

920 mg 4-Acetylamino-thiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[(p-Acetanilidodithio)methyl] -4-methoxy-3-methyl--1-(1,5,6,7-tetrahydro -5,5,7,7-tetramethyl-6-oxoindeno-[5,6-d]imidazol -2-yl)pyridiniumchlorid wird abfiltriert

und mit kaltem Essigester gewaschen; es zeigt einen Schmelzpunkt von 200° (Zersetzung).

## Beispiel 76

770 mg 2-Methoxy-thiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kritallisation weiter. Das erhaltene 4-Methoxy-2-[[(o-methoxyphenyl)dithio]methyl] -3-methyl--1-(1,5,6,7-tetrahydro -5,5,7,7-tetramethyl-6-oxoindeno-[5,6-d]imidazol -2-yl)pyridiniumchlorid wird abfiltriert und mit kaltem Essigester gewaschen; es zeigt einen Schmelzpunkt von 176° (Zersetzung).

## Beispiel 77

980 mg 3,4-Dichlorthiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(3,4-Dichlorphenyl)dithio]methyl] -4-methoxy-3-methyl -1-(1,5,6,7-tetrahydro -5,5,7,7-tetramethyl-6-oxoindeno-[5,6-d]imidazol -2-yl)pyridiniumchlorid wird abfiltriert und mit kaltem Essigester gewaschen; es zeigt einen Schmelzpunkt von 182° (Zersetzung).

## Beispiel 78

870 mg o-Chlorbenzylmercaptan werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(o-Chlorbenzyl)dithio]methyl] -4-methoxy-3-methyl--1-(1,5,6,7-tetrahydro -5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 183° (Zersetzung).

## Beispiel 79

90 mg m-Trifluormethyl-thiophenol werden in 5 ml einer 3,5N Lösung von gasförmigem Chlorwasserstoff in Tetrahydrofuran gelöst. Man versetzt mit 200 mg 5,7-Dihydro-2-[[(4--methoxy-3-methyl -2-pyridyl)methyl]sulfinyl] -5,5,7,7--tetramethylindeno[5,6-d]imidazol-6(1H)-on, worauf die Lösung teilweise eingeengt und mit Essigester versetzt wird. Der auskristallisierte Festkörper wird abfiltriert und mit Essigester gewaschen. Das erhaltene 4-Methoxy--3-methyl-1-(1,5,6,7-tetrahydro -5,5,7,7-tetramethyl-6-oxo-indeno[5,6-d]imidazol -2-yl)-2-[[[m-(trifluormethyl)-phenyl] dithio]methyl]pyridiniumchlorid zeigt einen Schmelzpunkt von 166° (Zersetzung).

## Beispiel 80

980 mg 1-Phenyl-5-mercapto-tetrazol werden in 20 ml etwa 6N methanolischer Salzsäure gelöst. Man versetzt mit 2 g 5,7-Dihydro-2-[[(4-methoxy-3-methyl -2-pyridyl)methyl]-sulfinyl] -5,5,7,7-tetramethylindeno[5,6-d]imidazol-6(1H)-

-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 4-Methoxy-3-methyl-2-[[(1-phenyl -1H-tetrazol-5-yl)dithio]methyl] -1-(1,5,6,7-tetrahydro-5,5,7,7-tetramethyl -6-oxoindeno[5,6-d]imidazol -2-yl)pyridiniumchlorid wird abfiltriert und mit kaltem Essigester gewaschen; es zeigt einen Schmelzpunkt von 150° (Zersetzung).

## Beispiel 81

940 mg 4-Hydroxy-2-mercapto-6-propylpyrimidin werden in 20 ml etwa 6N methanolischer Salzsäure gelöst. Man versetzt mit 2 g 5,7-Dihydro-2-[[(4-methoxy-3-methyl -2-pyridyl)-methyl]sulfinyl] -5,5,7,7-tetramethylindeno[5,6-d]-imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(4-Hydroxy-6-propyl -2-pyrimidinyl)dithio]methyl] -4-methoxy-3-methyl-1-(1,5,6,7-tetrahydro -5,5,7,7-tetramethyl -6-oxoindeno[5,6-d]imidazol-2-yl)pyridiniumchlorid wird abfiltriert und mit kaltem Essigester gewaschen; es zeigt einen Schmelzpunkt von 170° (Zersetzung).

## Beispiel 82

Eine Lösung von 750 mg 2-Mercaptobenzimidazol in 10 ml 1N wässeriger Salzsäure und 30 ml Methanol wird mit 2,05 g 5,7-Dihydro-2-[[(4-methoxy-3-methyl -2-pyridyl)methyl]sulfinyl] -5,5,7,7-tetramethylindeno[5,6-d]imidazol-6(1H)-on versetzt. Es entsteht eine klare Lösung, welche eingeengt und mit Essigester versetzt wird. Der auskristallisierte Festkörper wird abfiltriert und mit Essigester gewaschen. Das erhaltene 2-[(2-Benzimidazolyldithio)methyl] -

-4-methoxy-3-methyl -1-(1,5,6,7-tetrahydro-5,5,7,7-tetramethyl -6-oxoindeno[5,6-d]imidazol -2-yl)pyridiniumchlorid-hydrochlorid zeigt einen Schmelzpunkt von 161-175° (Zersetzung).

## Beispiel 83

1,04 g 4-Bromthiophenol werden in 20 ml etwa 6N methanolischer Salzsäure gelöst. Man versetzt mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(p-Bromphenyl)dithio]methyl] -4-methoxy-3-methyl--1-(1,5,6,7-tetrahydro -5,5,7,7-tetramethyl -6-oxoindeno-[5,6-d]imidazol -2-yl)pyridiniumchlorid wird abfiltriert und mit kaltem Essigester gewaschen; es zeigt einen Schmelzpunkt von 170° (Zersetzung).

## Beispiel 84

Eine Lösung von 610 mg 4-Mercaptopyridin in 15 ml 1N wässeriger Salzsäure und 30 ml Methanol wird mit 2 g 5,7-Dihydro-2-[[(4-methoxy-3-methyl -2-pyridyl)methyl]sulfinyl] -5,5,7,7-tetramethylindeno[5,6-d]imidazol-6(1H)-on versetzt. Es entsteht eine klare Lösung, welche eingeengt und mit Essigester vesetzt wird. Der auskristallisierte Festkörper wird abfiltriert und mit Essigester gewaschen. Das erhaltene 4-Methoxy-3-methyl-2-[(4-pyridyldithio)-methyl] -1-(1,5,6,7-tetrahydro -5,5,7,7-tetramethyl-6-oxo-indeno[5,6-d]imidazol -2-yl)pyridiniumchlorid-hydrochlorid zeigt einen Schmelzpunkt von 180° (Zersetzung).

Beispiel 85

77 mg 4-Methoxy-thiophenol werden in 5 ml etwa 6N methanolischer Salzsäure gelöst. Man versetzt mit 200 mg 5,7-Dihydro-2-[[(4-methoxy-3-methyl -2-pyridyl)methyl]sulfinyl] -5,5,7,7-tetramethylindeno[5,6-d]imidazol-6(1H)-on, rührt die Lösung bei Raumtemperatur und engt dann mittels einer Wasserstrahlpumpe teilweise ein. Anschliessend gibt man etwa 20 ml Essigester zu und rührt das Reaktionsgemisch bis zur beendeten Kristallisation weiter. Das erhaltene 2-[[(p-Methoxyphenyl)dithio]methyl] -4-methoxy-3-methyl] -1-(1,5,6,7-tetrahydro -5,5,7,7-tetramethyl-6-oxoindeno-[5,6-d]imidazol -2-yl)pyridiniumchlorid wird abfiltriert und mit kaltem Essigester gewaschen; es zeigt einen Schmelzpunkt von 166° (Zersetzung).

Beispiel 86

Eine Lösung von 3,59 g 1-Octadecanthiol in 45 ml 1N wässeriger Salzsäure und 170 ml Methanol wird mit 4,5 g 5,7-Dihydro-2-[[(4-methoxy-3-methyl -2-pyridyl)methyl]sulfinyl]-5,5,7,7-tetramethylindeno [5,6-d]imidazol-6(1H)-on versetzt. Es entsteht eine klare Lösung. Man rührt das Reaktionsgemisch bei Raumtemperatur, engt zum Teil ein und kühlt die Lösung kurz auf etwa 5° ab, worauf der kristalline Festkörper abfiltriert und mit Wasser gewaschen wird. Das erhaltene 4-Methoxy-3-methyl-2-[(octadecyldithio)-methyl]-1-(1,5,6,7 -tetrahydro-5,5,7,7-tetramethyl-6-oxoindeno[5,6-d]imidazol -2-yl)pyridiniumchlorid zeigt einen Schmelzpunkt von 150° (Zers.).

Beispiel 87

Eine Lösung von 0,98 g 2-Mercapto-5-nitrobenzimidazol in 20 ml 1N wässeriger Salzsäure und 70 ml Methanol wird mit 2 g 5,7-Dihydro-2-[[(4-methoxy-3-methyl-2 -pyridyl)-methyl]sulfinyl]-5,5,7,7-tetramethylindeno[5,6-d] imidazol-6-(1H)-on versetzt. Es entsteht eine klare Lösung. Man rührt das Reaktionsgemisch bei Raumtemperatur, engt ein, löst den Rückstand in Methanol/Essigester und kühlt die Lösung kurz auf etwa 5° ab, worauf der kristalline Festkörper abfiltriert und mit Essigester gewaschen wird. Das erhaltene 4-Methoxy-3-methyl-2-[[(5-nitro -2-benzimidazolyl)dithio]methyl]-1-(1,5,6,7-tetrahydro -5,5,7,7-tetramethyl-6-oxoindeno[5,6-d]imidazol -2-yl)pyridiniumchlorid zeigt einen Schmelzpunkt von 188° (Zers.).

Beispiel 88

Eine Lösung von 1,61 g tert.Octylmercaptan in 30 ml 1N wässeriger Salzsäure und 160 ml Methanol wird mit 4 g 5,7-Dihydro-2-[[(4-methoxy-3-methyl -2-pyridyl)methyl]sulfinyl]-5,5,7,7-tetramethylindeno[5,6-d] imidazol-6(1H)-on versetzt. Es entsteht eine klare Lösung. Man rührt das Reaktionsgemisch bei Raumtemperatur, engt zum Teil ein und kühlt die Lösung kurz auf etwa 5° ab, worauf der kristalline Festkörper abfiltriert und mit Wasser gewaschen wird. Das erhaltene 4-Methoxy-3-methyl-2-[(tert.octyldithio)-methyl] -1-(1,5,6,7-tetrahydro-5,5,7,7-tetramethyl -6-oxo-indeno[5,6-d]imidazol-2-yl)pyridiniumchlorid zeigt einen Schmelzpunkt von 150° (Zers.).

Beispiel 89

Eine Lösung von 1,09 g n-Heptylmercaptan in 30 ml 1N wässeriger Salzsäure und 180 ml Methanol wird mit 3 g 5,7-Dihydro-2-[[(4-methoxy-3-methyl -2-pyridyl)methyl]sulfinyl]-5,5,7,7-tetramethylindeno[5,6-d] imidazol-6(1H)-on versetzt. Es entsteht eine klare Lösung. Man rührt das Reaktionsgemisch bei Raumtemperatur, engt zum Teil ein und kühlt die Mischung kurz auf etwa 5° ab, worauf der kristalline Festkörper abfiltriert und mit Wasser gewaschen wird. Das erhaltene 2-[(Heptyldithio)methyl]-4-methoxy-3-methyl -1-(1,5,6,7-tetrahydro-5,5,7,7-tetramethyl -6-oxoindeno-

 

[5,6-d]imidazol-2-yl)pyridiniumchlorid zeigt einen Schmelzpunkt von 150° (Zers.).

Beispiel 90

0,8 g Cyclohexylmercaptan werden in 25 ml etwa 6N methanolischer Salzsäure gelöst. Man versetzt mit 2,5 g 5,7-Dihydro-2-[[(4-methoxy-3-methyl -2-pyridyl)methyl]sulfinyl]-5,5,7,7-tetramethylindeno[5,6-d] imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[(Cyclohexyldithio)methyl]-4-methoxy-3-methyl-1 -(1,5,6,7-tetrahydro-5,5,7,7-tetramethyl-6-oxoindeno[5,6-d] imidazol-2-yl)pyridiniumchlorid wird abfiltriert und mit kaltem Essigester gewaschen; es zeigt einen Schmelzpunkt von 200° (Zers.).

Beispiel 91

0,98 g 2,5-Dichlorthiophenol werden in 50 ml etwa 6N methanolischer Salzsäure gelöst. Man versetzt mit 2 g 5,7-Dihydro-2-[[(4-methoxy-3-methyl -2-pyridyl)methyl]sulfinyl]-5,5,7,7-tetramethylindeno[5,6-d] imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[[(2,5-Dichlorphenyl)dithio]methyl]-4-methoxy-3-methyl-1 -(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 169° (Zers.).

## Beispiel 92

0,98 g 2,6-Dichlorthiophenol werden in 50 ml etwa 6N methanolischer Salzsäure gelöst. Man versetzt mit 2 g 5,7-Dihydro-2-[[(4-methoxy-3-methyl -2-pyridyl)methyl]sulfinyl]-5,5,7,7-tetramethylindeno[5,6-d] imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Essigester zu und rührt das Reaktionsgemisch bis zur beendeten Kristallisation weiter. Das erhaltene 2-[[(2,6-Dichlorphenyl)dithio]methyl]-4-methoxy-3-methyl-1-(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 168° (Zers.).

## Beispiel 93

0,71 g 1-Octanthiol werden in 50 ml etwa 6N methanolischer Salzsäure gelöst. Man versetzt mit 2 g 5,7-Dihydro-2-[[(4-methoxy-3-methyl -2-pyridyl)methyl]sulfinyl]-5,5,7,7-tetramethylindeno[5,6-d] imidazol-6(1H)-on, rührt die Lösung bei Raumtemperatur und engt dann mittels einer Wasserstrahlpumpe so lange ein, bis ein Niederschlag auszukristallisieren beginnt. In diesem Moment gibt man etwa 20 ml Aether zu und rührt das Reaktionsgemisch bis zur beendeten Kristallisation weiter. Das erhaltene 4-Methoxy-3-methyl-2-[(octyldithio)methyl]-1-(1,5,6,7 -tetrahydro-5,5,7,7-tetramethyl-6-oxoindeno[5,6-d]imidazol -2-yl)pyridiniumchlorid wird abfiltriert und mit kaltem Aether gewaschen; es zeigt einen Schmelzpunkt von 164° (Zers.).

## Beispiel 94

Eine Lösung von 750 mg Thioäpfelsäure in 15 ml 1N wässeriger Salzsäure wird mit 1,73 g 6-[[(4-Methoxy-3-methyl-

-2-pyridyl)methyl]sulfinyl] -5H-1,3-dioxolo[4,5-f]benzimidazol versetzt. Es entsteht sofort eine Lösung, welche kurz auf 40° erwärmt wird und aus welcher ein Festkörper auszukristallisieren beginnt. Man rührt das Reaktionsgemisch 4 Stunden bei Raumtemperatur und kühlt dann kurz auf etwa 5° ab, worauf das kristalline Produkt abfiltriert und mit kaltem Wasser und Aether gewaschen wird. Das erhaltene 2-[[(1,2-Dicarboxyäthyl)dithio]methyl]-4-methoxy-3-methyl -1-(5H-1,3-dioxolo[4,5-f]benzimidazol-2-yl)pyridiniumchlorid zeigt einen Schmelzpunkt von 192° (Zers.).

## Beispiel 95

Eine Lösung von 590 mg Hexanthiol in 15 ml 1N wässeriger Salzsäure wird mit 1,73 g 6-[[(4-Methoxy-3-methyl--2-pyridyl)methyl]sulfinyl]-5H -1,3-dioxolo[4,5-f]benzimidazol versetzt. Die Lösung wird auf 40-50° erhitzt, und nach etwa 10 Minuten wird das Wasser abdestilliert. Man nimmt den Rückstand in Methanol auf, engt die Lösung ein, nimmt den Rückstand in Essigester auf und lässt bei etwa 5° kristallisieren, worauf das kristalline Produkt abfiltriert und mit kaltem Essigester gewaschen wird. Das erhaltene 1-(5H-1,3-Dioxolo[4,5-f]benzimidazol-2-yl) -2-[(hexyldithio)methyl]-4-methoxy-3-methylpyridiniumchlorid zeigt einen Schmelzpunkt von 135° (Zers.).

## Beispiel 96

Eine Lösung von 2 g 1-Dodecylthiol in 20 ml 1N wässeriger Salzsäure und 100 ml Methanol wird mit 3,4 g 6-[[(4-Methoxy-3-methyl-2-pyridyl)methyl]sulfinyl]-5H -1,3-dioxolo[4,5-f]benzimidazol versetzt. Es entsteht eine klare Lösung. Man rührt das Reaktionsgemisch bei Raumtemperatur, dampft das Lösungsmittel ab, löst den Rückstand dreimal in Methanol und dampft das Lösungsmittel stets wieder ab, worauf das Reaktionsprodukt aus Essigester kristallisiert und mit kaltem Essigester gewaschen wird.

Das erhaltene 1-(5H-1,3-Dioxolo[4,5-f]benzimidazol-6-yl) -2-[(dodecyldithio)methyl]-4-methoxy-3 -methyl]pyridiniumchlorid zeigt einen Schmelzpunkt von 138-140° (Zers.).

Beispiel 97

Eine Lösung von 560 mg 2-Mercaptopyrimidin in 15 ml 1N und 1 ml 5N wässeriger Salzsäure wird mit 50 ml Methanol und 1,7 g 6-[[(4-Methoxy-3-methyl-2-pyridyl)methyl]sulfinyl]-5H -1,3-dioxolo[4,5-f]benzimidazol versetzt. Nach 30-minütigem Rühren bei 40° dampft man das Lösungsmittelgemisch ab, löst den Rückstand dreimal in Methanol und dampft das Lösungsmittel stets wieder ab. Das Reaktionsprodukt wird aus wenig Methanol/Essigester kristallisiert und mit kaltem Essigester gewaschen. Das erhaltene 1-(5H-1,3-Dioxolo[4,5-f]benzimidazol-6-yl)-4-methoxy-3-methyl -2-[(2-pyrimidinyldithio)methyl]pyridiniumchlorid -hydrochlorid zeigt einen Schmelzpunkt von 158-160° (Zers.).

Beispiel 98

Eine Lösung von 0,35 g L-Cystein in 5,8 ml 1N wässeriger Salzsäure und 35 ml Wasser wird mit 1 g 2-[[(3,5-Dimethyl-4-methoxy-2-pyridyl)methyl]sulfinyl] -5-methoxybenzimidazol versetzt und im Ultraschallbad gelöst. Die Lösung wird lyophilisiert. Das erhaltene 2-[[(2-Amino-2-carboxyäthyl)dithio]methyl]-4-methoxy-1 (5-methoxy-2-benzimidazolyl)-3,5 -dimethylpyridiniumchlorid-hydrochlorid wird bei -20° aufbewahrt. Das Kation des Salzes wird in MS-FAB (Matrix: 3-Nitro-benzylalkohol) bestimmt und liefert die Masse 449.

Beispiel 99

Eine Lösung von 870 mg Thioäpfelsäure in 6 ml 1N wässeriger Salzsäure und 10 ml Wasser wird mit 2 g 2-[[(3,5-Dimethyl-4-methoxy-2-pyridyl)methyl]sulfinyl] -5-methoxybenz-

imidazol versetzt und im Ultraschallbad gelöst. Die Lösung wird lyophilisiert. Das erhaltene 2-[[(1,2-Dicarboxyäthyl)-dithio]methyl]-4-methoxy-1 -(5-methoxy-2-benzimidazolyl)--3,5-dimethylpyridiniumchlorid wird bei -20° aufbewahrt und liefert die folgende Mikroanalyse:

Ber. ($H_2O$-frei; mit 0,32 HCl)
C 47,98; H 4,66; N 7,99; S 12,20; Cl 8,90
Gef. C 47,69; H 4,66; N 7,87; S 12,24; Cl 8,65.

## Beispiel 100

Eine Lösung von 650 mg 2-Mercaptopyrimidin in 12 ml 1N wässeriger Salzsäure und 10 ml Wasser wird mit 2 g 2-[[(3,5-Dimethyl-4-methoxy-2-pyridyl)methyl]sulfinyl]-5-methoxybenzimidazol versetzt und im Ultraschallbad gelöst. Die Lösung wird lyophilisiert. Das erhaltene 4-Methoxy-1-(5-methoxy-2-benzimidazolyl)-3,5-dimethyl-2-[(2-pyrimidinyldithio)- methyl]pyridiniumchlorid -hydrochlorid wird bei -20° aufbewahrt. Das Kation des Salzes wird in MS-FAB (Matrix: 3-Nitro-benzylalkohol) bestimmt und liefert die Masse 440.

## Beispiel 101

Eine Lösung von 0,39 g D,L-Homocystein in 6,9 ml 1N wässeriger Salzsäure und 30 ml Wasser wird mit 1 g 2-[[(3,5-Dimethyl-4-methoxy-2-pyridyl)methyl]sulfinyl]-5-methoxybenzimidazol versetzt und im Ultraschallbad gelöst. Die Lösung wird lyophilisiert. Das erhaltene 2-[[(3--Amino-3-carboxypropyl)dithio]methyl]-4-methoxy-1 --(5-methoxy-2-benzimidazolyl)-3,5 -dimethylpyridiniumchlorid-hydrochlorid wird bei -20° aufbewahrt. Das Kation des Salzes wird in MS-FAB (Matrix: 3-Nitro-benzylalkohol) bestimmt und liefert die Masse 463.

Beispiel 102

a) Zu 500 ml einer 5%igen Lösung von Methyllithium in Aether tropft man bei Raumtemperatur unter Argon 1200 ml Aether, anschliessend 35,6 g 3,5-Lutidin (3,5-Dimethylpyridin) und schliesslich 400 ml Toluol zu. Der Aether wird vollständig abdestilliert, worauf die Lösung während 4 Stunden bei 100° gerührt wird. Unter Methanol/Eis-Kühlung gibt man dann portionenweise Eis zu, bis keine Wärmeentwicklung mehr eintritt. Die Toluol-Phase wird vom ausgefallenen Festkörper abgetrennt und mit 66 ml halbkonzentrierter Salzsäure extrahiert. Die abgetrennte wässrige Phase stellt man mit 3N Natronlauge unter Kühlung auf ein pH von etwa 10 und extrahiert sie zweimal mit 300 ml Aether. Die Aetherextrakte werden über Natriumsulfat getrocknet und eingedampft. Der Rückstand wird im Vakuum bei 20 mm/72-74° destilliert; man erhält 2,3,5-Collidin (2,3,5-Trimethylpyridin), welches gemäss Gaschromatogramm eine 99,15%ige Reinheit aufweist.

b) Zu 246,4 g 2,3,5-Collidin und 2400 ml Eisessig werden bei Raumtemperatur 420 ml 30%iges Wasserstoffperoxyd zugetropft. Die Lösung wird über Nacht bei 80° gerührt. Dann kühlt man das Reaktionsgemisch auf 40° ab, gibt nochmals 420 ml 30%iges Wasserstoffperoxyd zu und erhitzt für weitere 24 Stunden auf 80°. Nach Eindampfen im Vakuum wird der Rückstand in 300 ml Wasser gelöst, worauf die Lösung unter Kühlung mit konz. Natronlauge basisch gestellt, mit Natriumchlorid gesättigt und dreimal mit 1 l Methylenchlorid extrahiert wird. Die organischen Phasen werden über Natriumsulfat getrocknet und im Vakuum eingedampft. Der Rückstand wird aus Aether/Petroläther kristallisiert; man erhält 2,3,5-Trimethylpyridin-1-oxid vom Schmelzpunkt 42-44°.

c) Zu 210 ml konz. Schwefelsäure tropft man unter Kühlung 65 ml rauchende Salpetersäure (d=1,5) zu. Anschliessend werden bei 0-5° portionenweise 96,5 g 2,3,5-Trimethylpyridin-1-oxid zugegeben, worauf das Gemisch 1 Stunde bei Raumtemperatur gerührt, dann innerhalb von 3 Stunden auf 90° aufgeheizt und über Nacht bei dieser Temperatur belassen wird. Nach dem Abkühlen wird die Lösung auf 1,5 kg Eis gegossen, worauf mit konz. Natronlauge auf pH 3 gestellt und dreimal mit 500 ml Methylenchlorid extrahiert wird. Die vereinigten organischen Phasen werden mit 1 l Wasser gewaschen, über Natriumsulfat getrocknet und im Vakuum eingedampft. Der Rückstand wird aus Aether/Petroläther kristallisiert, und man erhält 2,3,5-Trimethyl--4-nitropyridin-1-oxid vom Schmelzpunkt 76-78°.

d) 22,6 g Natrium werden unter Argon in 4 l Methanol gelöst. Dann gibt man portionenweise 120 g 2,3,5-Trimethyl--4-nitropyridin-1-oxid zu und lässt die Lösung über Nacht unter Rückfluss kochen. Man stellt das pH unter Kühlung mittels 5N Chlorwasserstoff in Essigester auf 7 und dampft dann das Gemisch im Vakuum ein. Man nimmt den Rückstand in 1,5 l Methylenchlorid auf, filtriert die Lösung durch Kieselgur, das noch mit 0,5 l Methylenchlorid nachgewaschen wird, dampft die vereinigten Filtrate im Vakuum ein und kristallisiert den Rückstand aus Petroläther. Man erhält 4-Methoxy-2,3,5-trimethylpyridin-1-oxid vom Schmelzpunkt 48-50°.

e) Zu einer Lösung von 81,5 g 4-Methoxy-2,3,5-trimethylpyridin-1-oxid in 290 ml Chloroform werden bei Raumtemperatur 215 ml Acetanhydrid zugetropft. Nach 4 Stunden Kochen unter Rückfluss dampft man die Lösung ein, löst den Rückstand in 200 ml Toluol und dampft erneut ein. Der Rückstand wird in 500 ml Essigester aufgenommen und dreimal mit 250 ml gesättigter Natriumbicarbonatlösung ausgeschüttelt. Die organische Phase wird über Natriumsulfat getrocknet und im Vakuum eingedampft. Das als Rückstand verbleibende Rohprodukt wird an 400 g Kieselgel mit Aether chromatographiert. Man erhält (4-Methoxy-3,5-dimethyl-2-pyridyl)-methylacetat in Form eines Oels.

f) 94,9 g (4-Methoxy-3,5-dimethyl-2-pyridyl)methylacetat werden in 570 ml Aethanol gelöst. Dann tropft man bei 0° 285 ml 3N Natronlauge zu und rührt das Gemisch 3 Stunden bei Raumtemperatur. Anschliessend wird das Aethanol im Vakuum entfernt, worauf man die zurückgebliebene wässrige Lösung dreimal mit 300 ml Methylenchlorid extrahiert. Die organischen Auszüge werden über Natriumsulfat getrocknet und im Vakuum eingedampft. Der Rückstand wird aus Petroläther kristallisiert, und man erhält 4-Methoxy-3,5-dimethyl-2-pyridylmethanol vom Schmelzpunkt 49-51°.

g) Zu 38 ml Thionylchlorid in 400 ml Methylenchlorid werden bei 0° 75,8 g 4-Methoxy-3,5-dimethyl-2-pyridylmethanol, gelöst in 200 ml Methylenchlorid, zugetropft. Nach 16-stündigem Rühren bei Raumtemperatur tropft man unter Kühlung 1800 ml Aether zu und rührt das Gemisch 2 Stunden bei Raumtemperatur weiter. Die ausgefallenen Kristalle werden abgenutscht und mit Aether gewaschen. Man erhält 2-(Chlormethyl)-4-methoxy-3,5-dimethylpyridin-hydrochlorid vom Schmelzpunkt 130-131°.

h) 18,0 g (69,2 mMol) 5,7-Dihydro-2-mercapto-5,5,7,7-tetramethylindeno[5,6-d] imidazol-6(1H)-on werden in 400 ml Alkohol suspendiert und unter Eiskühlung mit 15,6 g (70,2 mMol) 2-(Chlormethyl)-4-methoxy-3,5-dimethylpyridin-hydrochlorid versetzt. Hernach tropft man eine Lösung von 5,6 g Natriumhydroxyd in 150 ml Wasser zu, lässt das Gemisch über Nacht am Rückfluss kochen und dampft es anschliessend im Vakuum zur Trockene ein. Der Rückstand wird in 1000 ml Methylenchlorid gelöst; die Lösung wird zuerst mit 500 ml 1,5N Natronlauge und dann mit 3 x 500 ml Wasser gewaschen, über Natriumsulfat getrocknet und im Vakuum eingedampft. Das Rohprodukt wird an 300 g Kieselgel mit Essigester/Methylenchlorid (1:1) als Eluierungsmittel gereinigt. Kristallisation aus Methylenchlorid/Petroläther ergibt 5,7-Dihydro-2-[[(4-methoxy-3,5-dimethyl 2-pyridyl)methyl]-thio]-5,5,7,7-tetramethylindeno[5,6-d] imidazol-6(1H)-on vom Schmelzpunkt 166-168°.

i) 8,3 g 5,7-Dihydro-2-[[(4-methoxy-3,5-dimethyl -2-pyridyl)methyl]thio]-5,5,7,7-tetramethylindeno[5,6-d] imidazol-6(1H)-on werden in 1000 ml Methylenchlorid gelöst und mit einem Eis/Methanol-Bad auf -10° gekühlt. Dann werden innerhalb von 25 Minuten 4,3 g aus Methylenchlorid/Petroläther umkristallisierte m-Chlorperbenzoesäure eingetragen. Man rührt die Lösung bei -10° noch 45 Minuten weiter und giesst sie dann in ein Gemisch von 100 ml 2N Natriumcarbonatlösung und Eis. Die wässerige Phase wird zweimal mit 300 ml Methylenchlorid extrahiert. Die vereinigten organischen Phasen werden dreimal mit 200 ml Wasser neutralgewaschen, über Natriumsulfat getrocknet und im Vakuum bei 35° auf ein Volumen von 150 ml eingeengt. Bei Zugabe von Petroläther kristallisiert 5,7-Dihydro-2-[[(4-methoxy--3,5-dimethyl-2 -pyridyl)methyl]sulfinyl]-5,5,7,7-tetramethylindeno[5,6-d] imidazol-6(1H)-on vom Schmelzpunkt 192-194°.

j) 576 mg 2-Chlorthiophenol werden in 5 ml einer 3,5N Lösung von gasförmigem Chlorwasserstoff in Tetrahydrofuran und 10 ml Tetrahydrofuran gelöst. Man versetzt die Lösung mit 1,7 g 5,7-Dihydro-2-[[(4-methoxy -3,5-dimethyl--2-pyridyl)methyl]sulfinyl]-5,5,7,7-tetramethyl indeno-[5,6-d]imidazol-6(1H)-on, rührt sie 10 Minuten bei 40° und dampft das Lösunsmittel mittels einer Wasserstrahlpumpe teilweise ab. Nach Zugabe von t-Butyl-methyläther wird das Reaktionsprodukt kristallisiert, filtriert und mit t-Butyl-methyläther gewaschen. Das erhaltene 2-[[(o-Chlorphenyl)dithio]methyl] -4-methoxy-3,5-dimethyl-1-(1,5,6,7--tetrahydro -5,5,7,7-tetramethyl-6-oxoindeno[5,6-d]imidazol-2-yl)pyridiniumchlorid zeigt einen Schmelzpunkt von

 

170-176° (Zersetzung).

## Beispiel 103

2,4 g n-Hexylmercaptan werden in 10 ml einer 3,5N Lösung von gasförmigem Chlorwasserstoff in Tetrahydrofuran und 20 ml Tetrahydrofuran gelöst. Man versetzt die Lösung mit 8,5 g 5,7-Dihydro-2-[[(4-methoxy -3,5-dimethyl-2-pyridyl)methyl]sulfinyl]-5,5,7,7-tetramethyl indeno[5,6-d]imidazol-6(1H)-on; dabei kristalisiert ein Festkörper, welcher durch Rühren bei 40-50° wieder vollständig in Lösung gebracht wird. Anschliessend wird das Lösungsmittel mittels Wasserstrahlpumpe teilweise abgedampft und durch t-Butyl-methyläther ersetzt. Das Reaktionsprodukt wird kristallisiert, abfiltriert und mit t-Butyl-methyläther gewaschen. Das erhaltene 2-[(Hexyldithio)methyl] -4-methoxy-3,5-dimethyl-1-(1,5,6,7-tetrahydro -5,5,7,7-tetramethyl-6-oxo-indeno[5,6-d]imidazol -2-yl)pyridiniumchlorid zeigt einen Schmelzpunkt von 123-125° (Zersetzung).

## Beispiel 104

750 mg Thioäpfelsäure werden in 5 ml einer 3,5N Lösung von gasförmigem Chlorwasserstoff in Tetrahydrofuran gelöst. Man versetzt die Lösung mit 2,1 g 5,7-Dihydro-2-[[(4-methoxy -3,5-dimethyl-2-pyridyl)methyl]sulfinyl]-5,5,7,7-tetramethyl indeno[5,6-d]imidazol-6(1H)-on, rührt sie eine halbe Stunde bei 40° und dampft das Lösungsmittel mittels einer Wasserstrahlpumpe teilweise ab. Anschliessend wird die Lösung mit Essigester versetzt, wobei das Reaktionsprodukt kristallisiert, abfiltriert und mit Essigester gewaschen wird. Das erhaltene 2-[[(1,2-Dicarboxyäthyl)dithio]methyl] -4-methoxy-3,5-dimethyl-1-(1,5,6,7-tetrahydro -5,5,7,7-tetramethyl-6-oxoindeno[5,6-d]imidazol -2-yl)pyridiniumchlorid zeigt einen Schmelzpunkt von 187-189° (Zersetzung).

## Beispiel 105

434 mg m-Chlorthiophenol werden in 3 ml einer 4N Lösung von gasförmigem Chlorwasserstoff in Tetrahydrofuran und 10 ml Tetrahydrofuran gelöst. Man versetzt die Lösung mit 1,28 g 5,7-Dihydro-2-[[(4-methoxy -3,5-dimethyl-2-pyridyl)-methyl]sulfinyl]-5,5,7,7-tetramethyl indeno[5,6-d]imidazol-6(1H)-on, rührt sie 10 Minuten bei 40° und dampft das Lösungsmittel mittels einer Wasserstrahlpumpe teilweise ab. Nach Zugabe von t-Butyl-methyläther wird das Reaktionsprodukt kristallisiert, abfiltriert und mit t-Butyl-methyläther gewaschen. Das erhaltene 2-[[(m-Chlorphenyl)dithio]-methyl] -4-methoxy-3,5-dimethyl-1-(1,5,6,7-tetrahydro -5,5,7,7-tetramethyl-6-oxoindeno[5,6-d]imidazol -2-yl)pyridiniumchlorid zeigt einen Schmelzpunkt von 155-160° (Zersetzung).

## Beispiel 106

3,3 ml n-Hexanthiol werden in 22 ml einer 3,5N Lösung von gasförmigem Chlorwasserstoff in Tetrahydrofuran und 50 ml Tetrahydrofuran gelöst. Man versetzt die Lösung mit 9 g 5,6,7,8-Tetrahydro-2-[[(4-methoxy -3-methyl-2-pyridyl)-methyl]sulfinyl] -5,5,8,8-tetramethyl-1H-naphth[2,3-d]imidazol, rührt sie etwa 15 Minuten bei Raumtemperatur und dampft das Lösungsmittel mittels einer Wasserstrahlpumpe ab. Der Rückstand wird mit Aether kristallisiert, abfiltriert und mit Aether gewaschen. Das erhaltene 2-[(Hexyldithio)methyl]-4-methoxy -3-methyl-1-(5,6,7,8-tetrahydro -5,5,8,8-tetramethyl-1H-naphtho[2,3-d]imidazol -2-yl)pyridiniumchlorid zeigt einen Schmelzpunkt von 153° (Zersetzung).

## Beispiel 107

145 mg 2-Chlorthiophenol werden in 2 ml einer 3,5N Lösung von gasförmigem Chlorwasserstoff in Tetrahydrofuran

und 3 ml Tetrahydrofuran gelöst. Man versetzt die Lösung mit 412 mg 5,6,7,8-Tetrahydro-2-[[(4-methoxy -3-methyl--2-pyridyl)methyl]sulfinyl] -5,5,8,8-tetramethyl--1H-naphth[2,3-d]imidazol, rührt sie kurz bei Raumtemperatur und dampft das Lösungsmittel mittels einer Wasserstrahlpumpe ab. Der Rückstand wird mit t-Butyl-methyläther kristallisiert, abfiltriert und mit t-Butyl-methyläther gewaschen. Das erhaltene 2-[[(o-Chlorphenyl)dithio]methyl] -4-methoxy-3-methyl-1-(5,6,7,8-tetrahydro -5,5,8,8-tetramethyl-1H-naphtho[2,3-d]imidazol -2-yl)pyridiniumchlorid zeigt einen Schmelzpunkt von 175° (Zersetzung).

## Beispiel 108

750 mg Thioäpfelsäure werden in 5 ml einer 3,5N Lösung von gasförmigem Chlorwasserstoff in Tetrahydrofuran gelöst. Man versetzt die Lösung mit 2,1 g 5,7-Dihydro -2-[[(4--methoxy-3,5-dimethyl-2-pyridyl)methyl]sulfinyl] -5,5,7,7--tetramethylindeno[5,6-d]imidazol-6(1H)-on, rührt sie bei 40° und dampft das Lösungsmittel mittels einer Wasserstrahlpumpe teilweise ab. Nach Zugabe von Essigester kristallisiert das Reaktionsprodukt, welches abfiltriert und mit Essigester gewaschen wird. Das erhaltene 2-[[(1,2--Dicarboxyäthyl)dithio]methyl]-4-methoxy -3,5-dimethyl--1-(1,5,6,7-tetrahydro -5,5,7,7-tetramethyl-6-oxoindeno-[5,6-d]imidazol -2-yl)pyridiniumchlorid zeigt einen Schmelzpunkt von 189° (Zersetzung).

## Beispiel 109

a) Eine Lösung von 5 g 5-Methoxy-2-[[(4-methoxy -3-methyl-2-pyridyl)methyl]thio]benzimidazol in 80 ml abs. Methylenchlorid wird unter Argon bei -30° innerhalb von 10 Minuten mit einer Lösung von 3 g m-Chlorperbenzoesäure in 30 ml abs. Methylenchlorid versetzt. Anschliessend wird die Lösung noch zusätzlich 10 Minuten gerührt, mit einer 10%-igen Natriumcarbonatlösung extrahiert, getrocknet und

eingedampft. Der Rückstand wird in Essigester gelöst und mit Aether kristallisiert. Das erhaltene 5-Methoxy--2-[[(4-methoxy-3-methyl -2-pyridyl)methyl]sulfinyl]benzimidazol zeigt einen Schmelzpunkt von 120° (Zersetzung).

b) Eine Lösung von 713 mg n-Hexylmercaptan und 2 g 5-Methoxy-2-[[(4-methoxy -3-methyl-2-pyridyl)methyl]sulfinyl]benzimidazol in 6 ml 1N wässeriger Salzsäure wird eingedampft, worauf der Rückstand in Methanol gelöst und die Lösung noch einmal eingeengt wird. Das Reaktionsprodukt wird aus Methanol/Essigester kristallisiert. Das erhaltene 2-[(Hexyldithio)methyl] -4-methoxy-1-(5-methoxy-2-benzimidazolyl) -3-methylpyridiniumchlorid zeigt einen Schmelzpunkt von 135° (Zersetzung).

## Beispiel 110

677 mg 2-Mercaptopyrimidin werden in 60 ml abs. Methanol und 12 ml etwa 6N methanolischer Salzsäure gelöst. Man versetzt mit 2 g 5-Methoxy-2-[[(4-methoxy -3-methyl--2-pyridyl)methyl]sulfinyl]benzimidazol, rührt die Lösung bei Raumtemperatur, engt dann mittels einer Wasserstrahlpumpe teilweise ein und gibt so viel Essigester zu, bis ein Niederschlag auszukristallisieren beginnt. Man rührt das Reaktionsgemisch bis zur beendeten Kristallisation weiter. Das erhaltene 4-Methoxy-1-(5-methoxy -2-benzimidazolyl)--3-methyl-2-[(2-pyrimidinyldithio) methyl]pyridiniumchlorid-hydrochlorid wird abfiltriert und mit kaltem Essigester gewaschen; es zeigt einen Schmelzpunkt von 144° (Zersetzung).

## Beispiel 111

930 mg Thiosalicylsäure werden in 6 ml einer 3,5N Lösung von gasförmigem Chlorwasserstoff in Tetrahydrofuran und 60 ml Tetrahydrofuran gelöst. Man versetzt die Lösung mit 2 g 5-Methoxy-2-[[(4-methoxy-3-methyl -2-pyridyl)-methyl]sulfinyl]benzimidazol. Das Reaktionsgemisch wird bei 40-50° gerührt und mit Wasser versetzt, bis der ausgefallene Niederschlag ganz in Lösung geht. Anschliessend wird das Lösungsmittel teilweise abgedampft und kontinuierlich durch Essigester ersetzt. Das kristalline Reaktionsprodukt wird abfiltriert und mit Essigester gewaschen. Das erhaltene 2-[[(o-Carboxyphenyl)dithio]methyl]-4-methoxy-1-(5-methoxy-2-benzimidazolyl)-3-methylpyridiniumchlorid zeigt einen Schmelzpunkt von 163° (Zersetzung).

## Beispiel 112

770 mg Thiosalicylsäure werden in 5 ml einer 3N Lösung von gasförmigem Chlorwasserstoff in Tetrahydrofuran und 15 ml Tetrahydrofuran gelöst und mit 1,7 g 6-[[(4-Methoxy-3-methyl -2-pyridyl)methyl]sulfinyl] -5H-1,3-dioxolo[4,5-f]benzimidazol versetzt. Anschliessend gibt man 5 ml 1N wässerige Salzsäure zu und kocht das Reaktionsgemisch kurz auf, wobei eine klare Lösung entsteht. Der Grossteil des Lösungsmittelgemisches wird abgedampft; das Reaktionsprodukt wird durch Zugabe von Essigester auskristallisiert, abfiltriert und mit Essigester gewaschen. Das erhaltene 2-[[(o-Carboxyphenyl)dithio]methyl]-1-(5H -1,3-dioxolo[4,5-f]benzimidazol-6-yl) -4-methoxy-3-methylpyridiniumchlorid zeigt einen Schmelzpunkt von 208° (Zersetzung).

## Beispiel 113

1 ml Aethylmercaptan wird in 25 ml Methanol und 10 ml etwa 3N methanolischer Salzsäure gelöst. Man versetzt mit 2 g 6-[[(4-Methoxy-3-methyl -2-pyridyl)methyl]sulfinyl]-5H-1,3-dioxolo[4,5-f] benzimidazol, rührt die Lösung 15 Minuten bei Rückflusstemperatur und dampft dann das Lösungsmittel mittels einer Wasserstrahlpumpe ab, wobei das Methanol kontinuierlich durch Essigester ersetzt wird. Das Reaktionsgemisch wird bis zur beendeten Kristallisation weitergerührt. Das erhaltene 1-[5H-1,3-Dioxolo[4,5-f]benzimidazol -2-yl]-2-[(äthyldithio)methyl] -4-methoxy--3-methylpyridiniumchlorid-hydrochlorid wird abfiltiert und mit kaltem Essigester gewaschen; es zeigt einen Schmelzpunkt von 158-160° (Zersetzung).

## Beispiel 114

904 mg Thioäpfelsäure werden in 6 ml einer 3,5N Lösung von gasförmigem Chlorwasserstoff in Tetrahydrofuran gelöst. Man versetzt die Lösung mit 2 g 5-Methoxy-2-[[(4-methoxy-3--methyl-2 -pyridyl)methyl]sulfinyl]benzimidazol und rührt die Suspension während 90 Minuten bei 50°. Der ausgefallene schwarze Schlamm wird abfiltriert. Das Filtrat wird am Wasserstrahlvakuum teilweise vom Lösungsmittel befreit, worauf man Essigester zugibt. Das Reaktionsprodukt wird kristallisiert, abfiltriert und mit wenig Essigester gewaschen. Das erhaltene 2-[[(1,2-Dicarboxyäthyl)dithio]methyl]--4-methoxy-1 -(5-methoxy-2-benzimidazolyl)-3-methyl-pyridiniumchlorid zeigt einen Schmelzpunkt von 170° (Zers.).

## Beispiel 115

750 mg 2-Chlorthiophenol werden in 5 ml etwa 3N methanolischer Salzsäure und 5 ml Methanol gelöst. Man versetzt die Lösung mit 1,5 g 5-Methoxy-2-[[(4-methoxy-3-methyl -2-pyridyl)methyl]sulfinyl]benzimidazol und rührt sie bei 50° für eine halbe Stunde. Das Lösungsmittel wird am Wasserstrahlvakuum teilweise entfernt und durch Essigester ersetzt. Das Reaktionsprodukt wird kristallisiert, abfiltriert und mit wenig Essigester gewaschen. Das erhaltene 2-[[(o-Chlorphenyl)dithio]methyl]-4-methoxy-1-(5-methoxy -2-benzimidazolyl)-3-methyl-pyridiniumchlorid wird abfiltriert und mit kaltem Essigester gewaschen; es zeigt einen Schmelzpunkt von 138° (Zers.).

Beispiel 116

112 mg 2-Mercaptopyrimidin werden in 1 ml 3N methanolischer Salzsäure und 15 ml Methanol gelöst. Man versetzt mit 425 mg 5,7-Dihydro-2-[[(4-methoxy-3,5-dimethyl -2-pyridyl)-methyl]sulfinyl]-5,5,7,7-tetramethyl -indeno[5,6-d]imidazol--6(1H)-on, rührt die Lösung während 5 Minuten bei 40° und dampft dann das Methanol bis auf wenige Milliliter mittels einer Wasserstrahlpumpe ab. Man gibt etwa 10 ml t-Butylmethyläther zu und kristallisiert das Reaktionsprodukt. Das erhaltene 4-Methoxy-3,5-dimethyl-2-[(2-pyrimidinyldithio)-methyl]-1 -(1,5,6,7-tetrahydro-5,5,7,7-tetramethyl -6-oxo-indeno[5,6-d]imidazol-2-yl)pyridiniumchlorid wird abfiltriert und mit kaltem t-Butylmethyläther gewaschen; es zeigt einen Schmelzpunkt von 170° (Zers.).

Beispiel 117

0,5 ml Aethylmercaptan werden in 4 ml einer 3N Lösung von gasförmigem Chlorwasserstoff in Tetrahydrofuran und 5 ml Tetrahydrofuran gelöst. Man versetzt die Lösung mit 1,2 g 5,7-Dihydro-2-[[(4-methoxy-3,5-dimethyl-2 -pyridyl)methyl]-sulfinyl]-5,5,7,7-tetramethyl -indeno[5,6-d]imidazol-6(1H)--on und rührt sie während 10 Minuten bei 40°. Das Tetrahydrofuran wird am Wasserstrahlvakuum teilweise entfernt; bei Zugabe von Essigester/Aether tritt Kristallisation ein. Das erhaltene 2-[(Aethyldithio)methyl]-4-methoxy-3,5-dimethyl-1--(1,5,6,7 -tetrahydro-5,5,7,7-tetramethyl-6-oxoindeno-[5,6-d]imidazol -2-yl)pyridiniumchlorid wird abfiltriert und mit Aether gewaschen; es zeigt einen Schmelzpunkt von 195° (Zers.).

Beispiel 118

760 mg 2-Bromthiophenol werden in 10 ml 3N methanolischer Salzsäure und 10 ml Methanol gelöst. Man versetzt die Lösung mit 1,6 g 5,7-Dihydro-2-[[(4-methoxy-3-methyl-2-pyridyl)methyl]sulfinyl] -5,5,7,7-tetramethylindeno[5,6-d]imidazol-6(1H)-on und rührt sie während 5 Minuten bei 40°. Das Methanol wird am Wasserstrahlvakuum teilweise entfernt und durch Essigester ersetzt. Das erhaltene, kristalline 2-[[(o-Bromphenyl)dithio]methyl]-4-methoxy-3-methyl-1 -(1,5,6,7-tetrahydro-5,5,7,7-tetramethyl -6-oxoindeno[5,6-d]imidazol-2-yl)pyridiniumchlorid wird abfiltriert und mit kaltem Essigester gewaschen; es zeigt einen Schmelzpunkt von 175-178° (Zers.).

## Beispiel 119

720 mg 2,3-Dichlorthiophenol werden in 8 ml 3N methanolischer Salzsäure und 12 ml Methanol gelöst. Man versetzt die Lösung mit 1,64 g 5,7-Dihydro-2-[[(4-methoxy-3-methyl-2-pyridyl)methyl]sulfinyl] -5,5,7,7-tetramethylindeno[5,6-d]imidazol-6(1H)-on und rührt sie während 5 Minuten bei Raumtemperatur. Das Methanol wird am Wasserstrahlvakuum teilweise entfernt und durch Essigester ersetzt. Das erhaltene, kristalline 2-[[(2,3-Dichlorphenyl)dithio]methyl]-4-methoxy-3-methyl -1-(1,5,6,7-tetrahydro-5,5,7,7-tetramethyl-6 -oxoindeno[5,6-d]imidazol-2-yl)pyridiniumchlorid wird abfiltriert und mit kaltem Essigester gewaschen; es zeigt einen Schmelzpunkt von 180° (Zers.).

## Beispiel 120

630 mg m-Fluorthiophenol werden in 10 ml 3N methanolischer Salzsäure und 20 ml Methanol gelöst. Man versetzt die Lösung mit 2 g 5,7-Dihydro-2-[[(4-methoxy-3-methyl-2-pyridyl)methyl]sulfinyl] -5,5,7,7-tetramethylindeno[5,6-d]imidazol-6(1H)-on und rührt sie während 5 Minuten bei Raumtemperatur. Das Methanol wird am Wasserstrahlvakuum teilweise entfernt und durch Essigester ersetzt. Das erhaltene, kristalline 2-[[(m-Fluorphenyl)dithio]methyl]-4-methoxy-3-methyl-1 -(1,5,6,7-tetrahydro-5,5,7,7-tetramethyl-6 -oxoindeno[5,6-d]imidazol-2-yl)pyridiniumchlorid wird abfiltriert und mit kaltem Essigester gewaschen; es zeigt einen Schmelzpunkt von 173° (Zers.).

Beispiel 121

720 mg 2,4-Dichlorthiophenol werden in 8 ml 3N methanolischer Salzsäure und 20 ml Methanol gelöst. Man versetzt die Lösung mit 1,65 g 5,7-Dihydro-2-[[(4-methoxy-3-methyl-2--pyridyl)methyl]sulfinyl] -5,5,7,7-tetramethylindeno[5,6-d]-imidazol-6(1H)-on und rührt sie während 5 Minuten bei 50°. Das Methanol wird am Wasserstrahlvakuum teilweise entfernt und durch Essigester ersetzt; das Reaktionsprodukt beginnt schon aus Methanol zu kristallisieren. Das erhaltene 2-[[(2,4-Dichlorphenyl)dithio]methyl]-4-methoxy-3-methyl-1 -(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 197° (Zers.).

Beispiel 122

195 mg m-Bromthiophenol werden in 1 ml 3N methanolischer Salzsäure und 5 ml Methanol gelöst. Man versetzt die Lösung mit 411 mg 5,7-Dihydro-2-[[(4-methoxy-3-methyl-2-pyridyl)-methyl]sulfinyl] -5,5,7,7-tetramethylindeno[5,6-d]imidazol--6(1H)-on und rührt sie während 5 Minuten bei Raumtemeratur. Das Methanol wird am Wasserstrahlvakuum teilweise entfernt und durch Essigester ersetzt. Das erhaltene, kristalline 2-[[(m-Bromphenyl)dithio]methyl]-4-methoxy-3-methyl-1 -(1,5,6,7-tetrahydro-5,5,7,7-tetramethyl -6-oxoindeno-[5,6-d]imidazol-2-yl)pyridiniumchlorid wird abfiltriert und mit kaltem Essigester gewaschen; es zeigt einen Schmelzpunkt von 160° (Zers.).

Beispiel 123

750 mg 3-Chlorthiophenol werden in 5 ml 3N methanolischer Salzsäure und 20 ml Methanol gelöst. Man versetzt die Lösung mit 1,725 g 6-[[(4-Methoxy-3-methyl-2-pyridyl)-methyl]sulfinyl]-5H -1,3-dioxolo[4,5-f]benzimidazol und erhitzt sie so lange auf dem Wasserbad, bis eine klare Lösung entsteht. Anschliessend wird die Lösung während 30 Minuten bei 45° gerührt, mit Essigester versetzt und teilweise eingeengt. Das erhaltene, kristalline 2-[[(m--Chlorphenyl)dithio]methyl]-1-(5H-1,3 -dioxolo[4,5-f]benzimidazol-6-yl)-4-methoxy -3-methylpyridiniumchlorid wird abfiltriert und mit kaltem Essigester gewaschen; es zeigt einen Schmelzpunkt von 191° (Zers.).

## Beispiel 124

1 ml n-Propylmercaptan werden in 5 ml 3N methanolischer Salzsäure und 20 ml Methanol gelöst. Man versetzt die Lösung mit 1,7 g 6-[[(4-Methoxy-3-methyl-2-pyridyl)methyl]sulfinyl]-5H -1,3-dioxolo[4,5-f]benzimidazol und erhitzt unter Rückfluss während 15 Minuten, bis alles gelöst ist. Anschliessend wird die Reaktionsmischung während 20 Minuten bei 40-45° gerührt, worauf das Lösungsmittel teilweise abgedampft und durch Essigester ersetzt wird. Das erhaltene, kristalline 1-(5H-1,3-Dioxolo[4,5-f]benzimidazol-6-yl)-4--methoxy -3-methyl-2-[(propyldithio)methyl]pyridiniumchlorid wird abfiltriert und mit kaltem Essigester gewaschen; es zeigt einen Schmelzpunkt von 165° (Zers.).

## Beispiel 125

a) 14,8 g (76,7 mMol) 5H-1,3-Dioxolo[4,5-f]benzimidazol-6--thiol werden in 300 ml Alkohol suspendiert und unter Eiskühlung mit 17,0 g (76,5 mMol) 2-Chlormethyl-4-methoxy-3,5--dimethylpyridin-hydrochlorid versetzt. Hernach tropft man eine Lösung von 6,0 g Natriumhydroxyd in 150 ml Wasser zu, lässt das Gemisch über Nacht am Rückfluss kochen und dampft es anschliessend im Vakuum zur Trockne ein. Der Rückstand wird in 1000 ml Methylenchlorid gelöst. Die Lösung wird

zuerst mit 500 ml 1,5N Natronlauge und dann mit 3 x 500 ml Wasser gewaschen, über Natriumsulfat getrocknet und im Vakuum eingedampft. Das Rohprodukt reinigt man an 300 g Kieselgel mit Essigester/Methylenchlorid (1:1) als Elujerungsmittel. Kristallisation aus Methylenchlorid/Petroläther ergibt 6-[[(4-Methoxy-3,5-dimethyl-2-pyridyl)methyl]thio]-5H -1,3-dioxolo[4,5-f]benzimidazol vom Schmelzpunkt 178-179°.

b) 13,3 g 6-[[(4-Methoxy-3,5-dimethyl-2-pyridyl)methyl]-thio]-5H -1,3-dioxolo[4,5-f]benzimidazol werden in 300 ml Methylenchlorid gelöst und mit einem Eis/Methanol-Bad auf -10° gekühlt. Dann werden innerhalb von 30 Minuten 7,5 g aus Methylenchlorid/Petroläther umkristallisierte m-Chlorperbenzoesäure eingetragen. Man rührt die Lösung bei -10° noch 120 Minuten weiter und giesst sie dann in ein Gemisch von 300 ml 2N Natriumcarbonatlösung und Eis. Die wässrige Phase wird zweimal mit 300 ml Methylenchlorid extrahiert. Die vereinigten organischen Phasen werden dreimal mit 250 ml Wasser neutralgewaschen, über Natriumsulfat getrocknet und im Vakuum bei 35° auf ein Volumen von 150 ml eingeengt. Bei Zugabe von Petroläther erfolgt Kristallisation von 6-[[(4--Methoxy-3,5-dimethyl-2-pyridyl)methyl]sulfinyl]-5H -1,3--dioxolo[4,5-f]benzimidazol; Smp. 192-194°.

c) 0,5 ml n-Propylmercaptan werden in 4 ml 3N methanolischer Salzsäure und 20 ml Methanol gelöst. Man versetzt die Lösung mit 1,8 g 6-[[(4-Methoxy-3,5-dimethyl-2-pyridyl)-methyl]sulfinyl]-5H -1,3-dioxolo[4,5-f]benzimidazol und rührt sie während 20 Minuten bei 45°. Anschliessend wird das Lösungsmittel am Wasserstrahlvakuum teilweise entfernt und durch Essigester ersetzt. Das erhaltene, kristalline 1-(5H--1,3-Dioxolo[4,5-f]benzimidazol-6-yl)-4-methoxy -3,5-dimethyl-2-[(propyldithio)methyl]pyridiniumchlorid wird abfiltriert und mit kaltem Essigester gewaschen; es zeigt einen Schmelzpunkt von 111-120° (Zers.).

## Beispiel 126

906 mg 3-Chlorthiophenol werden in 6 ml 3N methanolischer Salzsäure und 20 ml Methanol gelöst. Man versetzt die Lösung mit 2 g 5-Methoxy-2-[[(4-methoxy-3-methyl-2 -pyridyl)methyl]sulfinyl]benzimidazol und rührt sie während 2 Stunden bei 40°. Anschliessend wird das Lösungsmittel am Wasserstrahlvakuum teilweise entfernt und durch Essigester ersetzt. Das erhaltene, kristalline 2-[[(m-Chlorphenyl)dithio]methyl]-4-methoxy-3-methyl-1 -(5-methoxy-2-benzimidazolyl)pyridiniumchlorid wird abfiltriert und mit kaltem Essigester gewaschen; es zeigt einen Schmelzpunkt von 137° (Zers.).

## Beispiel 127

508 mg n-Propylmercaptan werden in 6 ml 3N methanolischer Salzsäure und 20 ml Methanol gelöst. Man versetzt die Lösung mit 2 g 5-Methoxy-2-[[(4-methoxy-3-methyl-2 -pyridyl)methyl]sulfinyl]benzimidazol und rührt sie während 10 Minuten bei 40°. Anschliessend wird das Lösungsmittel am Wasserstrahlvakuum teilweise entfernt und durch Essigester ersetzt. Das erhaltene, kristalline 4-Methoxy-1-(5-methoxy--2-benzimidazolyl)-3-methyl -2-[(propyldithio)methyl]pyridiniumchlorid wird abfiltriert und mit kaltem Essigester gewaschen; es zeigt einen Schmelzpunkt von 144° (Zers.).

## Beispiel 128

408 mg n-Propylmercaptan werden in 5 ml einer 3,5N Lösung von gasförmigem Chlorwasserstoff in Tetrahydrofuran und 20 ml Tetrahydrofuran gelöst. Man versetzt die Lösung mit 2 g 5,6,7,8-Tetrahydro-2-[[(4-methoxy-3-methyl -2-pyridyl)methyl]sulfinyl]-5,5,8,8-tetramethyl -1H-naphth[2,3-d]-imidazol und rührt sie während 5 Minuten bei Raumtemperatur. Anschliessend wird das Tetrahydrofuran am Wasserstrahlvakuum teilweise entfernt und durch Essigester ersetzt. Das erhaltene, kristalline 4-Methoxy-3-methyl-2-[(propyldithio)-methyl]-1-(5,6,7,8 -tetrahydro-5,5,8,8-tetramethyl-1H--naphth[2,3-d]imidazol -2-yl)pyridiniumchlorid wird abfiltriert und mit kaltem Essigester gewaschen; es zeigt einen Schmelzpunkt von 163° (Zers.).

Beispiel 129

1 ml Aethylmercaptan wird in 10 ml 3N methanolischer Salzsäure und 50 ml Methanol gelöst. Man versetzt die Lösung mit 2 g 5,6,7,8-Tetrahydro-2-[[(4-methoxy-3-methyl -2-pyridyl)methyl]sulfinyl]-5,5,8,8-tetramethyl -1H-naphth[2,3-d]-imidazol und rührt sie während 10 Minuten bei Raumtemperatur. Anschliessend wird das Lösungsmittel am Wasserstrahlvakuum teilweise entfernt und durch Aether ersetzt. Das erhaltene, kristalline 2-[(Aethyldithio)methyl]-4-methoxy-3--methyl-1 -(5,6,7,8-tetrahydro-5,5,8,8-tetramethyl-1H -naphth[2,3-d]imidazol-2-yl)pyridiniumchlorid wird abfiltriert und mit kaltem Aether gewaschen; es zeigt einen Schmelzpunkt von 160° (Zers.).

Beispiel 130

1,84 ml Thiosalicylsäure werden in 100 ml 20%iger alkoholischer Salzsäure gerührt und unter Eiskühlung mit 2,6 g 2-[(2-Pyridylmethyl)sulfinyl]benzimidazol versetzt. Das Gemisch wird über Nacht bei Raumtemperatur weitergerührt und dann abgenutscht. Die Kristalle werden mit Aether gewaschen und im Vakuum bei 40° getrocknet. Das Rohprodukt kristallisiert man aus Alkohol/abs. Aether um. Man erhält 1-(2-Benzimidazolyl)-2 -[[(o-carboxyphenyl)dithio]methyl]pyridiniumchlorid vom Schmelzpunkt 142-145°.

Beispiel 131

2,6 g 2-[(2-Pyridylmethyl)sulfinyl]benzimidazol werden in 50 ml Dioxan und 50 ml 3N Salzsäure vorgelegt und mit

2,02 g Dodecanthiol versetzt. Nach 30 Minuten Rühren bei Raumtemperatur wird das Gemisch im Vakuum eingedampft. Der Rückstand wird in Alkohol gelöst und nach Zugabe von Toluol erneut eingedampft, dann mit Aether zum Kristallisieren gebracht, abgenutscht und mit Aether gewaschen. Man erhält 1-(2-Benzimidazolyl)-2 -[(dodecyldithio)methyl]pyridiniumchlorid vom Schmelzpunkt 117-120°.

## Beispiel 132

1,18 g 1-Hexanthiol werden in 30 ml Methanol und 70 ml 3N Salzsäure vorgelegt und unter Eiskühlung zwischen 5-10° mit 2,6 g 2-[(2-Pyridylmethyl)sulfinyl]benzimidazol versetzt. Das Gemisch wird 3 Stunden bei 5-10°, dann nach Zugabe von 30 ml 3N Salzsäure noch 1,5 Stunden bei Raumtemperatur gerührt und anschliessend im Hochvakuum bei 20° eingedampft. Der Rückstand ergibt nach der Kristallisation aus Essigester/abs. Aether 1-(2-Benzimidazolyl)-2-[(hexyldithio)methyl]pyridiniumchlorid vom Schmelzpunkt 125-127°.

## Beispiel 133

1,0 g 2-Mercaptoäthanol werden in 30 ml Methanol und 70 ml 3N Salzsäure vorgelegt und unter Eiskühlung zwischen 5-10° mit 2,6 g 2-[(2-Pyridylmethyl)sulfinyl]benzimidazol versetzt. Das Gemisch wird 3 Stunden bei 5-10°, dann nach Zugabe von 30 ml 3N Salzsäure noch 1,5 Stunden bei Raumtemperatur gerührt und anschliessend im Hochvakuum bei 20° eingedampft. Der Rückstand ergibt nach der Kristallisation aus Methanol/abs. Aether 1-(2-Benzimidazolyl)-2-[[(2 -hydroxyäthyl)dithio]methyl]pyridiniumchlorid vom Schmelzpunkt 130-132°.

## Beispiel 134

0,7 ml tert.Butylmercaptan werden in 30 ml Methanol und 70 ml 3N Salzsäure vorgelegt und unter Eiskühlung zwischen

5-10° mit 2,6 g 2-[(2-Pyridylmethyl)sulfinyl]benzimidazol versetzt. Das Gemisch wird 3 Stunden bei 5-10°, dann nach Zugabe von 30 ml 3N Salzsäure noch 1,5 Stunden bei Raumtemperatur gerührt und anschliessend im Hochvakuum bei 20° eingedampft. Der Rückstand ergibt nach der Kristallisation aus Methanol/abs. Aether 1-(2-Benzimidazolyl)-2 -[(tert--butyldithio)methyl]pyridiniumchlorid vom Schmelzpunkt 192-194°.

## Beispiel 135

2,6 g 2-[(2-Pyridylmethyl)sulfinyl]benzimidazol werden in 250 ml 20%iger alkoholischer Salzsäure vorgelegt. Dazu werden 1,24 g p-Thiokresol in 50 ml Alkohol getropft. Das Gemisch wird 2 Stunden bei Raumtemperatur gerührt und dann abgenutscht. Man dampft das Filtrat im Vakuum ein und kristallisiert den Rückstand aus Alkohol/Aether. Man erhält 1-(2-Benzimidazolyl)-2 -[(p-tolyldithio)methyl]pyridiniumchlorid vom Schmelzpunkt 124-126°.

## Beispiel 136

2,6 g 2-[(2-Pyridylmethyl)sulfinyl]benzimidazol werden in 30 ml Tetrahydrofuran gelöst und mit 1,0 ml n-Propylmercaptan versetzt. Bei 40° Innentemperatur werden 10 ml 3N Salzsäure zugegeben. Das Gemisch wird 5 Minuten bei 40° gerührt und dann im Vakuum eingedampft. Der Rückstand wird aus Aethanol/Aether kristallisiert. Man erhält 1-(2-Benzimidazolyl) -2-[(propyldithio)methyl]pyridiniumchlorid vom Schmelzpunkt 144-145°.

## Beispiel 137

2,6 g 2-[(2-Pyridylmethyl)sulfinyl]benzimidazol werden in 30 ml Tetrahydrofuran gelöst und mit 1,5 g Thioäpfelsäure versetzt. Bei 40° Innentemperatur werden 10 ml 3N Salzsäure zugegeben. Nach 5 Minuten Rühren bei 40° wird das Gemisch im

Vakuum eingedampft. Der Rückstand wird aus Methanol/tert.-Butylmethyläther/Essigester kristallisiert. Man erhält 1-(2-Benzimidazolyl)-2-[[(1,2 -dicarboxyäthyl)dithio]-methyl]pyridiniumchlorid vom Schmelzpunkt 113-115°.

## Beispiel 138

2,6 2-[(2-Pyridylmethyl)sulfinyl]benzimidazol werden in 50 ml Acetonitril gelöst und mit 1,0 ml Aethylmercaptan versetzt. Bei 40° Innentemperatur werden 50 ml 3N Salzsäure zugegeben. Das Gemisch wird über Nacht bei Raumtemperatur gerührt und dann im Vakuum eingedampft. Der Rückstand wird aus Methanol/abs. Aether kristallisiert, und man erhält 1-(2-Benzimidazolyl)-2-[(äthyldithio)methyl]pyridiniumchlorid vom Schmelzpunkt 113-115°.

## Beispiel 139

a) 64,8 g 2-Benzimidazolthiol werden in 400 ml Alkohol suspendiert und unter Eiskühlung mit 95,9 g 2-Chlormethyl-4--methoxy-3,5-dimethylpyridin-hydrochlorid versetzt. Hernach tropft man eine Lösung von 34,5 g Natriumhydroxyd in 1,5 l Wasser zu, lässt das Gemisch über Nacht am Rückfluss kochen und dampft es anschliessend im Vakuum zur Trockene ein. Der Rückstand wird in 2,0 l Methylenchlorid gelöst. Die Lösung wird zuerst mit 600 ml 1,5N Natronlauge, dann 3 x mit 2,2 l Wasser gewaschen, über Natriumsulfat getrocknet und im Vakuum eingedampft. Das Rohprodukt wird an 1300 g Kieselgel mit Essigester/Methylenchlorid (1:1) als Eluierungsmittel gereinigt. Kristallisation aus Methylenchlorid/Petroläther ergibt 2-[[(4-Methoxy-3,5-dimethyl-2-pyridyl)methyl]thio]-benzimidazol vom Schmelzpunkt 129-131°.

b) 53,3 g 2-[[(4-Methoxy-3,5-dimethyl-2-pyridyl)methyl]-thio]benzimidazol werden in 1,2 l Methylenchlorid und 100 ml Methanol gelöst und dann auf -20° abgekühlt. Innerhalb von 10 Minuten werden 33,4 g aus Methylenchlorid/Petroläther

umkristallisierte m-Chlorperbenzoesäure eingetragen. Man rührt die Lösung bei -20° noch 60 Minuten weiter und giesst sie dann in ein Gemisch von 250 ml 2N Natriumcarbonatlösung und Eis. Die organische Phase wird mit Wasser neutral gewaschen, über Natriumsulfat getrocknet und im Vakuum eingedampft. Kristallisation aus Methylenchlorid/Methanol/Petroläther ergibt 2-[[(4-Methoxy-3,5-dimethyl-2 -pyridyl)methyl]-sulfinyl]benzimidazol vom Schmelzpunkt 157-159°.

c) 3,15 g 2-[[(4-Methoxy-3,5-dimethyl-2 -pyridyl)methyl]-sulfinyl]benzimidazol werden bei 40° in 30 ml Tetrahydrofuran gelöst und mit 1 ml n-Propylmercaptan und mit 10 ml 3N Salzsäure versetzt und 5 Minuten gerührt. Anschliessend wird das Gemisch im Vakuum eingedampft. Der Rückstand wird aus Methanol/Aether kristallisiert, und man erhält 1-(2-Benzimidazolyl)-4-methoxy-3,5-dimethyl -2-[(propyldithio)methyl]-pyridiniumchlorid vom Schmelzpunkt 175-177°.

## Beispiel 140

2,6 g Thiosalicylsäure werden in 50 ml 20%iger alkoholischer Salzsäure unter Rühren mit 2,6 g 2-[[(4-Methoxy-3--methyl-2-pyridyl)methyl]sulfinyl] -5-(trifluormethyl)benzimidazol versetzt. Das Gemisch wird über Nacht bei Raumtemperatur gerührt, dann abgenutscht und mit abs. Aether gewaschen. Kristallisation aus Alkohol/abs. Aether ergibt 2-[[(o-Carboxyphenyl)dithio]methyl]-4-methoxy-3-methyl -1-[5-(trifluormethyl]-2-benzimidazolyl]pyridiniumchlorid vom Schmelzpunkt 180-182°.

## Beispiel 141

2,48 g p-Thiokresol werden in 10 ml Alkohol vorgelegt und mit 3,69 g 2-[[(4-Methoxy-3-methyl-2-pyridyl)methyl]sulfinyl] -5-(trifluormethyl)benzimidazol versetzt. Man tropft 50 ml 20%iger alkoholischer Salzsäure unter Eiskühlung und Rühren hinzu. Das Gemisch wird über Nacht bei Raumtemperatur

gerührt, dann abgenutscht und mit abs. Aether gewaschen. Kristallisation aus Alkohol/Aether ergibt 4-Methoxy-3--methyl-2-[(p-tolyldithio)methyl] -1-[5-(trifluormethyl)-2--benzimidazolyl]pyridiniumchlorid vom Schmelzpunkt 138-140°.

## Beispiel 142

3,0 g Thioäpfelsäure werden in 50 ml Alkohol vorgelegt und mit 3,69 g 2-[[(4-Methoxy-3-methyl-2-pyridyl)methyl]sulfinyl] -5-(trifluormethyl)benzimidazol versetzt. Man tropft 50 ml 20%iger alkoholischer Salzsäure unter Eiskühlung und Rühren hinzu. Das Gemisch wird über Nacht bei Raumtemperatur gerührt und dann im Hochvakuum bei 20° eingedampft. Kristallisation aus Alkohol/abs. Aether ergibt 2-[[[1,2-Bis--(äthoxycarbonyl)äthyl]dithio]methyl]-4-methoxy -3-methyl--1-[5-(trifluormethyl)-2 -benzimidazolyl]pyridiniumchlorid vom Schmelzpunkt 96-97°.

## Beispiel 143

2,2 g 2-[[(4-Methoxy-3-methyl-2-pyridyl)methyl]sulfinyl] -5-(trifluormethyl)benzimidazol werden in 30 ml Tetrahydrofuran gelöst und mit 0,6 ml n-Propylmercaptan versetzt. Bei 40° Innentemperatur gibt man 6 ml 3N Salzsäure dazu. Nach 5 Minuten Rühren wird das Gemisch im Vakuum eingedampft, in Alkohol gelöst und die Verunreinigungen mit Aether gefällt. Die Lösung wird filtriert und im Vakuum eingedampft. Den Rückstand kristallisiert man aus tert.Butylmethyläther/Aether und erhält 2-[(Propyldithio)methyl]-4-methoxy-3--methyl -1-[5-(trifluormethyl)-2-benzimidazolyl]pyridiniumchlorid vom Schmelzpunkt 152-153°.

## Beispiel 144

3,69 g 2-[[(4-Methoxy-3-methyl-2-pyridyl)methyl]sulfinyl] -5-(trifluormethyl)benzimidazol werden in 50 ml Acetonitril gelöst und mit 1,5 g Thioäpfelsäure versetzt.

Bei 40° Innentemperatur werden 50 ml 3N Salzsäure zugegeben, und das Gemisch wird über Nacht bei Raumtemperatur weitergerührt und anschliessend im Vakuum eingedampft. Der Rückstand wird aus abs. Aether kristallisiert, und man erhält 2-[[(1,2-Dicarboxyäthyl)dithio]methyl]-4-methoxy-3-methyl -1-[5-(trifluormethyl)-2-benzimidazolyl]pyridiniumchlorid vom Schmelzpunkt 110-115°.

## Beispiel 145

5,16 g 2-[[(4-Methoxy-3-methyl-2-pyridyl)methyl]sulfinyl] -5-(trifluormethyl)benzimidazol werden in 100 ml Methanol gelöst und mit 1,65 g Hexanthiol versetzt. Bei 40° Innentemperatur werden 85 ml 3N Salzsäure zugegeben. Man rührt das Gemisch 15 Minuten bei dieser Temperatur; dann dampft man es im Vakuum ein. Kristallisation aus tert.Butylmethyläther/Aether ergibt ein Produkt vom Schmelzpunkt 130-132°, welches aus Essigester/Aether/Petroläther (tiefs.) umkristallisiert wird und dann bei 116-118° schmilzt. Nochmalige Umkristallisation aus Alkohol/Aether ergibt 2-[(Hexyldithio)methyl]-4-methoxy-3-methyl -1-[5-(trifluormethyl)-2-benzimidazolyl]pyridiniumchlorid vom Schmelzpunkt 124-125°.

## Beispiel 146

3,69 g 2-[[(4-Methoxy-3-methyl-2-pyridyl)methyl]sulfinyl] -5-(trifluormethyl)benzimidazol werden in 60 ml Tetrahydrofuran gelöst und mit 1,44 g 2-Chlorthiophenol versetzt. Bei 40° Innentemperatur werden 12 ml 3N Salzsäure zugegeben. Das Gemisch wird 5 Minuten bei 40°, dann 1 Stunde bei Raumtemperatur gerührt und anschliessend im Vakuum eingedampft. Der Rückstand wird aus Methanol/Aether umkristallisiert, und man erhält 2-[[(o-Chlorphenyl)dithio]methyl]-4-methoxy-3-methyl -1-[5-(trifluormethyl)-2-benzimidazolyl]pyridiniumchlorid vom Schmelzpunkt 148-150°.

0214479

Beispiel 147

3,69 g 2-[[(4-Methoxy-3-methyl-2-pyridyl)methyl]sulfinyl] -5-(trifluormethyl)benzimidazol werden in 100 ml Acetonitril gelöst und mit 1,44 g 3-Chlorthiophenol versetzt. Bei 55° gibt man eine Lösung von 18 ml 3N Salzsäure und 18 ml Methanol dazu und rührt das Gemisch noch 10 Minuten weiter. Nach Eindampfen im Vakuum wird der Rückstand aus Methanol/Essigester/abs. Aether kristallisiert. Man erhält 2-[[(m-Chlorphenyl)dithio]methyl]-4-methoxy-3-methyl -1-[5-(trifluormethyl)-2-benzimidazolyl]pyridiniumchlorid vom Schmelzpunkt 76-80°.

Beispiel 148

3,69 g 2-[[(4-Methoxy-3-methyl-2-pyridyl)methyl]sulfinyl] -5-(trifluormethyl)benzimidazol werden in 100 ml Acetonitril gelöst und mit 1,12 g 2-Pyrimidinthiol versetzt. Bei 55° Innentemperatur gibt man eine Lösung von 18 ml 3N Salzsäure und 18 ml Methanol zu und rührt das Gemisch bei dieser Temperatur noch 10 Minuten weiter. Nach Eindampfen im Vakuum wird der Rückstand aus tert.Butyl-methyläther kristallisiert. Man erhält 4-Methoxy-3-methyl-1-[5-(trifluormethyl)-2-benzimidazolyl] -2-[(2-pyrimidinyldithio)methyl]pyridiniumchlorid vom Schmelzpunkt 95-100°.

Beispiel 149

3,69 g 2-[[(4-Methox-3-methyl-2-pyridyl)methyl]sulfinyl] -5-(trifluormethyl)benzimidazol werden in 50 ml Tetrahydrofuran gelöst und mit 1,21 g L-Cystein versetzt. Bei 40° Innentemperatur werden 20 ml 3N Salzsäure zugegeben und 10 Minuten gerührt. Nach Eindampfen im Vakuum wird der Rückstand aus tert.Butyl-methyläther/Aether kristallisiert. Man erhält 2-[[[(R)-2-Amino-2-carboxyäthyl]dithio]methyl]-4--methoxy -3-methyl-1-[5-(trifluormethyl)-2 -benzimidazolyl]-

pyridiniumchlorid vom Schmelzpunkt 168-170°.

## Beispiel 150

3,69 g 2-[[(4-Methoxy-3-methyl-2-pyridyl)methyl]sulfinyl] -5-(trifluormethyl)benzimidazol werden in 50 ml Tetrahydrofuran gelöst und mit 1 ml Aethylmercaptan versetzt. Bei 40° Innentemperatur werden 12 ml 3N Salzsäure zugegeben. Nach 10 Minuten Rühren bei 40° wird das Gemisch im Vakuum eingedampft. Der schaumige Rückstand wird in tert.Butyl-methyläther im Vakuum mehrere Stunden gerührt, wobei eine kristalline Masse ausfällt. Man erhält 2-[(Aethyldithio)methyl]-4-methoxy-3-methyl -1-[5-(trifluormethyl)-2-benzimidazolyl]pyridiniumchlorid vom Schmelzpunkt 130-132°.

## Beispiel 151

3,69 g 2-[[(4-Methoxy-3-methyl-2-pyridyl)methyl]sulfinyl] -5-(trifluormethyl)benzimidazol werden in 50 ml Tetrahydrofuran gelöst und mit 0,8 ml 2-Mercaptoäthanol versetzt. Bei 40° Innentemperatur werden 20 ml 3N Salzsäure zugegeben. Nach 10 Minuten Rühren bei 40° wird das Gemisch im Vakuum eingedampft. Der schaumige Rückstand wird über Nacht in tert.Butylmethyläther/abs. Aether gerührt. Man erhält 2-[[(2-Hydroxyäthyl)dithio]methyl]-4-methoxy-3-methyl -1-[5-(trifluormethyl)-2-benzimidazolyl]pyridiniumchlorid vom Schmelzpunkt 55-60°.

## Beispiel 152

3,69 g 2-[[(4-Methoxy-3-methyl-2-pyridyl)methyl]sulfinyl] -5-(trifluormethyl)benzimidazol werden in 50 ml Tetrahydrofuran gelöst und mit 3,07 g L-Glutathion versetzt. Bei 40° Innentemperatur werden 25 ml 3N Salzsäure zugegeben. Das Gemisch wird 10 Minuten bei 40° gerührt und dann im Vakuum eingedampft. Der Rückstand wird aus tert.Butylmethyl-

äther/abs. Aether kristallisiert. Man erhält 2-[[[(R)-2--[(S)-4-Amino-4-carboxybutyramido] -2-[(carboxymethyl)carbamoyl]äthyl]dithio]methyl]-4-methoxy -3-methyl-1-[5-(trifluormethyl)-2 -benzimidazolyl]pyridiniumchlorid vom Schmelzpunkt 95-100°.

## Beispiel 153

3,69 g 2-[[(4-Methoxy-3-methyl-2-pyridyl)methyl]sulfinyl] -5-(trifluormethyl)benzimidazol werden in 50 ml Tetrahydrofuran gelöst und mit 0,92 g Thioglykolsäure versetzt. Bei 40° Innentemperatur werden 20 ml 3N Salzsäure zugegeben. Das Gemisch wird 10 Minuten bei 40° gerührt und dann im Vakuum eingedampft. Der Rückstand wird aus tert.-Butylmethyläther kristallisiert, und man erhält 2-[[(Carboxymethyl)dithio]methyl]-4-methoxy-3-methyl-1 -[5-(trifluormethyl)-2-benzimidazolyl]pyridiniumchlorid vom Schmelzpunkt 105-110.

## Beispiel 154

3,69 g 2-[[(4-Methoxy-3-methyl-2-pyridyl)methyl]sulfinyl] -5-(trifluormethyl)benzimidazol werden in 50 ml Tetrahydrofuran gelöst und mit 0,77 g Cysteamin versetzt. Bei 40° Innentemperatur werden 25 ml 3N Salzsäure zugegeben. Das Gemisch wird 10 Minuten bei 40° gerührt und dann im Vakuum eingedampft. Der Rückstand wird aus tert.Butylmethyläther kristallisiert. Man erhält 2-[[(2-Aminoäthyl)dithio]-methyl]-4-methoxy-3-methyl -1-[5-(trifluormethyl)-2-benzimidazolyl]pyridiniumchlorid vom Schmelzpunkt 105-110°.

## Beispiel 155

3,69 g 2-[[(4-Methoxy-3-methyl-2-pyridyl)methyl]sulfinyl] -5-(trifluormethyl)benzimidazol werden in 50 ml Tetrahydrofuran gelöst und mit 1,20 g Thioglykoläthylat versetzt. Bei 40° Innentemperatur werden 25 ml 3N Salzsäure

zugegeben. Das Gemisch wird bei 40° gerührt und dann im Vakuum eingedampft. Der Rückstand wird aus tert.Butylmethyläther/abs. Aether kristallisiert. Man erhält 2-[[[(Aethoxycarbonyl)methyl]dithio]methyl]-4-methoxy-3-methyl -1-[5--(trifluormethyl)-2-benzimidazolyl]pyridiniumchlorid vom Schmelzpunkt 125-130°.

## Beispiel 156

a) 21,8 g 5-(Trifluormethyl)-2-benzimidazol-thiol werden in 400 ml Alkohol suspendiert und unter Eiskühlung mit 22,2 g 2-Chlormethyl-4-methoxy-3,5-dimethylpyridin-hydrochlorid versetzt. Hernach tropft man eine Lösung von 8,0 g Natriumhydroxyd in 300 ml Wasser zu, lässt das Gemisch über Nacht am Rückfluss kochen und dampft es anschliessend im Vakuum zur Trockene ein. Der Rückstand wird in 1,5 l Methylenchlorid gelöst. Die Lösung wird zuerst mit 200 ml 1,5N Natronlauge, dann 3 x mit 700 ml Wasser gewaschen, über Natriumsulfat getrocknet und im Vakuum eingedampft. Das Rohprodukt wird an 360 g Kieselgel mit Essigester/Methylenchlorid (1:1) als Eluierungsmittel gereinigt. Kristallisation aus Methylenchlorid/Petroläther ergibt 2-[[(4-Methoxy-3,5-dimethyl-2--pyridyl)methyl]thio] -5-(trifluormethyl)benzimidazol vom Schmelzpunkt 164-166°.

b) 31,4 g 2-[[(4-Methoxy-3,5-dimethyl-2-pyridyl)methyl]-thio] -5-(trifluormethyl)benzimidazol werden in 1,3 l Methylenchlorid und 50 ml Methanol gelöst und dann auf -20° abgekühlt. Innerhalb von 10 Minuten werden 16,9 g aus Methylenchlorid/Petroläther umkristallisierte m-Chlorperbenzoesäure eingetragen. Man rührt die Lösung bei -20° noch 60 Minuten weiter und giesst sie dann in ein Gemisch von 120 ml 2N Natriumcarbonatlösung und Eis. Die organische Phase wird mit Wasser neutral gewaschen, über Natriumsulfat getrocknet und im Vakuum eingedampft. Kristallisation aus Methylenchlorid/Methanol/Petroläther ergibt 2-[[(4-Methoxy-3,5-dimethyl-2--pyridyl)methyl]sulfinyl] -5-(trifluormethyl)benzimidazol

vom Schmelzpunkt 152-154°.

c) 3,83 g 2-[[(4-Methoxy-3,5-dimethyl-2-pyridyl)methyl]sulfinyl] -5-(trifluormethyl)benzimidazol werden in 100 ml Acetonitril gelöst und mit 1,5 g Thioäpfelsäure versetzt. Bei 55° tropft man 25 ml 3N Salzsäure dazu. Die Lösung wird 10 Minuten bei dieser Temperatur gerührt und dann im Vakuum eingedampft. Kristallisation aus Acetonitril/abs. Aether ergibt 2-[[(1,2-Dicarboxyäthyl)dithio]methyl]-4-methoxy-3,5-dimethyl -1-[5-(trifluormethyl)-2-benzimidazolyl]pyridiniumchlorid vom Schmelzpunkt 110-115°.

Beispiel 157

a) 6,54 g 5-(Trifluormethyl)-2-benzimidazol-thiol werden in 200 ml Alkohol suspendiert und unter Eiskühlung mit 5,34 g 2-Chlormethyl-3-methylpyridin-hydrochlorid versetzt. Hernach tropft man eine Lösung von 2,4 g Natriumhydroxyd in 100 ml Wasser zu, lässt das Gemisch über Nacht am Rückfluss kochen und dampft es anschliessend im Vakuum zur Trockene ein. Der Rückstand wird in 950 ml Methylenchlorid gelöst. Die Lösung wird zuerst mit 200 ml 1,5N Natronlauge, dann 3 x mit 250 ml Wasser gewaschen, über Natriumsulfat getrocknet und im Vakuum eingedampft. Das Rohprodukt wird an 100 g Kieselgel mit Essigester/Methylenchlorid (1:1) als Eluierungsmittel gereinigt. Kristallisation aus Methylenchlorid/Petroläther ergibt 2-[[(3-Methyl-2-pyridyl)methyl]thio] 5-(trifluormethyl)benzimidazol vom Schmelzpunkt 180-182°.

b) 6,7 g 2-[[(3-Methyl-2-pyridyl)methyl]thio] -5-(trifluormethyl)benzimidazol werden in 250 ml Methylenchlorid und 20 ml Methanol gelöst und dann auf -20° abgekühlt. Innerhalb von 10 Minuten werden 4,7 g aus Methylenchlorid/Petroläther umkristallisierte m-Chlorperbenzoesäure eingetragen. Man rührt die Lösung bei -20° noch 60 Minuten weiter und giesst sie dann in ein Gemisch von 100 ml 2N Natriumcarbonatlösung und Eis. Die organische Phase wird mit Wasser neutral gewaschen, über Natriumsulfat getrocknet und im Vakuum eingedampft. Kristallisation aus Methylenchlorid/Methanol/Petroläther ergibt 2-[[(3-Methyl-2-pyridyl)methyl]sulfinyl] -5-(trifluormethyl)benzimidazol vom Schmelzpunkt 156-158°.

c) Zu 120 ml 1N Salzsäure und 1,18 g n-Hexanthiol werden zuerst 3,39 g 2-[[(3-Methyl-2-pyridyl)methyl]sulfinyl] -5-(trifluormethyl)benzimidazol und dann 60 ml Methanol zugegeben. Das Gemisch wird bei Raumtemperatur 12 Stunden gerührt und dann im Vakuum eingedampft. Der Rückstand wird aus tert.Butylmethyläther/Aether kristallisiert. Man erhält 2-[(Hexyldithio)methyl]-3-methyl-1-[5-(trifluormethyl) -2-benzimidazolyl]pyridiniumchlorid vom Schmelzpunkt 152-153°.

## Beispiel 158

3,39 g 2-[[(3-Methyl-2-pyridyl)methyl]sulfinyl] -5-(trifluormethyl)benzimidazol werden in 50 ml Tetrahydrofuran gelöst und mit 1,21 g L-Cystein versetzt. Bei 40° Innentemperatur werden 40 ml 3N Salzsäure zugegeben. Nach 5 Minuten Rühren bei 40° und 48 Stunden bei Raumtemperatur wird das Gemisch im Vakuum eingedampft. Der Rückstand wird aus tert.Butylmethyläther/abs. Aether kristallisiert. Man erhält 2-[[(2-Amino-2-carboxyäthyl)dithio]methyl]-3-methyl -1-[5--(trifluormethyl)-2-benzimidazolyl]pyridiniumchlorid vom Schmelzpunkt 116-120°.

## Beispiel 159

3,39 g 2-[[(3-Methyl-2-pyridyl)methyl]sulfinyl] -5-(trifluormethyl)benzimidazol werden in 50 ml Tetrahydrofuran gelöst und mit 1,0 ml n-Propylmercaptan versetzt. Bei 40° Innentemperatur werden 40 ml 3N Salzsäure zugegeben. Nach 5 Minuten Rühren bei 40° gibt man 50 ml Methanol zu und rührt das Gemisch 48 Stunden bei Raumtemperatur weiter. Anschliessend wird es im Vakuum eingedampft. Der Rückstand

wird aus tert.Butylmethyläther/abs. Aether kristallisiert. Man erhält 2-[(Propyldithio)methyl]-3-methyl-1-[5-(trifluormethyl) -2-benzimidazolyl]pyridiniumchlorid vom Schmelzpunkt 144-146°.

## Beispiel 160

3,39 g 2-[[(3-Methyl-2-pyridyl)methyl]sulfinyl] -5-(trifluormethyl)benzimidazol werden in 50 ml Tetrahydrofuran gelöst und mit 0,78 g 2-Mercptoäthanol versetzt. Bei 40° Innentemperatur werden 20 ml 3N Salzsäure zugegeben. Nach 10 Minuten Rühren bei 40° wird das Gemisch im Vakuum eingedampft. Der Rückstand wird aus tert.Butylmethyläther kristallisiert. Man erhält 2-[[(2-Hydroxyäthyl)dithio]methyl]-3-methyl-1 -[5-(trifluormethyl)-2-benzimidazolyl]pyridiniumchlorid vom Schmelzpunkt 70-75°.

## Beispiel 161

3,39 g 2-[[(3-Methyl-2-pyridyl)methyl]sulfinyl] -5-(trifluormethyl)benzimidazol werden in 50 ml Tetrahydrofuran gelöst und mit 1 ml Aethylmercaptan versetzt. Bei 40° Innentemperatur werden 40 ml 3N Salzsäure zugegeben. Nach 5 Minuten Rühren bei 40° und 1 Stunde bei 20° gibt man 50 ml Methanol zu, rührt das Gemisch 24 Stunden bei 20° weiter und dampft es anschliessend im Vakuum ein. Der Rückstand wird aus tert.Butyläther/abs. Aether kristallisiert. Man erhält 2-[(Aethyldithio)methyl]-3-methyl-1-[5-(trifluormethyl) -2-benzimidazolyl]pyridiniumchlorid vom Schmelzpunkt 142-144°.

## Beispiel 162

3,39 g 2-[[(3-Methyl-2-pyridyl)methyl]sulfinyl] -5-(trifluormethyl)benzimidazol werden in 50 ml Tetrahydrofuran gelöst und mit 0,77 g Cysteamin versetzt. Bei 40° Innentemperatur werden 40 ml 3N Salzsäure zugegeben. Nach 10 Minuten

Rühren bei 40° und über Nacht bei Raumtemperatur wird das Gemisch im Vakuum eingedampft. Der Rückstand wird aus tert.-Butylmethyläther/Aether kristallisiert. Man erhält 2-[[(2--Aminoäthyl)dithio]methyl]-3-methyl-1 [5-(trifluormethyl)-2--benzimidazolyl]pyridiniumchlorid vom Schmelzpunkt 95-100°.

Beispiel 163

3,39 g 2-[[(3-Methyl-2-pyridyl)methyl]sulfinyl] -5-(trifluormethyl)benzimidazol werden in 200 ml Aethylmethylketon gelöst und mit 1,20 g Thioglykolsäure-äthylester versetzt. Bei 40° Innentemperatur werden 30 ml 1N Salzsäure zugegeben. Nach 10 Minuten Rühren bei 40° wird das Gemisch im Vakuum eingedampft. Der Rückstand wird aus Aceton/Aether kristallisiert. Man erhält 2-[[[(Aethoxycarbonyl)methyl]dithio]-methyl]-3-methyl-1 -[5-(trifluormethyl)-2-benzimidazolyl]-pyridiniumchlorid vom Schmelzpunkt 144-146°.

Beispiel 164

3,39 g 2-[[(3-Methyl-2-pyridyl)methyl]sulfinyl] -5-(trifluormethyl)benzimidazol werden in 150 ml Tetrahydrofuran gelöst und mit 0,92 g Thioglykolsäure versetzt. Bei 40° Innentemperatur werden 20 ml 3N Salzsäure zugegeben. Nach Rühren über Nacht bei 40° wird das Gemisch im Vakuum eingedampft. Der Rückstand wird aus Aceton/tert.Butylmethyläther/Aether kristallisiert. Man erhält 2-[[(Carboxymethyl)-dithio]methyl]-3-methyl-1 -[5-(trifluormethyl)-2-benzimidazolyl]pyridiniumchlorid vom Schmelzpunkt 110-115°.

Beispiel 165

3,39 g 2-[[(3-Methyl-2-pyridyl)methyl]sulfinyl] -5-(trifluormethyl)benzimidazol werden in 250 ml Aethylmethylketon gelöst und mit 1,84 g Thiosalicylsäure versetzt. Bei 40° Innentemperatur werden 90 ml 3N Salzsäure zugegeben. Nach 10 Minuten Rühren bei 40° und über Nacht bei Raumtemperatur

wird das Gemisch im Vakuum eingedampft. Der Rückstand wird aus tert.Butylmethyläther/Aether kristallisiert. Man erhält 2-[[(o-Carboxyphenyl)dithio]methyl]-3-methyl-1 -[5-(trifluormethyl)-2-benzimidazolyl]pyridiniumchlorid vom Schmelzpunkt 186-188°.

Beispiel 166

3,39 g 2-[[(3-Methyl-2-pyridyl)methyl]sulfinyl] -5-(trifluormethyl)benzimidazol werden in 250 ml Aethylmethylketon gelöst und mit 1,5 g Thioäpfelsäure versetzt. Bei 40° Innentemperatur werden 90 ml 3N Salzsäure zugegeben. Nach 10 Minuten Rühren bei 40° und über Nacht bei Raumtemperatur wird das Gemisch im Vakuum eingedampft. Der Rückstand wird aus tert.Butylmethyläther/Aether kristallisiert. Man erhält 2-[[(1,2-Dicarboxyäthyl)dithio]methyl]-3-methyl-1 -[5-(trifluormethyl)-2-benzimidazolyl]pyridiniumchlorid vom Schmelzpunkt 115-120°.

Beispiel 167

160 ml 3N Salzsäure und 1,44 g 2-Chlorthiophenol werden vorgelegt und unter Eis/Methanol-Kühlung mit 3,39 g 2-[[(3--Methyl-2-pyridyl)methyl]sulfinyl] -5-(trifluormethyl)benzimidazol versetzt. Das Gemisch wird dann über Nacht bei Raumtemperatur gerührt und anschliessend abgenutscht, und der Filterrückstand wird mit Wasser gewaschen. Man erhält 2-[[(o-Chlorphenyl)dithio]methyl]-3-methyl-1 -[5-(trifluormethyl)-2-benzimidazolyl]pyridiniumchlorid vom Schmelzpunkt 68-70°.

Beispiel 168

Unter Eis/Methanol-Kühlung werden 160 ml 3N Salzsäure und 1,12 g 2-Pyrimidinthiol vorgelegt und mit 3,39 g 2-[[(3--Methyl-2-pyridyl)methyl]sulfinyl] -5-(trifluormethyl)benzimidazol versetzt. Das Gemisch wird 3 Stunden bei dieser

Temperatur gerührt und dann im Vakuum eingedampft. Der Rückstand wird aus tert.Butylmethyläther/Aether kristallisiert. Man erhält 2-[(Pyrimidinyldithio)methyl]-3-methyl-1-[5-(trifluormethyl) -2-benzimidazolyl]pyridiniumchlorid vom Schmelzpunkt 135-140°.

Beispiel 169

Unter Eis/Methanol-Kühlung werden 120 ml 1,5N Salzsäure und 1,44 g 3-Chlorthiophenol vorgelegt und mit 3,39 g 2-[[(3-Methyl-2-pyridyl)methyl]sulfinyl] -5-(trifluormethyl)benzimidazol versetzt und 30 Minuten gerührt. Nach Zugabe von 100 ml 3N Salzsäure rührt man das Gemisch über Nacht bei Raumtemperatur. Nach Eindampfen im Vakuum wird der Rückstand aus tert.Butylmethyläther/Aether kristallisiert. Man erhält 2-[[(m-Chlorphenyl)dithio]methyl]-3-methyl-1 -[5-(trifluormethyl)-2-benzimidazolyl]pyridiniumchlorid vom Schmelzpunkt 70-75°.

Beispiel 170

a) 6,54 g 5-(Trifluormethyl)-2-benzimidazol-thiol werden in 400 ml Alkohol suspendiert und unter Eiskühlung mit 5,76 g 2-Chlormethyl-3,5-dimethylpyridin-hydrochlorid versetzt. Hernach tropft man eine Lösung von 2,4 g Natriumhydroxid in 100 ml Wasser zu, lässt das Gemisch über Nacht am Rückfluss kochen und dampft es anschliessend im Vakuum zur Trockene ein. Der Rückstand wird in 800 ml Methylenchlorid gelöst. Die Lösung wird zuerst mit 200 ml 1,5N Natronlauge, dann 3 x mit 250 ml Wasser gewaschen, über Natriumsulfat getrocknet und im Vakuum eingedampft. Das Rohprodukt wird an 110 g Kieselgel mit Essigester/Methylenchlorid (1:1) als Elutionsmittel gereinigt. Kristallisation aus Methylenchlorid/Petroläther ergibt 2-[[(3,5-Dimethyl-2-pyridyl)methyl]thio] -5-(trifluormethyl)benzimidazol vom Schmelzpunkt 137-138°.

b) 5,5 g 2-[[(3,5-Dimethyl-2-pyridyl)methyl]thio] -5-(trifluormethyl)benzimidazol werden in 200 ml Methylenchlorid und 10 ml Methanol gelöst und dann auf -20° abgekühlt. Innerhalb von 10 Minuten werden 4,0 g aus Methylenchlorid/ Petroläther umkristallisierte m-Chlorperbenzoesäure eingetragen. Man rührt die Lösung bei -20° noch 60 Minuten weiter und giesst sie dann in ein Gemisch von 200 ml 2N Natriumcarbonatlösung und Eis. Die organische Phase wird mit Wasser neutral gewaschen, über Natriumsulfat getrocknet und im Vakuum eingedampft. Kristallisation aus Methylenchlorid/ Methanol/Petroläther ergibt 2-[[(3,5-Dimethyl-2-pyridyl)-methyl]sulfinyl] -5-(trifluormethyl)benzimidazol vom Schmelzpunkt 166-168°.

c) 2,9 g 2-[[(3,5-Dimethyl-2-pyridyl)methyl]sulfinyl] -5-(trifluormethyl)benzimidazol werden in 50 ml Tetrahydrofuran gelöst und mit 1 ml n-Propylmercaptan versetzt. Bei 40° Innentemperatur gibt man 10 ml 3N Salzsäure zu. Das Gemisch wird zuerst 10 Minuten bei 40°, dann über Nacht bei Raumtemperatur gerührt und anschliessend im Vakuum eingedampft. Der Rückstand wird aus tert.Butyl-methyläther/Aether kristallisiert. Man erhält 2-[(Propyldithio)methyl]-3,5-dimethyl-1-[5-(trifluormethyl) -2-benzimidazolyl]pyridiniumchlorid vom Schmelzpunkt 110-112°.

## Beispiel 171

3,53 g 2-[[(3,5-Dimethyl-2-pyridyl)methyl]sulfinyl] -5-(trifluormethyl)benzimidazol werden in 50 ml Tetrahyrofuran gelöst und mit 1,18 g n-Hexanthiol versetzt. Bei 40° Innentemperatur gibt man 40 ml 3N Salzsäure zu. Das Gemisch wird bei 40° über Nacht gerührt und anschliessend im Vakuum eingedampft. Der Rückstand wird aus tert.Butyl-methyläther/ Aether kristallisiert. Man erhält 2-[(n-Hexyldithio)methyl]-3,5-dimethyl-1-[5-(trifluormethyl) -2-benzimidazolyl]pyridiniumchlorid vom Schmelzpunkt 80-85°.

Beispiel 172

3,53 g 2-[[(3,5-Dimethyl-2-pyridyl)methyl]sulfinyl]-5-(trifluormethyl)benzimidazol werden in 50 ml Tetrahydrofuran gelöst und mit 1,12 g L-Cystein versetzt. Bei 40° Innentemperatur gibt man 40 ml 3N Salzsäure zu. Das Gemisch wird zuerst 10 Minuten bei 40°, dann über Nacht bei Raumtemperatur gerührt und anschliessend im Vakuum eingedampft. Der Rückstand wird aus tert.Butyl-methyläther/Aether kristallisiert. Man erhält 2-[[[(R)-2-Amino-2-carboxyäthyl]dithio]-methyl]-3,5-dimethyl -1-[5-(trifluormethyl)-2-benzimidazolyl]pyridiniumchlorid vom Schmelzpunkt 148-150°.

Beispiel 173

3,53 g 2-[[(3,5-Dimethyl-2-pyridyl)methyl]sulfinyl]-5-(trifluormethyl)benzimidazol werden in 50 ml Tetrahydrofuran gelöst und mit 1,2 g Thioglykolsäure-äthylester versetzt. Bei 40° Innentemperatur gibt man 40 ml 3N Salzsäure zu. Das Gemisch wird bei 40° über Nacht gerührt und anschliessend im Vakuum eingedampft. Der Rückstand wird aus tert.Butyl-methyläther/Aether kristallisiert. Man erhält 2-[[(Carboxymethyl)dithio]methyl]-3,5-dimethyl-1 -[5-(trifluormethyl)-2-benzimidazolyl]pyridiniumchlorid vom Schmelzpunkt 75-80°.

Beispiel 174

3,53 g 2-[[(3,5-Dimethyl-2-pyridyl)methyl]sulfinyl]-5-(trifluormethyl)benzimidazol werden in 50 ml Tetrahydrofuran gelöst und mit 0,77 g Cysteamin versetzt. Bei 40° Innentemperatur gibt man 40 ml 3N Salzsäure dazu. Das Gemisch wird bei 40° über Nacht gerührt und anschliessend im Vakuum eingedampft. Der Rückstand wird aus tert.Butyl-methyläther/Aether kristallisiert. Man erhält 2-[[(2-Aminoäthyl)dithio]methyl]-3,5-dimethyl-1 -[5-(trifluormethyl)-2-

 

-benzimidazolyl]pyridiniumchlorid vom Schmelzpunkt 115-120°.

## Beispiel 175

3,53 g 2-[[(3,5-Dimethyl-2-pyridyl)methyl]sulfinyl] -5-(trifluormethyl)benzimidazol werden in 50 ml Tetrahydrofuran gelöst und mit 1 ml 2-Mercaptoäthanol versetzt. Bei 40° Innentemperatur gibt man 40 ml 3N Salzsäure dazu. Das Gemisch wird bei 40° über Nacht gerührt und anschliessend im Vakuum eingedampft. Der Rückstand wird aus Aceton/Aether kristallisiert. Man erhält 2-[[(2-Hydroxyäthyl)dithio]-methyl]-3,5-dimethyl-1 -[5-(trifluormethyl)-2-benzimidazolyl]pyridiniumchlorid vom Schmelzpunkt 105-110°.

## Beispiel 176

3,53 g 2-[[(3,5-Dimethyl-2-pyridyl)methyl]sulfinyl] -5-(trifluormethyl)benzimidazol werden in 200 ml Tetrahydrofuran gelöst und mit 1,84 g Thiosalicylsäure versetzt. Bei 40° Innentemperatur gibt man 40 ml 3N Salzsäure zu. Das Gemisch wird zuerst 10 Minuten bei 40°, dann über Nacht bei Raumtemperatur gerührt und anschliessend im Vakuum eingedampft. Der Rückstand wird aus tert.Butyl-methyläther/Aether kristallisiert. Man erhält 2-[[(o-Carboxyphenyl)dithio]-methyl]-3,5-dimethyl-1 -[5-(trifluormethyl)-2-benzimidazolyl]pyridiniumchlorid vom Schmelzpunkt 165-167°.

## Beispiel 177

3,53 g 2-[[(3,5-Dimethyl-2-pyridyl)methyl]sulfinyl] -5-(trifluormethyl)benzimidazol werden in 200 ml Tetrahydrofuran gelöst und mit 1,5 g Thioäpfelsäure versetzt. Bei 40° Innentemperatur gibt man 40 ml 3N Salzsäure zu. Das Gemisch wird zuerst 10 Minuten bei 40°, dann über Nacht bei Raumtemperatur gerührt und anschliessend im Vakuum eingedampft. Der Rückstand wird aus tert.Butyl-methyläther/Aether kristallisiert. Man erhält 2-[[(1,2-Dicarboxyäthyl)dithio]methyl]-

0214479

-3,5-dimethyl-1 -[5-(trifluormethyl)-2-benzimidazolyl]pyridiniumchlorid vom Schmelzpunkt 140-145°.

Beispiel 178

3,53 g 2-[[(3,5-Dimethyl-2-pyridyl)methyl]sulfinyl]-5-(trifluormethyl)benzimidazol werden in 200 ml Tetrahydrofuran gelöst und mit 1 ml Aethylmercaptan versetzt. Bei 40° Innentemperatur gibt man 40 ml 3N Salzsäure zu. Das Gemisch wird zuerst 10 Minuten bei 40°, dann über Nacht bei Raumtemperatur gerührt und anschliessend im Vakuum eingedampft. Der Rückstand wird aus Aceton/Aether kristalisiert. Man erhält 2-[(Aethyldithio)methyl]-3,5-dimethyl-1-[5-(trifluormethyl)-2-benzimidazolyl]pyridiniumchlorid vom Schmelzpunkt 134-136°.

Beispiel 179

3,53 g 2-[[(3,5-Dimethyl-2-pyridyl)methyl]sulfinyl]-5-(trifluormethyl)benzimidazol werden in 200 ml Tetrahydrofuran gelöst und mit 1,44 g 3-Chlorthiophenol versetzt. Bei 40° Innentemperatur gibt man 40 ml 3N Salzsäure dazu. Das Gemisch wird zuerst 10 Minuten bei 40°, dann über Nacht bei Raumtemperatur gerührt und anschliessend im Vakuum eingedampft. Der Rückstand wird aus tert.Butyl-methyläther/Aether kristallisiert. Man erhält 2-[[(m-Chlorphenyl)dithio]-methyl]-3,5-dimethyl-1 -[5-(trifluormethyl)-2-benzimidazolyl]pyridiniumchlorid vom Schmelzpunkt 140-145°.

Beispiel 180

3,53 g 2-[[(3,5-Dimethyl-2-pyridyl)methyl]sulfinyl]-5-(trifluormethyl)benzimidazol werden in 250 ml Aethylmethylketon gelöst und mit 1,12 g 2-Pyrimidinthiol versetzt. Das Gemisch wird mit einem Eis/Methanol-Bad abgekühlt und nach Zugabe von 90 ml 3N Salzsäure 2 Stunden bei dieser Temperatur gerührt. Nach Eindampfen im Vakuum wird der Rückstand aus Aceton/Aether kristallisiert. Man erhält 2-[(2--Pyrimidinyldithio)methyl]-3,5-dimethyl-1 -[5-(trifluormethyl)-2-benzimidazolyl]pyridiniumchlorid vom Schmelzpunkt 75-80°.

Beispiel 181

160 ml 3N Salzsäure und 1,44 g 2-Chlorthiophenol werden vorgelegt und unter Eis/Methanol-Kühlung mit 3,53 g 2-[[(3,5-Dimethyl-2-pyridyl)methyl]sulfinyl] -5-(trifluormethyl)benzimidazol versetzt. Das Gemisch wird über Nacht bei Raumtemperatur gerührt und anschliessend im Vakuum eingedampft. Der Rückstand wird aus tert.Butyl-methyläther kristallisiert. Man erhält 2-[[(o-Chlorphenyl)dithio]-methyl]-3,5-dimethyl-1 -[5-(trifluormethyl)-2-benzimidazolyl]pyridiniumchlorid vom Schmelzpunkt 153-155°.

Beispiel 182

Eine Lösung von 0,794 g N-(2-Mercaptopropionyl)glycin in 10 ml 1N wässeriger Salzsäure wird mit 2 g 5,7-Dihydro-2--[[(4-methoxy-3-methyl-2-pyridyl)methyl]sulfinyl] -5,5,7,7--tetramethylindeno[5,6-d]imidazol-6(1H)-on versetzt. Man engt die klare Lösung ein, löst den Rückstand in Aceton, versetzt mit Essigester und kühlt auf etwa 5° ab, worauf das kristalline Produkt abfiltriert und mit kaltem Essigester gewaschen wird. Das erhaltene 2-[[[1-[(Carboxymethyl)carbamoyl]äthyl]dithio]methyl] -4-methoxy-3-methyl-1-(1,5,6,7--tetrahydro-5,5,7,7 -tetramethyl-6-oxoindeno[5,6-d]imidazol--2-yl)pyridiniumchlorid zeigt einen Schmelzpunkt von 172° (Zers.).

**Beispiel A**

Kristalline Verbindungen der Formel I können als Wirkstoffe zur Herstellung von Hartgelatinekapseln verwendet werden, deren Inhalt pro Kapsel folgende Zusammensetzung aufweist:

| | |
|---|---|
| Wirkstoff | 50,0 mg |
| Milchzucker pulv. | 40,0 mg |
| Milchzucker krist. | 130,0 mg |
| Maisstärke weiss | 20,0 mg |
| Talk | 8,0 mg |
| Magnesiumstearat | 2,0 mg |
| Füllgewicht pro Kapsel | 250,0 mg |

Der Wirkstoff und die Hilfsstoffe werden miteinander vermischt, und das Gemisch wird in Hartgelatinekapseln geeigneter Grösse abgefüllt. Erforderlichenfalls werden die Kapseln anschliessend mit einem magensaftresistenten Lack, bestehend aus Hydroxypropylmethylcellulosephthalat, versehen.

0214479

## Patentansprüche

1. Benzimidazol-2-yl-pyridiniumverbindungen der allgemeinen Formel

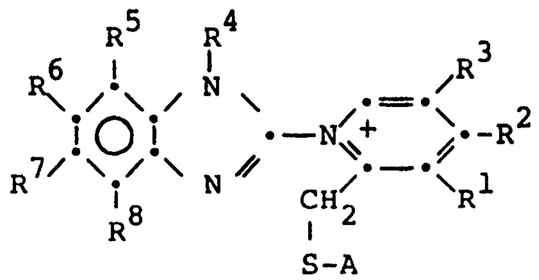

I

worin

A einen Rest der Formel

$-SR^9$, $-SO_3^-$ oder $-S-SO_3^-$ ;

$R^1$ und $R^3$ je Wasserstoff oder $(C_1-C_7)$-Alkyl;

$R^2$ Wasserstoff, $(C_1-C_7)$-Alkyl, $(C_1-C_7)$-Alkoxy oder ein negativ geladenes Sauerstoffatom;

$R^4$ Wasserstoff oder eine negative Ladung;

$R^5$, $R^6$, $R^7$ und $R^8$ je Wasserstoff, $(C_1-C_7)$-Alkyl, Aryl, Halogen, Cyan, Nitro, Formyl, $(C_2-C_7)$-Alkanoyl, Arylcarbonyl, Carboxy, Carboxy-$(C_1-C_7)$-alkyl, $(C_1-C_7)$-Alkoxycarbonyl, Aryloxycarbonyl, Aryl-$(C_1-C_7)$-alkoxycarbonyl, $(C_1-C_7)$-Alkoxycarbonyl-$(C_1-C_7)$-alkyl, Carbamoyl, Mono- oder Di-$(C_1-C_7)$-alkylcarbamoyl, Pyrrolidinocarbonyl, Piperidinocarbonyl, Carbamoyl-$(C_1-C_7)$-alkyl, Mono- oder Di-$(C_1-C_7)$-alkylcarbamoyl-$(C_1-C_7)$-alkyl, Pyrrolidinocarbonyl-$(C_1-C_7)$-alkyl, Piperidinocarbonyl-$(C_1-C_7)$-alkyl, Hydroxy, $(C_1-C_7)$-Alkoxy, $(C_2-C_7)$-Alkanoyloxy, Aryloxy, Arylcarbonyloxy, $(C_1-C_7)$-Alkoxycarbonyloxy, Aryl-$(C_1-C_7)$-alkoxycarbonyloxy, Aryloxycarbonyloxy, Carbamoyloxy, Mono- oder Di-$(C_1-C_7)$-alkylcarbamoyloxy, Pyrrolidinocarbonyloxy, Piperidinocarbonyloxy, Hydroxy-$(C_1-C_7)$-alkyl, Trifluormethyl, Di-$(C_1-C_7)$-alkoxymethyl oder $(C_2-C_3)$-Alkylendioxymethyl oder

zwei benachbarte dieser Substituenten gemeinsam und zusammen mit den Kohlenstoffatomen, an welche sie gebunden sind, einen 5-, 6- oder 7-gliedrigen Ring; und

$R^9$ $(C_1-C_{20})$-Alkyl, $(C_3-C_7)$-Cycloalkyl, $(C_3-C_7)$-Alkenylalkyl, $(C_3-C_7)$-Alkinylalkyl, substituiertes $(C_3-C_7)$-Alkenylalkyl, Aryl, Aryl-$(C_1-C_7)$-alkyl, Hydroxy-$(C_2-C_7)$-alkyl, $(C_1-C_7)$-Alkoxy-$(C_2-C_7)$-alkyl, $(C_1-C_7)$-Alkoxycarbonyl-$(C_1-C_7)$-alkyl, Carboxy-$(C_1-C_7)$-alkyl, Di-$(C_1-C_7)$-alkoxycarbonyl-$(C_2-C_7)$-alkyl, Dicarboxy-$(C_2-C_7)$-alkyl, Carboxy-$(C_1-C_7)$-alkylcarbamoyl-$(C_1-C_7)$-alkyl, gegebenenfalls N-substituiertes Amino-$(C_2-C_7)$-alkyl, gegebenenfalls N-substituiertes Amino-carboxy-$(C_2-C_7)$-alkyl, gegebenenfalls N-substituiertes Amino-$(C_1-C_7)$-alkoxycarbonyl-$(C_2-C_7)$-alkyl, Heteroaryl, Heteroaryl-$(C_1-C_7)$- -alkyl oder einen durch Elimination der SH-Gruppe von einem cysteinhaltigen Oligopeptid abgeleiteten Rest bedeuten;

wobei das Molekül gesamthaft ungeladen oder einfach positiv geladen ist und wobei im letzteren Fall ein externes Anion vorliegt;

sowie pharmazeutisch annehmbare Säureadditionssalze von basisch substituierten Verbindungen der Formel I;

mit Ausnahme derjenigen Verbindungen, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten.

2. Verbindungen gemäss Anspruch 1, worin A einen Rest der Formel $-SO_3^-$, $-S-SO_3^-$ oder $-SR^9$ und $R^9$ $(C_1-C_{20})$-Alkyl, $(C_3-C_7)$- -Cycloalkyl, $(C_3-$ $-C_7)$-Alkenylalkyl, $(C_3-C_7)$- -Alkinylalkyl, substituiertes $(C_3-C_7)$-Alkenylalkyl, Aryl, Aryl-$(C_1-$ $-C_7)$-alkyl, Hydroxy-$(C_2-C_7)$-alkyl, $(C_1-C_7)$-Alkoxy-$(C_2-C_7)$-

-alkyl, $(C_1-C_7)$-Alkoxycarbonyl-$(C_1-C_7)$-alkyl, Carboxy-$(C_1-C_7)$-alkyl, Di-$(C_1-C_7)$-alkoxycarbonyl-$(C_2-C_7)$-alkyl, Dicarboxy-$(C_2-C_7)$-alkyl, gegebenenfalls N-substituiertes Amino-$(C_2-C_7)$-alkyl, gegebenenfalls N-substituiertes Amino-carboxy-$(C_2-C_7)$-alkyl, gegebenenfalls N-substituiertes Amino-$(C_1-C_7)$-alkoxy-carbonyl-$(C_2-C_7)$-alkyl, Heteroaryl, Heteroaryl-$(C_1$-$-C_7)$-alkyl oder einen durch Elimination der SH-Gruppe von einem cysteinhaltigen Oligopeptid abgeleiteten Rest bedeuten.

3. Verbindungen gemäss Anspruch 1 oder 2, worin zwei benachbarte der Substituenten $R^5$, $R^6$, $R^7$ und $R^8$ gemeinsam und zusammen mit den Kohlenstoffatomen, an welche sie gebunden sind, einen 5-, 6- oder 7-gliedrigen Ring und die beiden übrigen dieser Substituenten je Wasserstoff bedeuten.

4. Verbindungen gemäss Anspruch 3, worin $R^5$ und $R^6$ gemeinsam und zusammen mit den Kohlenstoffatomen, an welche sie gebunden sind, einen Thiazol-, Thiadiazol- oder 1,3-Dioxanring bedeuten oder worin $R^6$ und $R^7$ gemeinsam und zusammen mit den Kohlenstoffatomen, an welche sie gebunden sind, einen Ring der Formel

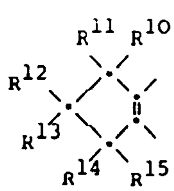
(a)
,
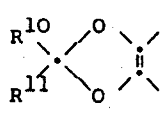
(b)
,
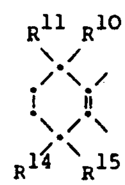
(c)
,
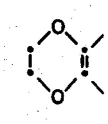
(d)
oder
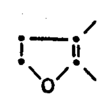
(e)

$R^{10}$, $R^{11}$, $R^{14}$ und $R^{15}$ je Wasserstoff oder $(C_1-C_7)$-Alkyl; und

entweder $R^{12}$ Wasserstoff und $R^{13}$ Wasserstoff, Hydroxy, $(C_1-C_7)$-Alkoxy oder $(C_2-C_7)$-Alkanoyloxy oder $R^{12}$ und $R^{13}$ zusammen Oxo, Oximino oder

$(C_1-C_7)$-Alkoxyimino bedeuten.

5. Verbindungen gemäss Anspruch 4, worin $R^{10}$, $R^{11}$, $R^{14}$ und $R^{15}$ entweder alle Wasserstoff oder alle Methyl und $R^{12}$ und $R^{13}$ zusammen Oxo bedeuten.

6. Verbindungen gemäss Anspruch 4 oder 5, worin $R^6$ und $R^7$ gemeinsam und zusammen mit den Kohlenstoffatomen, an welche sie gebunden sind, einen Ring der Formel (a), (b) oder (c) bedeuten.

7. Verbindungen gemäss Anspruch 1 oder 2, worin keiner der Substituenten $R^5$, $R^6$, $R^7$ und $R^8$ Bestandteil eines Rings ist.

8. Verbindungen gemäss Anspruch 7, worin $R^5$, $R^7$ und $R^8$ je Wasserstoff bedeuten.

9. Verbindungen gemäss Anspruch 8, worin $R^6$ Wasserstoff bedeutet.

10. Verbindungen gemäss Anspruch 8, worin $R^6$ Methoxy oder Trifluormethyl bedeutet.

11. Verbindungen gemäss einem der Ansprüche 1-10, worin $R^1$ Wasserstoff oder Methyl bedeutet.

12. Verbindungen gemäss einem der Ansprüche 1-11, worin $R^2$ Wasserstoff, Methyl, t-Butyl, Methoxy, Aethoxy, n-Propoxy oder ein negativ geladenes Sauerstoffatom bedeutet.

13. Verbindungen gemäss einem der Ansprüche 1-12, worin $R^3$ Wasserstoff oder Methyl bedeutet.

14. Verbindungen gemäss einem der Ansprüche 2-13, worin A einen Rest der Formel $-SR^9$ und $R^9$ Methyl, Aethyl, n-Propyl, i-Propyl, s-Butyl, t-Butyl, n-Pentyl, n-Hexyl,

n-Dodecyl, n-Hexadecyl, n-Octadecyl, Allyl, Cyclopentyl, 2-Hydroxyäthyl, Methoxycarbonylmethyl, 1,2-Dicarboxyäthyl, 2-Carboxyäthyl, 1-Carboxyäthyl, 2-Amino-2-carboxyäthyl, 2-Amino-2- -äthoxycarbonyläthyl, 3-Amino-3-carboxypropyl, 2-(4-Amino-4-carboxybutyramido) -2-(carboxymethylcarbamoyl)-äthyl, Phenyl, p-Tolyl, m-Chlorphenyl, o-Carboxyphenyl, p-Fluorphenyl, p-Chlorphenyl, m-Methoxyphenyl, Pentafluorphenyl, Benzyl, Triphenylmethyl, 2-Pyridyl, 2-Pyrimidinyl, 5-Amino-1,3,4- -thiadiazol-2-yl, 1H-s-triazol-3-yl, Purin--6-yl, 4,6-Dimethyl-2-pyrimidinyl, Furfuryl oder 2-Dimethylaminoäthyl bedeuten oder worin A einen Rest der Formel $-SO_3^-$ oder $-S-SO_3^-$ bedeutet.

15. Verbindungen gemäss einem der Ansprüche 1-13, worin A einen Rest der Formel $-SR^9$ und $R^9$ n-Octyl, t-Octyl, Cyclohexyl, 2-Chlorallyl, o-Tolyl, o-Chlorphenyl, m-Fluorphenyl, p-Methoxyphenyl, o-Methoxyphenyl, 2,6-Dichlorphenyl, 2,5-Dichlorphenyl, 2,3-Dichlorphenyl, 2,4-Dichlorphenyl, 3,4-Dichlorphenyl, o-Methoxycarbonylphenyl, p-Nitrophenyl, m-Bromphenyl, p-Bromphenyl, m-Aminophenyl, o-Aminophenyl, p-Aetylaminophenyl, m-Trifluormethylphenyl, o-Nitrobenzyl, p-Fluorbenzyl, p-Methoxybenzyl, m-Trifluormethylbenzyl, 2-Phenyläthyl, o-Chlorbenzyl, p-Chlorbenzyl, m-Nitrobenzyl, 3,4-Dichlorbenzyl, 2,4-Dichlorbenzyl, 5-Methyl-1,3,4-thiadiazol-2-yl, 4-Methyl-4H-1,2,4-triazol-3-yl, 1-Phenyl--1H-tetrazol-5-yl, 4-Hydroxy-6-propyl-2-pyrimidinyl, 2-Benzimidazolyl, 4-Pyridyl, 5-Nitro-2-benzimidazolyl, Aethoxycarbonylmethyl, Carboxymethyl, 1,2-Bis-(äthoxycarbonyl)-äthyl, 2-Amino-2-carboxy-1,1-dimethyläthyl, 2-Aminoäthyl oder 1-[(Carboxymethyl)carbamoyl]äthyl bedeuten.

16. Verbindungen gemäss Anspruch 1, worin $R^1$ $(C_1-C_7)$-Alkyl, $R^2$ $(C_1-C_7)$-Alkoxy, $R^3$ Wasserstoff oder $(C_1-C_7)$-Alkyl, A einen Rest der Formel $-SR^9$, $R^9$ n-Propyl, 2-Amino-2-carboxyäthyl, 2-(4-Amino-4-carboxy-butyramido) -2-(carboxymethylcarbamoyl)äthyl, n-Hexyl, o-Chlorphenyl, Aethyl, 2-Aminoäthyl, m-Chlorphenyl,

1,2-Bis-(äthoxycarbonyl)äthyl, 2-Amino-2-äthoxycarbonyläthyl, 2-Hydroxyäthyl, 3-Amino-3-carboxypropyl, Cyclopentyl, 2-Dimethylaminoäthyl, o-Carboxyphenyl, Isopropyl, 2-Pyrimidinyl, p-Fluorphenyl, 2-Carboxyäthyl, 1-Carboxyäthyl, p-Chlorphenyl, 3-Furfuryl, n-Pentyl, 2-Chlorallyl, o-Nitrobenzyl, 3,4-Dichlorphenyl, p-Methoxyphenyl, 5-Nitro-2-benzimidazolyl, 1-[(Carboxymethyl)carbamoyl]äthyl oder o-Chlorphenyl, $R^5$ und $R^8$ je Wasserstoff und entweder $R^6$ Trifluormethyl und $R^7$ Wasserstoff oder $R^6$ und $R^7$ zusammen mit den Kohlenstoffatomen, an welche sie gebunden sind, einen Rest der in Anspruch 4 definierten Formel (a) bedeuten.

17. Verbindungen gemäss Anspruch 16, worin $R^1$ Methyl, $R^2$ Methoxy und $R^3$ Wasserstoff oder Methyl bedeuten und worin, wenn $R^6$ und $R^7$ zusammen mit den Kohlenstoffatomen, an welche sie gebunden sind, einen Rest der in Anspruch 4 definierten Formel (a) bedeuten, $R^{12}$ und $R^{13}$ zusammen Oxo und $R^{10}$, $R^{11}$, $R^{14}$ und $R^{15}$ alle Methyl bedeuten.

18. 2-[(Propyldithio)methyl]-4-methoxy-3-methyl -1-[5-(trifluormethyl)-2-benzimidazolyl]pyridiniumchlorid.

19. 2-[[[(R)-2-Amino-2-carboxyäthyl]dithio]methyl -4-methoxy-3-methyl-1-[5-(trifluormethyl)-2-benzimidazolyl]-pyridiniumchlorid.

20. 2-[[[(R)-2-[(S)-4-Amino-4-carboxybutyramido] -2-[(carboxymethyl)carbamoyl]äthyl]dithio]methyl] -4-methoxy-3-methyl-1-[(5-trifluormethyl)-2-benzimidazolyl] -pyridiniumchlorid.

21. 2-[(Hexyldithio)methyl]-4-methoxy-3-methyl -1-[5-(trifluormethyl)-2-benzimidazolyl]-pyridiniumchlorid.

22. 2-[[(o-Chlorphenyl)dithio]methyl]-4-methoxy -3-methyl-1-[5-(trifluormethyl) -2-benzimidazolyl]pyridiniumchlorid.

23. 2-[(Aethyldithio)methyl]-4-methoxy-3-methyl -1-[5-(trifluormethyl)-2-benzimidazolyl]pyridiniumchlorid.

24. 2-[[(2-Aminoäthyl)dithio]methyl]-4-methoxy -3-methyl-1-[5-(trifluormethyl) -2-benzimidazolyl]pyridiniumchlorid.

25. 2-[[(m-Chlorphenyl)dithio]methyl]-4-methoxy -3-methyl-1-(1,5,6,7-tetrahydro-5,5,7,7-tetramethyl -5-oxo-indeno[5,6-d]imidazol-2-yl)pyridiniumchlorid.

26. 2-[[[(R)-2-Amino-2-carboxyäthyl]dithio]methyl] -4-methoxy-3-methyl-1-(1,5,6,7-tetrahydro -5,5,7,7-tetramethyl-5-oxoindeno[5,6-d]imidazol -2-yl)pyridiniumchlorid.

27. 2-[[[1,2-Bis-(äthoxycarbonyl)äthyl]dithio]methyl] -4-methoxy-3-methyl-1-[5-(trifluormethyl) -2-benzimidazolyl]pyridiniumchlorid.

28. 2-[(Aethyldithio)methyl]-4-methoxy-3-methyl -1-(1,5,6,7-tetrahydro-5,5,7,7-tetramethyl-5-oxoindeno [5,6-d]imidazol-2-yl)pyridiniumchlorid.

29. 4-Methoxy-3-methyl-2-[(propyldithio)methyl] -1-(1,5,6,7-tetrahydro-5,5,7,7-tetramethyl-5-oxoindeno [5,6-d]imidazol-2-yl)pyridiniumchlorid.

30. 2-[[[(R)-2-Amino-2-(äthoxycarbonyl)äthyl]dithio] methyl]-4-methoxy-3-methyl-1-(1,5,6,7-tetrahydro -5,5,7,7-tetramethyl-5-oxoindeno[5,6-d]imidazol -2-yl)pyridiniumchlorid.

31. Intramolekular deprotonisiertes 3-[[[2-(Dimethylaminoäthyl]dithio]methyl]-4-methoxy -3-methyl-1-(1,5,6,7--tetrahydro-5,5,7,7-tetramethyl -6-oxoindeno[5,6-d]-imidazol-2-yl-pyridiniumkation.

32. 2-[[(2-Hydroxyäthyl)dithio]methyl]-4-methoxy -3-methyl-1-(1,5,6,7-tetrahydro-5,5,7,7-tetramethyl -5-oxo-indeno[5,6-d]imidazol-2-yl)pyridiniumchlorid.

33. 2-[[[(RS)-3-Amino-3-carboxypropyl]dithio]methyl] -4-methoxy-3-methyl-1-(1,5,6,7-tetrahydro-5,5,7,7-tetramethyl-5-oxoindeno[5,6-d]imidazol-2-yl)pyridiniumchlorid.

34. 2-[(Cyclopentyldithio)methyl]-4-methoxy-3-methyl -1-(1,5,6,7-tetrahydro-5,5,7,7-tetramethyl-5-oxoindeno [5,6-d]imidazol-2-yl)pyridiniumchlorid.

35. 2-[[(o-Carboxyphenyl)dithio]methyl]-4-methoxy-3-methyl-1-(1,5,6,7-tetrahydro-5,5,7,7-tetramethyl-5-oxoin-deno[5,6-d]imidazol-2-yl)pyridiniumchlorid;

2-[(Isopropyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[(2-Pyrimidinyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Fluorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(2-Carboxyäthyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(1-Carboxyäthyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Chlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[(Hexyldithio)methyl]-4-methoxy-3-methyl-1-(1,5,6,7-tetrahydro-5,5,7,7-tetramethyl-5-oxoindeno[5,6-d]imidazol-2-yl)pyridiniumchlorid;

Intramolekular deprotonisiertes 2-[(3-Furfuryldithio)-methyl]-4-methoxy-3-methyl-1-(1,5,6,7-tetrahydro-5,5,7,7-tetramethyl-6-oxoindeno[5,6-d]imidazol-2-yl)pyridiniumkation;

4-Methoxy-3-methyl-2-[(pentyldithio)methyl]-1-(1,5,6,7-tetrahydro-5,5,7,7-tetramethyl-5-oxoindeno[5,6-d]imidazol-2-yl)pyridiniumchlorid;

2-[[(2-Chlorallyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

4-Methoxy-3-methyl-2-[[(o-nitrobenzyl)dithio]methyl]-1-(1,5,6,7-tetrahydro-5,5,7,7-tetramethyl-5-oxoindeno[5,6-d]imidazol-2-yl)pyridiniumchlorid;

2-[[(3,4-Dichlorphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

2-[[(p-Methoxyphenyl)dithio]methyl]-4-methoxy-3-methyl-1-(1,5,6,7-tetrahydro-5,5,7,7-tetramethyl-5-oxoindeno[5,6-d]imidazol-2-yl)pyridiniumchlorid;

4-Methoxy-3-methyl-2-[[(5-nitro-2-benzimidazolyl)dithio]-methyl]-1-(1,5,6,7-tetrahydro-5,5,7,7-tetramethyl-5-oxoindeno[5,6-d]imidazol-2-yl)pyridiniumchlorid;

2-[[[-1-[(Carboxymethyl)carbamoyl]äthyl]dithio]methyl-4-methoxy-3-methyl-1-(1,5,6,7-tetrahydro-5,5,7,7-tetramethyl-5-oxoindeno[5,6-d]imidazol-2-yl)pyridiniumchlorid; und

2-[[(o-Chlorphenyl)dithio]methyl]-4-methoxy-3,5-dimethyl-1-(1,5,6,7-tetrahydro-5,5,7,7-tetramethyl-5-oxoindeno[5,6-d]imidazol-2-yl)pyridiniumchlorid.

36. Verbindungen gemäss einem der Ansprüche 1 bis 35 und solche der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, zur Anwendung als therapeutische Wirkstoffe.

37. Verbindungen gemäss einem der Ansprüche 1 bis 35 und solche der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, zur Anwendung als therapeutische Wirkstoffe mit magensäuresekretionshemmenden und/oder mucosaprotektiven Eigenschaften bei der Bekämpfung oder Verhütung von Ulcus ventriculi und/oder duodeni.

38. Verfahren zur Herstellung von Verbindungen gemäss einem der Ansprüche 1 bis 35, dadurch gekennzeichnet, dass man

a) eine Verbindung der allgemeinen Formel

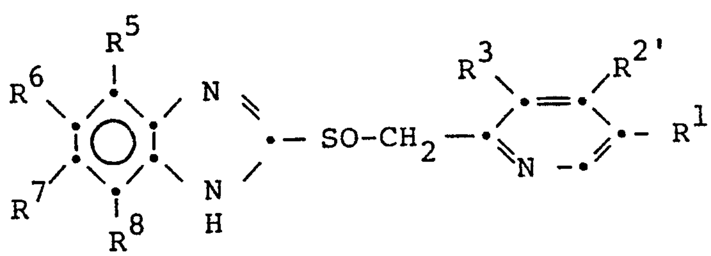

II

worin $R^1$, $R^3$, $R^5$, $R^6$, $R^7$ und $R^8$ die in Anspruch 1 angegebene Bedeutung besitzen und $R^{2'}$ Wasserstoff, $(C_1-C_7)$-Alkyl oder $(C_1-C_7)$-Alkoxy bedeutet,

in Gegenwart von Säure mit einer Verbindung der allgemeinen Formel

$$HS-R^9 \qquad III$$

worin $R^9$ die in Anspruch 1 angegebene Bedeutung besitzt,

oder mit $SO_2$ oder mit einem Alkalimetallthiosulfat umsetzt; oder

b) in einer Verbindung der in Anspruch 1 definierten allgemeinen Formel I, worin $R^2$ $(C_1-C_7)$-Alkoxy bedeutet, die durch $R^2$ bezeichnete $(C_1-C_7)$-Alkoxygruppe spaltet oder durch eine andere $(C_1-C_7)$-Alkoxygruppe ersetzt; oder

c) in einer Verbindung der in Anspruch 1 definierten allgemeinen Formel I, worin A einen Rest der Formel $-SR^9$ bedeutet und $R^9$ eine oder mehrere Carboxylgruppe(n) enthält, diese Carboxylgruppe(n) in eine bzw. mehreren $(C_1-C_7)$-Alkoxycarboxylgruppe(n) überführt; oder

d) in einer Verbindung der in Anspruch 1 definierten allgemeinen Formel I, worin A einen Rest der Formel $-SR^9$ bedeutet und $R^9$ eine oder mehrere $(C_1-C_7)$-Alkoxycarbonylgruppe(n) enthält, diese $(C_1-C_7)$-Alkoxycarbonylgruppe(n) zu einer bzw. mehreren Carboxylgruppe(n) hydrolysiert;

worauf man das erhaltene Produkt als Salz oder inneres Salz isoliert und erwünschtenfalls ein basisches Produkt in ein pharmazeutisch annehmbares Säureadditionssalz überführt.

39. Arzeimittel, enthaltend eine Verbindung gemäss einem der Ansprüche 1 bis 35 oder eine solche der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, und ein therapeutisch inertes Excipiens.

40. Arzneimittel gemäss Anspruch 39 mit magensäuresekretionshemmenden und/oder mucosaprotektiven Eigenschaften zur Bekämpfung oder Verhütung von Ulcus ventriculi und/oder duodeni.

41. Verwendung von Verbindungen gemäss einem der Ansprüche 1-35 und von solchen der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, bei der Bekämpfung oder Verhütung von Krankheiten.

42. Verwendung von Verbindungen gemäss einem der Ansprüche 1-35 und von solchen der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, bei der Bekämpfung oder Verhütung von Ulcus ventriculi und/oder duodeni.

43. Verwendung von Verbindungen gemäss einem der Ansprüche 1-35 und von solchen der in Anspruch 1 angegebenen Formel I, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten, zur Herstellung von Arzneimitteln gegen Ulcus ventriculi und/oder duodeni.

***

Patentansprüche für Oesterreich

1. Verfahren zur Herstellung von Benzimidazol-2-yl-pyridiniumverbindungen der allgemeinen Formel

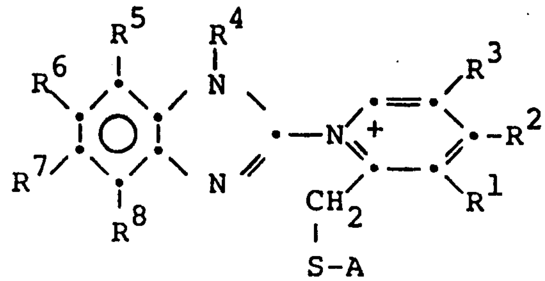

I

worin

A einen Rest der Formel

$-SR^9$, $-SO_3^-$ oder $-S-SO_3^-$;

$R^1$ und $R^3$ je Wasserstoff oder $(C_1-C_7)$-Alkyl;

$R^2$ Wasserstoff, $(C_1-C_7)$-Alkyl, $(C_1-C_7)$-Alkoxy oder ein negativ geladenes Sauerstoffatom;

$R^4$ Wasserstoff oder eine negative Ladung;

$R^5$, $R^6$, $R^7$ und $R^8$ je Wasserstoff, $(C_1-C_7)$-Alkyl, Aryl, Halogen, Cyan, Nitro, Formyl, $(C_2-C_7)$-Alkanoyl, Arylcarbonyl, Carboxy, Carboxy-$(C_1-C_7)$-alkyl, $(C_1-C_7)$-Alkoxycarbonyl, Aryloxycarbonyl, Aryl-$(C_1-C_7)$-alkoxycarbonyl, $(C_1-C_7)$-Alkoxycarbonyl-$(C_1-C_7)$-alkyl, Carbamoyl, Mono- oder Di-$(C_1-C_7)$-alkylcarbamoyl, Pyrrolidinocarbonyl, Piperidinocarbonyl, Carbamoyl-$(C_1-C_7)$-alkyl, Mono- oder Di-$(C_1-C_7)$-alkylcarbamoyl-$(C_1-C_7)$-alkyl, Pyrrolidinocarbonyl-$(C_1-C_7)$-alkyl, Piperidinocarbonyl-$(C_1-C_7)$-alkyl, Hydroxy, $(C_1-C_7)$-Alkoxy, $(C_2-C_7)$-Alkanoyloxy, Aryloxy, Arylcarbonyloxy, $(C_1-C_7)$-Alkoxycarbonyloxy, Aryl-$(C_1-C_7)$-alkoxycarbonyloxy, Aryloxycarbonyloxy, Carbamoyloxy, Mono- oder Di-$(C_1-C_7)$-alkylcarbamoyloxy, Pyrrolidinocarbonyloxy, Piperidinocarbonyloxy, Hydroxy-$(C_1-C_7)$-alkyl, Trifluormethyl, Di-$(C_1-C_7)$-alkoxymethyl oder $(C_2-C_3)$-Alkylendioxymethyl oder

zwei benachbarte dieser Substituenten gemeinsam und zusammen mit den Kohlenstoffatomen, an welche sie gebunden sind, einen 5-, 6- oder 7-gliedrigen Ring; und

$R^9$ ($C_1$-$C_{20}$)-Alkyl, ($C_3$-$C_7$)-Cycloalkyl, ($C_3$--$C_7$)-Alkenylalkyl, ($C_3$-$C_7$)-Alkinylalkyl, substituiertes ($C_3$-$C_7$)-Alkenylalkyl, Aryl, Aryl-($C_1$--$C_7$)-alkyl, Hydroxy-($C_2$-$C_7$)-alkyl, ($C_1$-$C_7$)--Alkoxy-($C_2$-$C_7$)-alkyl, ($C_1$-$C_7$)-Alkoxycarbonyl--($C_1$-$C_7$)-alkyl, Carboxy-($C_1$-$C_7$)-alkyl, Di-($C_1$-$C_7$)-alkoxycarbonyl-($C_2$-$C_7$)-alkyl, Dicarboxy-($C_2$-$C_7$)-alkyl, Carboxy-($C_1$-$C_7$)-alkylcarbamoyl-($C_1$-$C_7$)-alkyl, gegebenenfalls N-substituiertes Amino-($C_2$-$C_7$)-alkyl, gegebenenfalls N-substituiertes Amino-carboxy-($C_2$-$C_7$)-alkyl, gegebenenfalls N-substituiertes Amino-($C_1$-$C_7$)-alkoxycarbonyl-($C_2$-$C_7$)-alkyl, Heteroaryl, Heteroaryl--($C_1$-$C_7$)- -alkyl oder einen durch Elimination der SH-Gruppe von einem cysteinhaltigen Oligopeptid abgeleiteten Rest bedeuten;

wobei das Molekül gesamthaft ungeladen oder einfach positiv geladen ist und wobei im letzteren Fall ein externes Anion vorliegt;

sowie von pharmazeutisch annehmbare Säureadditionssalzen von basisch substituierten Verbindungen der Formel I;

mit Ausnahme derjenigen Verbindungen, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten; dadurch gekennzeichnet, dass man

a) eine Verbindung der allgemeinen Formel

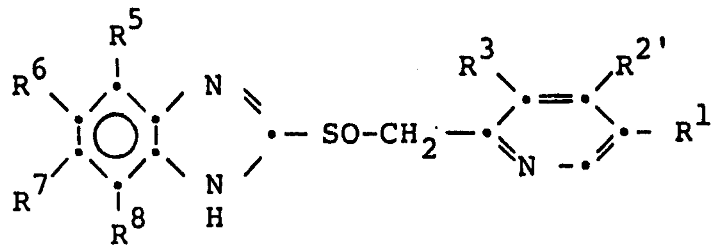

II

worin $R^1$, $R^3$, $R^5$, $R^6$, $R^7$ und $R^8$ obige Bedeutung besitzen und $R^{2'}$ Wasserstoff, $(C_1-C_7)$-Alkyl oder $(C_1-C_7)$-Alkoxy bedeutet, in Gegenwart von Säure mit einer Verbindung der allgemeinen Formel

$$HS-R^9 \qquad III$$

worin $R^9$ obige Bedeutung besitzt, oder mit $SO_2$ oder mit einem Alkalimetallthiosulfat umsetzt; oder

b) in einer Verbindung der obigen allgemeinen Formel I, worin $R^2$ $(C_1-C_7)$-Alkoxy bedeutet, die durch $R^2$ bezeichnete $(C_1-C_7)$-Alkoxygruppe spaltet oder durch eine andere $(C_1-C_7)$-Alkoxygruppe ersetzt; oder

c) in einer Verbindung der obigen allgemeinen Formel I, worin A einen Rest der Formel $-SR^9$ bedeutet und $R^9$ eine oder mehrere Carboxylgruppe(n) enthält, diese Carboxylgruppe(n) in eine bzw. mehreren $(C_1-C_7)$-Alkoxycarboxylgruppe(n) überführt; oder

d) in einer Verbindung der obigen allgemeinen Formel I, worin A einen Rest der Formel $-SR^9$ bedeutet und $R^9$ eine oder mehrere $(C_1-C_7)$-Alkoxycarbonylgruppe(n) enthält, diese $(C_1-C_7)$-Alkoxycarbonylgruppe(n) zu einer bzw. mehreren Carboxylgruppe(n) hydrolysiert;

worauf man das erhaltene Produkt als Salz oder inneres Salz

isoliert und erwünschtenfalls ein basisches Produkt in ein pharmazeutisch annehmbares Säureadditionssalz überführt.

2. Verfahren gemäss Anspruch 1, dadurch gekennzeichnet, dass A einen Rest der Formel $-SO_3^-$, $-S-SO_3^-$ oder $-SR^9$ und $R^9$ $(C_1-C_{20})$-Alkyl, $(C_3-C_7)$-Cycloalkyl, $(C_3-C_7)$-Alkenylalkyl, $(C_3-C_7)$-Alkinylalkyl, substituiertes $(C_3-C_7)$-Alkenylalkyl, Aryl, Aryl-$(C_1-C_7)$-alkyl, Hydroxy-$(C_2-C_7)$-alkyl, $(C_1-C_7)$-Alkoxy-$(C_2-C_7)$-alkyl, $(C_1-C_7)$-Alkoxycarbonyl-$(C_1-C_7)$-alkyl, Carboxy-$(C_1-C_7)$-alkyl, Di-$(C_1-C_7)$-alkoxycarbonyl-$(C_2-C_7)$-alkyl, Dicarboxy-$(C_2-C_7)$-alkyl, gegebenenfalls N-substituiertes Amino-$(C_2-C_7)$-alkyl, gegebenenfalls N-substituiertes Amino-carboxy-$(C_2-C_7)$-alkyl, gegebenenfalls N-substituiertes Amino-$(C_1-C_7)$-alkoxycarbonyl-$(C_2-C_7)$-alkyl, Heteroaryl, Heteroaryl-$(C_1-C_7)$-alkyl oder einen durch Elimination der SH-Gruppe von einem cysteinhaltigen Oligopeptid abgeleiteten Rest bedeuten und dass man nach Varianten a) oder b) arbeitet.

3. Verfahren gemäss Anspruch 1 oder 2, dadurch gekennzeichnet, dass zwei benachbarte der Substituenten $R^5$, $R^6$, $R^7$ und $R^8$ gemeinsam und zusammen mit den Kohlenstoffatomen, an welche sie gebunden sind, einen 5-, 6- oder 7-gliedrigen Ring und die beiden übrigen dieser Substituenten je Wasserstoff bedeuten.

4. Verfahren gemäss Anspruch 3, dadurch gekennzeichnet, dass $R^5$ und $R^6$ gemeinsam und zusammen mit den Kohlenstoffatomen, an welche sie gebunden sind, einen Thiazol-, Thiadiazol- oder 1,3-Dioxanring bedeuten oder worin $R^6$ und $R^7$ gemeinsam und zusammen mit den Kohlenstoffatomen, an welche sie gebunden sind, einen Ring der Formel

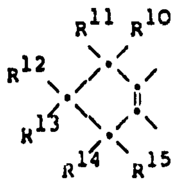 , 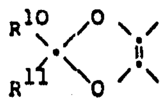 , 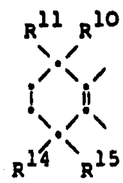 , 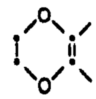 oder 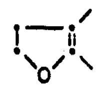

(a) (b) (c) (d) (e)

$R^{10}$, $R^{11}$, $R^{14}$ und $R^{15}$ je Wasserstoff oder $(C_1$-$C_7)$-Alkyl; und entweder $R^{12}$ Wasserstoff und $R^{13}$ Wasserstoff, Hydroxy, $(C_1$-$C_7)$-Alkoxy oder $(C_2$-$C_7)$-Alkanoyloxy oder $R^{12}$ und $R^{13}$ zusammen Oxo, Oximino oder $(C_1$-$C_7)$-Alkoxyimino bedeuten.

5. Verfahren gemäss Anspruch 4, dadurch gekennzeichnet, dass $R^{10}$, $R^{11}$, $R^{14}$ und $R^{15}$ entweder alle Wasserstoff oder alle Methyl und $R^{12}$ und $R^{13}$ zusammen Oxo bedeuten.

6. Verfahren gemäss Anspruch 4 oder 5, dadurch gekennzeichnet, dass $R^6$ und $R^7$ gemeinsam und zusammen mit den Kohlenstoffatomen, an welche sie gebunden sind, einen Ring der Formel (a), (b) oder (c) bedeuten.

7. Verfahren gemäss Anspruch 1 oder 2, dadurch gekennzeichnet, dass keiner der Substituenten $R^5$, $R^6$, $R^7$ und $R^8$ Bestandteil eines Rings ist.

8. Verfahren gemäss Anspruch 7, dadurch gekennzeichnet, dass $R^5$, $R^7$ und $R^8$ je Wasserstoff bedeuten.

9. Verfahren gemäss Anspruch 8, dadurch gekennzeichnet, dass $R^6$ Wasserstoff bedeutet.

10. Verfahren gemäss Anspruch 8, dadurch gekennzeichnet, dass $R^6$ Methoxy oder Trifluormethyl

bedeutet.

11. Verfahren gemäss einem der Ansprüche 1-10, dadurch gekennzeichnet, dass $R^1$ Wasserstoff oder Methyl bedeutet.

12. Verfahren gemäss einem der Ansprüche 1-11, dadurch gekennzeichnet, dass $R^2$ Wasserstoff, Methyl, t-Butyl, Methoxy, Aethoxy, n-Propoxy oder ein negativ geladenes Sauerstoffatom bedeutet.

13. Verfahren gemäss einem der Ansprüche 1-12, dadurch gekennzeichnet, dass $R^3$ Wasserstoff oder Methyl bedeutet.

14. Verfahren gemäss einem der Ansprüche 2-13, dadurch gekennzeichnet, dass A einen Rest der Formel $-SR^9$ und $R^9$ Methyl, Aethyl, n-Propyl, i-Propyl, s-Butyl, t-Butyl, n-Pentyl, n-Hexyl, n-Dodecyl, n-Hexadecyl, n-Octadecyl, Allyl, Cyclopentyl, 2-Hydroxyäthyl, Methoxycarbonylmethyl, 1,2-Dicarboxyäthyl, 2-Carboxyäthyl, 1-Carboxyäthyl, 2-Amino-2-carboxyäthyl, 2-Amino-2-äthoxycarbonyläthyl, 3-Amino-3-carboxypropyl, 2-(4-Amino-4-carboxybutyramido)-2-(carboxymethylcarbamoyl)äthyl, Phenyl, p-Tolyl, m-Chlorphenyl, o-Carboxyphenyl, p-Fluorphenyl, p-Chlorphenyl, m-Methoxyphenyl, Pentafluorphenyl, Benzyl, Triphenylmethyl, 2-Pyridyl, 2-Pyrimidinyl, 5-Amino-1,3,4-thiadiazol-2-yl, 1H-s-triazol-3-yl, Purin-6-yl, 4,6-Dimethyl-2-pyrimidinyl, Furfuryl oder 2-Dimethylaminoäthyl bedeuten oder worin A einen Rest der Formel $-SO_3^-$ oder $-S-SO_3^-$ bedeutet.

15. Verfahren gemäss einem der Ansprüche 1-13, dadurch gekennzeichnet, dass A einen Rest der Formel $-SR^9$ und $R^9$ n-Octyl, t-Octyl, Cyclohexyl, 2-Chlorallyl, o-Tolyl, o-Chlorphenyl, m-Fluorphenyl, p-Methoxyphenyl, o-Methoxyphenyl, 2,6-Dichlorphenyl, 2,5-Dichlorphenyl, 2,3-Dichlorphenyl, 2,4-Dichlorphenyl, 3,4-Dichlorphenyl, o-Methoxycarbonylphenyl, p-Nitrophenyl, m-Bromphenyl, p-Bromphenyl,

m-Aminophenyl, o-Aminophenyl, p-Aetylaminophenyl, m-Trifluormethylphenyl, o-Nitrobenzyl, p-Fluorbenzyl, p-Methoxybenzyl, m-Trifluormethylbenzyl, 2-Phenyläthyl, o-Chlorbenzyl, p-Chlorbenzyl, m-Nitrobenzyl, 3,4-Dichlorbenzyl, 2,4-Dichlorbenzyl, 5-Methyl-1,3,4-thiadiazol-2-yl, 4-Methyl-4H-1,2,4-triazol-3-yl, 1-Phenyl-1H-tetrazol-5-yl, 4-Hydroxy-6-propyl-2-pyrimidinyl, 2-Benzimidazolyl, 4-Pyridyl, 5-Nitro-2-benzimidazolyl, Aethoxycarbonylmethyl, Carboxymethyl, 1,2-Bis-(äthoxycarbonyl)äthyl, 2-Amino-2-carboxy-1,1-dimethyläthyl, 2-Aminoäthyl oder 1-[(Carboxymethyl)carbamoyl]äthyl bedeuten.

16. Verfahren gemäss Anspruch 1, dadurch gekennzeichnet, dass $R^1$ $(C_1-C_7)$-Alkyl, $R^2$ $(C_1-C_7)$-Alkoxy, $R^3$ Wasserstoff oder $(C_1-C_7)$-Alkyl, A einen Rest der Formel $-SR^9$, $R^9$ n-Propyl, 2-Amino-2-carboxyäthyl, 2-(4-Amino-4-carboxybutyramido) -2-(carboxymethylcarbamoyl)-äthyl, n-Hexyl, o-Chlorphenyl, Aethyl, 2-Aminoäthyl, m-Chlorphenyl, 1,2-Bis-(äthoxycarbonyl)äthyl, 2-Amino-2-äthoxycarbonyläthyl, 2-Hydroxyäthyl, 3-Amino-3-carboxypropyl, Cyclopentyl, 2-Dimethylaminoäthyl, o-Carboxyphenyl, Isopropyl, 2-Pyrimidinyl, p-Fluorphenyl, 2-Carboxyäthyl, 1-Carboxyäthyl, p-Chlorphenyl, 3-Furfuryl, n-Pentyl, 2-Chlorallyl, o-Nitrobenzyl, 3,4-Dichlorphenyl, p-Methoxyphenyl, 5-Nitro-2-benzimidazolyl, 1-[(Carboxymethyl)carbamoyl]äthyl oder o-Chlorphenyl, $R^5$ und $R^8$ je Wasserstoff und entweder $R^6$ Trifluormethyl und $R^7$ Wasserstoff oder $R^6$ und $R^7$ zusammen mit den Kohlenstoffatomen, an welche sie gebunden sind, einen Rest der in Anspruch 4 definierten Formel (a) bedeuten.

17. Verfahren gemäss Anspruch 16, dadurch gekennzeichnet, dass $R^1$ Methyl, $R^2$ Methoxy und $R^3$ Wasserstoff oder Methyl bedeuten und worin, wenn $R^6$ und $R^7$ zusammen mit den Kohlenstoffatomen, an welche sie gebunden sind, einen Rest der in Anspruch 4 definierten Formel (a) bedeuten, $R^{12}$ und $R^{13}$ zusammen Oxo und $R^{10}$, $R^{11}$, $R^{14}$

und $R^{15}$ alle Methyl bedeuten.

18. Verfahren gemäss Anspruch 1, dadurch gekennzeichnet, dass man 2-[(Propyldithio)methyl]-4-methoxy--3-methyl -1-[5-(trifluormethyl)-2-benzimidazolyl]pyridiniumchlorid herstellt.

19. Verfahren gemäss Anspruch 1, dadurch gekennzeichnet, dass man 2-[[[(R)-2-Amino-2-carboxyäthyl]dithio]-methyl -4-methoxy-3-methyl-1-[5-(trifluormethyl)-2-benzimidazolyl]-pyridiniumchlorid herstellt.

20. Verfahren gemäss Anspruch 1, dadurch gekennzeichnet, dass man 2-[[[(R)-2-[(S)-4-Amino-4-carboxybutyramido] -2-[(carboxymethyl)carbamoyl]äthyl]dithio]methyl] -4-methoxy-3-methyl-1-[(5-trifluormethyl)-2-benzimidazolyl] -pyridiniumchlorid herstellt.

21. Verfahren gemäss Anspruch 1, dadurch gekennzeichnet, dass man 2-[(Hexyldithio)methyl]-4-methoxy--3-methyl -1-[5-(trifluormethyl)-2-benzimidazolyl]-pyridiniumchlorid herstellt.

22. Verfahren gemäss Anspruch 1, dadurch gekennzeichnet, dass man 2-[[(o-Chlorphenyl)dithio]methyl]--4-methoxy -3-methyl-1-[5-(trifluormethyl) -2-benzimidazolyl]pyridiniumchlorid herstellt.

23. Verfahren gemäss Anspruch 1, dadurch gekennzeichnet, dass man 2-[(Aethyldithio)methyl]-4-methoxy--3-methyl -1-[5-(trifluormethyl)-2-benzimidazolyl]pyridiniumchlorid herstellt.

24. Verfahren gemäss Anspruch 1, dadurch gekennzeichnet, dass man 2-[[(2-Aminoäthyl)dithio]methyl]--4-methoxy -3-methyl-1-[5-(trifluormethyl) -2-benzimidazolyl]pyridiniumchlorid herstellt.

25. Verfahren gemäss Anspruch 1, dadurch gekennzeichnet, dass man 2-[[(m-Chlorphenyl)dithio]methyl]-4-methoxy -3-methyl-1-(1,5,6,7-tetrahydro-5,5,7,7-tetramethyl -5-oxoindeno[5,6-d]imidazol-2-yl)pyridiniumchlorid herstellt.

26. Verfahren gemäss Anspruch 1, dadurch gekennzeichnet, dass man 2-[[[(R)-2-Amino-2-carboxyäthyl]dithio]-methyl] -4-methoxy-3-methyl-1-(1,5,6,7-tetrahydro -5,5,7,7-tetramethyl-5-oxoindeno[5,6-d]imidazol -2-yl)pyridiniumchlorid herstellt.

27. Verfahren gemäss Anspruch 1, dadurch gekennzeichnet, dass man 2-[[[1,2-Bis-(äthoxycarbonyl)äthyl]dithio]methyl] -4-methoxy-3-methyl-1-[5-(trifluormethyl) -2-benzimidazolyl]pyridiniumchlorid herstellt.

28. Verfahren gemäss Anspruch 1, dadurch gekennzeichnet, dass man 2-[(Aethyldithio)methyl]-4-methoxy-3-methyl -1-(1,5,6,7-tetrahydro-5,5,7,7-tetramethyl-5-oxoindeno [5,6-d]imidazol-2-yl)pyridiniumchlorid herstellt.

29. Verfahren gemäss Anspruch 1, dadurch gekennzeichnet, dass man 4-Methoxy-3-methyl-2-[(propyldithio)-methyl] -1-(1,5,6,7-tetrahydro-5,5,7,7-tetramethyl-5-oxoindeno [5,6-d]imidazol-2-yl)pyridiniumchlorid herstellt.

30. Verfahren gemäss Anspruch 1, dadurch gekennzeichnet, dass man 2-[[[(R)-2-Amino-2-(äthoxycarbonyl)-äthyl]dithio] methyl]-4-methoxy-3-methyl-1-(1,5,6,7-tetrahydro -5,5,7,7-tetramethyl-5-oxoindeno[5,6-d]imidazol -2-yl)pyridiniumchlorid herstellt.

31. Verfahren gemäss Anspruch 1, dadurch gekennzeichnet, dass man intramolekular deprotonisiertes 3-[[[2-(Dimethylaminoäthyl]dithio]methyl]-4-methoxy -3-methyl-1-(1,5,6,7- -tetrahydro-5,5,7,7-tetramethyl -6-oxoindeno[5,6-d]-imidazol-2-yl-pyridiniumkation herstellt.

32. Verfahren gemäss Anspruch 1, dadurch gekennzeichnet, dass man 2-[[(2-Hydroxyäthyl)dithio]methyl]- -4-methoxy -3-methyl-1-(1,5,6,7-tetrahydro-5,5,7,7-tetramethyl -5-oxoindeno[5,6-d]imidazol-2-yl)pyridiniumchlorid herstellt.

33. Verfahren gemäss Anspruch 1, dadurch gekennzeichnet, dass man 2-[[[(RS)-3-Amino-3-carboxypropyl]dithio]methyl] -4-methoxy-3-methyl-1-(1,5,6,7-tetrahydro- -5,5,7,7-tetramethyl-5-oxoindeno[5,6-d]imidazol-2-yl)pyridiniumchlorid herstellt.

34. Verfahren gemäss Anspruch 1, dadurch gekennzeichnet, dass man 2-[(Cyclopentyldithio)methyl]-4-methoxy- -3-methyl -1-(1,5,6,7-tetrahydro-5,5,7,7-tetramethyl- -5-oxoindeno [5,6-d]imidazol-2-yl)pyridiniumchlorid herstellt.

35. Verwendung von Verbindungen der in Anspruch 1 definierten allgemeinen Formel I (einschliesslich solcher, worin A den Rest $-SR^9$, $R^9$ Aethyl, 2-Hydroxyäthyl oder Benzyl und entweder $R^1$ und $R^3$ je Methyl, $R^2$ Methoxy, $R^5$, $R^7$ und $R^8$ je Wasserstoff und $R^6$ Methoxy oder $R^1$, $R^2$, $R^3$, $R^5$, $R^6$, $R^7$ und $R^8$ je Wasserstoff bedeuten) oder von pharmazeutisch annehmbaren Säureadditionssalzen davon zur Herstellung von Arzneimitteln gegen Ulcus ventriculi und/oder duodeni.

***